# EUROPEAN PATENT APPLICATION

(11) **EP 4 520 824 A1**
(43) Date of publication of application: **12.03.2025**
(21) Application number: 23196423.0
(22) Date of filing: 10.09.2023
(51) Int. Cl.: C12N 15/113, C12Q 1/68

(54) **DEOXYRIBOZYME THAT GENERATES FLUORESCENCE AND USES THEREOF**

(71) Applicant: Ústav organické chemie a biochemie Akademie ved Ceske Republiky, v.v.i., 16000 Prague (CZ)
(72) Inventor: Curtis, Edward, Praha (CZ); Volek, Martin, Zihle (CZ); Kurfürst, Jaroslav, Praha (CZ)
(74) Representative: Harber IP s.r.o.

(57) **Abstract**

The present disclosure provides deoxyribozymes that generate fluorescent or chromogeneic products by catalyzing the transfer of a phosphate from a substrate. Kits comprising any of the deoxyribozymes described herein are also provided by the present disclosure. The present disclosure also provides methods for identifying such deoxyribozymes. Oligonucleotide sensors that utilize the deoxyribozymes described herein and are capable of generating a fluorescent or chromogeneic product only in the presence of a specific ligand (e.g., a particular target nucleic acid sequence) are also provided by the present disclosure, as well as methods of using the same to detect the presence of a target nucleic acid sequence. The present disclosure further provides sensors that utilize the deoxyribozymes described herein to detect RNase activity.

## Description

### BACKGROUND OF THE INVENTION

Protein enzymes are powerful tools in biotechnology due to their high catalytic efficiencies and wide range of functions. However, DNA enzymes, or deoxyribozymes, can be useful alternatives for some applications. Advantages of deoxyribozymes (and functional DNA molecules in general) include their low cost of synthesis, high chemical stability, and ability to function over a wide range of conditions (Silverman, S.K. Catalytic DNA: Scope, Applications, and Biochemistry of Deoxyribozymes. Trends Biochem. Sci. 2016, 41, 595-609). Powerful methods of artificial evolution have also been developed which make it possible to identify DNA and RNA molecules with new or modified functions from large nucleic acid libraries. Such experiments have shown that deoxyribozymes can catalyze a wide range of simple chemical reactions. These methods have also been used to identify deoxyribozymes with applications in applied research. Examples include RNA-cleaving deoxyribozymes that can detect modified bases in RNA transcripts, peroxidase deoxyribozymes that generate chromogenic signals, and allosterically regulated deoxyribozymes activated by specific ligands. Aptamers provide additional functionality to the toolkit of functional DNA parts. They can bind ligands with affinities comparable to those of protein antibodies, and have been used as reagents for affinity chromatography, in sandwich assays, to enhance the fluorescence of small-molecule ligands, and for a number of other applications. A chip containing thousands of aptamers to different human proteins has also been developed and used to identify biomarkers of human disease.

There is an interest in developing additional DNA components with useful functions. From the perspective of applications such as diagnostics and assay development, DNA enzymes that generate signals could be especially useful. An existing method uses a G-quadruplex deoxyribozyme that catalyzes a nonspecific peroxidase reaction in the presence of a hemin cofactor and hydrogen peroxide (Travascio, P., Li, Y., Sen, D. DNA-enhanced peroxidase activity of a DNA-aptamer-hemin complex. Chem. Biol. 1998, 5, 505-517). This approach is typically used to generate a chromogenic product, but can also produce other types of signals in the presence of appropriate substrates. A limitation of this peroxidase deoxyribozyme is that the hemin cofactor also promotes these reactions in the absence of deoxyribozyme, which results in high background and low signal-to-noise ratios. Another is that hydrogen peroxide can damage hemin (which limits the lifetime of the reaction) and is incompatible with certain types of assays. Motivated by these limitations, we previously developed a chemiluminescent deoxyribozyme called Supernova (described in EP 4043566 A1 and Svehlova, K., Lukšan, O., Jakubec, M., Curtis, E. A. Supernova: A Deoxyribozyme that Catalyzes a Chemiluminescent Reaction. *Angew. Chem. Int. Ed Engl.* **2022,** *61*, e202109347).

### SUMMARY OF THE INVENTION

In this patent application we describe deoxyribozyme motifs that generate additional types of signals. These differ from the G-quadruplex peroxidase deoxyribozyme in a number of ways: they have different folds and sequence requirements, they are kinases rather than peroxidases, they require zinc rather than hemin and hydrogen peroxidase for activity, and they use substrates such as 4-MUP and 4-NPP rather than substrates such as ABTS. They differ from the chemiluminescent deoxyribozyme Supernova with respect to folds and sequence requirements, use different substrates (such as 4-MUP and 4-NPP rather than such as CDP-Star), and generate fluorescent or chromogenic products rather than chemiluminescent ones. We also show that these new deoxyribozymes can be modified to function as sensors, and use one of these sensors to identify enzyme inhibitors in a high-throughput screen.

The present disclosure describes the use of artificial evolution to search a library of randomly mutagenized variants of Supernova for deoxyribozymes that catalyze the dephosphorylation of 4-MUP, a coumarin substrate having weak fluorescence, to generate 4-MU, a product having purple fluorescence. As described further herein, this led to the discovery of Aurora, a deoxyribozyme that is distinct from Supernova with respect to secondary structure, substrate specificity, and type of signal generated. The sequence requirements of Aurora were initially characterized using a randomly mutagenized library in combination with artificial evolution, high-throughput sequencing, and comparative analysis. This information was then used to design a second library enriched for the secondary structure of Aurora, and active variants were once again identified using artificial evolution. Together, these selections yielded a variant of Aurora with a signal-to-noise ratio of fluorescence of 700-fold that produced a detectable signal in minutes. Like Supernova, Aurora displays the best activity in the presence of divalent ions (e.g., zinc ions) for catalytic activity, suggesting possible mechanistic links to protein enzymes that catalyze similar reactions (such as alkaline phosphatase). NMR experiments indicated that these divalent ions are important for folding and leave open the possibility that they also play catalytic roles. Several architectures were also developed that could be used to construct sensors comprising Aurora that produce fluorescence in the presence, but not the absence, of a ligand of interest. One of these architectures was used to make a sensor that can detect picomolar concentrations of the protein enzyme ribonuclease A in a homogeneous assay that did not require wash steps or biochemical purifications. Taken together, the present disclosure provides new ways to generate fluorescent sensors using DNA and represents an important step in the development of orthogonal deoxyribozymes for diagnostic applications.

In addition, the present disclosure describes extension of this approach to the identification of deoxyribozymes that generate a chromogenic product by catalyzing the dephosphorylation of 4-nitrophenyl phosphate (4-NPP), a clear substrate, to generate 4-nitrophenol (4-NP), a yellow product. An optimized variant of one of these deoxyribozymes phosphorylates itself with a catalytic efficiency approaching 10³ M⁻¹ min⁻¹ and generates a yellow product with a signal-to-noise ratio of 200-fold. Like our chemiluminescent and fluorescent deoxyribozymes, multiple zinc ions are required for activity. An engineered version of this dexoyribozyme was constructed that only reacts in the presence of target oligonucleotides with specific sequences, and another architecture was developed that can sense the presence of ribonucleases. An advantage of this deoxyribozyme for some applications is that it generates a product that can detected without specialized equipment. By analyzing datasets of deoxyribozymes selected for the ability to use CDP-Star, 4-MUP, or 4-NPP by high-throughput sequencing, it was possible to identify deoxyribozymes that use new substrates. These results highlight the versatility of our method, and show that it can be used to identify deoxyribozymes that generate a wide range of signals.

Thus, in one aspect, the present disclosure provides deoxyribozymes capable of catalyzing the formation of a fluorescent product. In some aspects, the present disclosure provides deoxyribozymes capable of catalyzing the transfer of a phosphate from a substrate containing at least one phosphate moiety, concomitantly generating a fluorescent product. In some embodiments, the phosphate moiety is transferred to the deoxyribozyme. For example, the phosphate moiety may be transferred to a hydroxyl group on the deoxyribozyme (*e.g*., the 5' hydroxyl group of the deoxyribozyme). In some embodiments, the fluorescent product has greater emissions at one or more wavelengths when illuminated at one or more wavelengths than the substrate. In some embodiments, the substrate containing at least one phosphate moiety is selected from the group consisting of 4-methylumbelliferyl phosphate (4-MUP), 6,8-Difluoro-4-methylumbelliferyl phosphate (diFMUP), and 9H-(1,3-Dichloro-9,9-dimethylacridin-2-one-7-yl) phosphate (DDAO). In certain embodiments, the substrate containing at least one phosphate moiety is 4-MUP. In some embodiments, the deoxyribozymes provided herein comprise the nucleic acid sequence of any one of SEQ ID NOs: 7, 8, 12, 13, 14, 23, and 24, or a nucleic acid sequence that is at least 60%, at least 70%, at least 80%, at least 90%, at least 95%, at least 96%, at least 97%, at least 98%, or at least 99% identical to the sequence of any one of SEQ ID NOs: 7, 8, 12, 13, 14, 23, and 24. In some embodiments, the deoxyribozyme comprises the consensus sequence GGAE₁GDDNNNZ₁Z₃NATF₁X₁P₁CGGNNCCZ₅NNNZ₆GGSNNGHGP₂X₂GF₂GTE₂Z₄Z₂ (SEQ ID NO: 37), wherein each instance of S independently represents C or G, each instance of H independently represents A, C, or T, each instance of D independently represents A, G, or T, and each instance of N independently represents A, C, G, or T; and wherein each instance of F₁ and F₂ independently represents a G-C base pair, a G-T base pair, or an A-T base pair, each instance of X₁ and X₂ independently represents a T-A base pair or a C-G base pair, each instance of P₁ and P₂ independently represents a T-A base pair, a C-G base pair, or a T-G base pair, each instance of Z₁ and Z₂ independently represents an A-T base pair, a T-A base pair, a G-C base pair, a C-G base pair, a G-T base pair, or a T-G base pair, each instance of Z₃ and Z₄ independently represents an A-T base pair, a T-A base pair, a G-C base pair, a C-G base pair, a G-T base pair, or a T-G base pair, each instance of Z₅ and Z₆ independently represents an A-T base pair, a TA base pair, a G-C base pair, a C-G base pair, a G-T base pair, or a T-G base pair, and each instance of E₁ and E₂ independently represents an A-T interaction, a G-A interaction, an A-A interaction, or an A-G interaction.

In another aspect, the present disclosure provides methods of identifying at least one deoxyribozyme capable of catalyzing the transfer of a phosphate moiety from a substrate containing at least one phosphate moiety. In some embodiments, the methods provided herein comprise the steps of:
a. annealing a library containing a plurality of single-stranded deoxyribonucleotides or regenerated according to step (j) of this method to a blocking oligonucleotide,
b. incubating the library annealed to the blocking oligonucleotide of step (a) with a substrate containing at least one phosphate moiety,
c. isolating the DNA of step (b),
d. ligating a deoxyribonucleotide oligo using a splint oligo to the purified DNAs of step (c) containing a 5' phosphate,
e. isolating the deoxyribonucleotide ligated products of step (d),
f. amplifying the isolated deoxyribonucleotide ligation products of step (e) using one forward primer which contains a ribonucleic acid (RNA) linkage 5' of the phosphorylation site and a second reverse deoxyribonucleic acid primer which contains a 5' phosphate,
g. isolating the DNA of step (f),
h. incubating the amplified deoxyribonucleotide ligation products of step (g) with lambda exonuclease to selectively degrade the antisense strand,"
i. digesting the ribonucleic acid (RNA) linkage 5' of the phosphorylation site in the sense strands of DNA of step (h),
j. generating a plurality of single stranded deoxyribonucleotides having the sequence of the deoxyribonucleotide ligation products isolated in step (e), and
k. repeating steps (a)-(j) one or more times.

In some embodiments, the pool of single-stranded deoxyribonucleotides of step (a) comprises the sequences of SEQ ID NO: 1 or SEQ ID NO: 9. In some embodiments, the pool of single-stranded deoxyribonucleotides of step (a) comprises one or more secondary structure libraries.

In another aspect, the present disclosure provides oligonucleotide sensors. In some embodiments, the oligonucleotide sensors provided herein comprise a deoxyribozyme capable of catalyzing the transfer of a phosphate moiety from a substrate containing at least one phosphate, concomitantly generating a fluorescent product only when the deoxyribozyme is bound to a target nucleotide sequence. The oligonucleotide sensors described herein may be used to detect any target nucleic acid sequence of interest. In some embodiments, a target nucleic acid sequence is at least 8, at least 9, at least 10, at least 11, at least 12, at least 13, at least 14, at least 15, at least 16, at least 17, at least 18, at least 19, at least 20, at least 30, at least 40, at least 50, at least 60, at least 70, at least 80, at least 90, at least 100, at least 150, at least 200, at least 250, or at least 300, nucleotides long. In some embodiments, a target nucleic acid sequence is not more than 20, not more than 25, not more than 30, not more than 35, not more than 40, not more than 45, not more than 50, not more than 55, not more than 60, not more than 65, not more than 70, not more than 75, not more than 80, not more than 85, not more than 90, not more than 95, not more than 100, not more than 150, not more than 200, not more than 250, or not more than 300, nucleotides long. In certain embodiments, the oligonucleotide sensor comprises the sequence of any one of SEQ ID NOs: 25-29, or a sequence that is at least 80%, at least 90%, at least 95%, at least 96%, at least 97%, at least 98%, or at least 99% identical to the sequence of any one of SEQ ID NOs: 25-29.

In another aspect, the present disclosure provides RNase detection systems comprising a 5' nucleotide extension to a deoxyribozyme comprising at least 1, 2, 3, 4, 5, 10, 15, 20, 30, 40, or 50 nucleotides, of which the 3' most residue of the extension is a ribonucleotide, and can be cleaved from the RNase detection system by RNase to generate a deoxyribozyme capable of catalyzing the transfer of a phosphate moiety from a substrate containing at least one phosphate or at least one diphosphate moiety, concomitantly generating a fluorescent product. In some embodiments, the RNase detection system comprises the sequence of SEQ ID NO: 35 or SEQ ID NO:36, or a sequence that is at least 80%, at least 90%, at least 95%, at least 96%, at least 97%, at least 98%, or at least 99% identical to the sequence of any one of SEQ ID NO: 35 or SEQ ID NO:36.

In another aspect, the present disclosure provides methods for detecting the presence of a target nucleotide sequence using any of the deoxyribozymes or oligonucleotide sensors provided herein. In some embodiments, the method comprises contacting a sample comprising a plurality of nucleotide sequences with any of the oligonucleotide sensors provided herein, wherein fluorescence is observed when the target nucleotide sequence is present in the sample.

It should be appreciated that the foregoing concepts, and additional concepts discussed below, may be arranged in any suitable combination, as the present disclosure is not limited in this respect. Further, other advantages and novel features of the present disclosure will become apparent from the following detailed description of various non-limiting embodiments when considered in conjunction with the accompanying figures.

### BRIEF DESCRIPTION OF THE DRAWINGS

The following drawings form part of the present specification and are included to further demonstrate certain aspects of the present disclosure, which can be better understood by reference to one or more of these drawings in combination with the detailed description of specific embodiments presented herein.

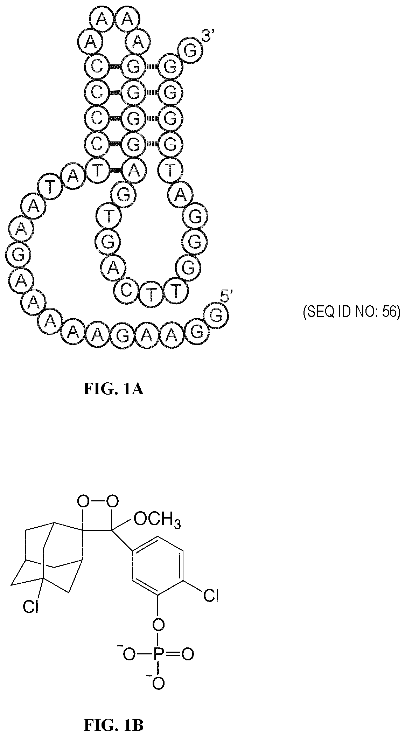

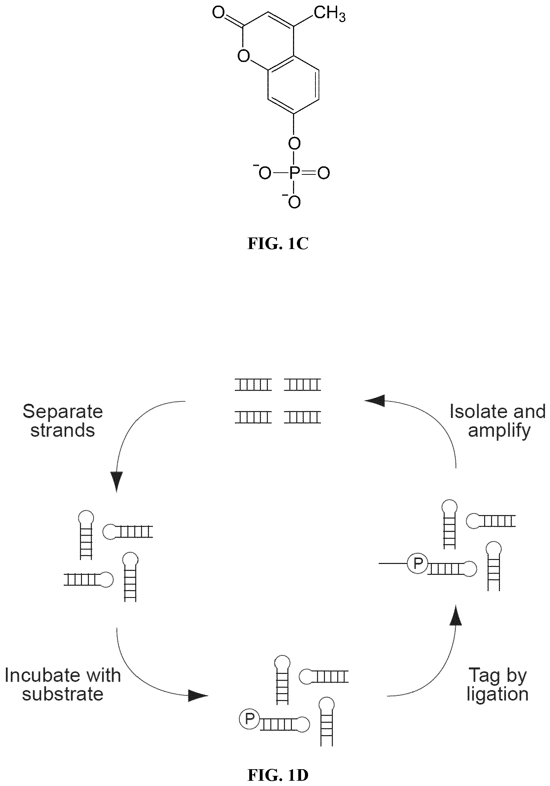

**FIGs. 1A-1D** show identification of deoxyribozymes that generate fluorescence. **FIG. 1A** shows the secondary structure of Supernova, a chemiluminescent deoxyribozyme. **FIG. 1B** shows the chemical structure of CDP-Star, the chemiluminescent substrate used by Supernova. **FIG. 1C** shows the chemical structure of 4-MUP, the fluorescent substrate used by Aurora and other deoxyribozymes related to Aurora. **FIG. 1D** shows the artificial evolution protocol to identify deoxyribozymes from a library of Supernova variants that phosphorylate themselves in the presence of 4-MUP.

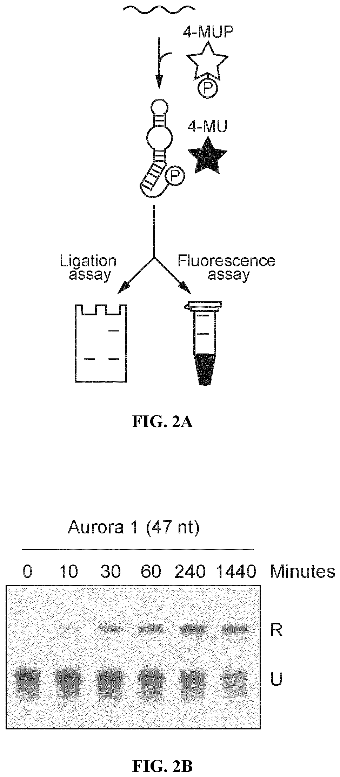

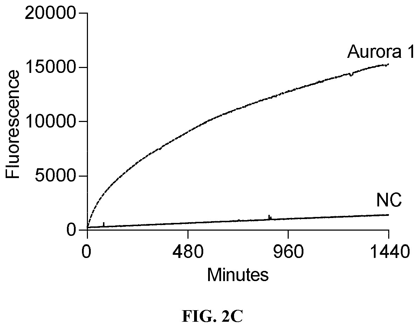

**FIGs. 2A-2C** show that sequences identified in the selection for deoxyribozymes that can react with 4-MUP catalyze self-phosphorylation and generate fluorescence. **FIG. 2A** shows assays for self-phosphorylation (ligation assay) and fluorescence. **FIG. 2B** shows an example of a self-phosphorylation reaction analyzed using the ligation assay. **FIG. 2C** shows an example of a fluorescence assay analyzed using a plate reader.

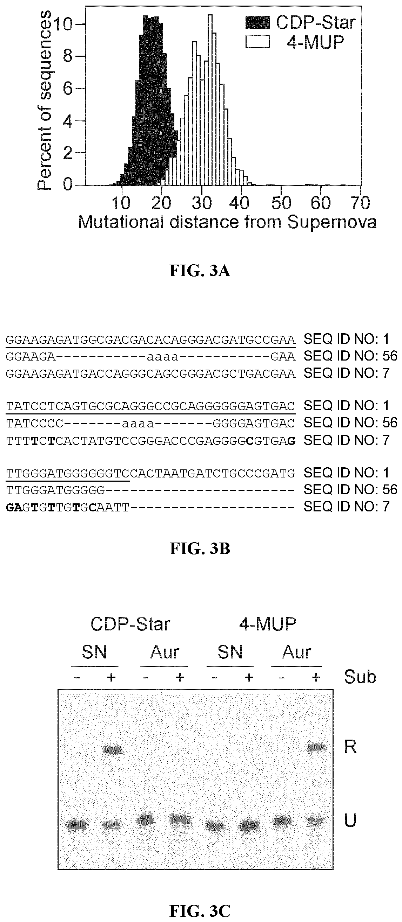

**FIGs. 3A-3C** show that Aurora is structurally and functionally distinct from Supernova. **FIG. 3A** shows the distribution of mutational distances of sequences in the evolved library with respect to Supernova (the sequence used as the starting point for the randomly mutagenized library) when the selection was performed using CDP-Star (described in EP 4043566 A1) or 4-MUP. **FIG. 3B** compares the sequence of the catalytic core of Supernova with the sequence of Aurora 1 full-length. Top sequence: library based on the chemiluminescent DNAzyme Supernova (described in EP 4043566 A1) that was used as the starting point to isolate fluorescent DNAzymes. Underlined positions were randomly mutated at a rate of 21% per position. Middle sequence: minimized catalytic core of Supernova. Nucleotides that were replaced by AAAA spacers are shown in lower case font. Bottom sequence: Aurora 1 full-length (one of the fluoresecent DNAzymes described in this patent application). Nucleotides that are not consistent with the sequence requirements of Supernova are shown in bold. **FIG. 3C** shows catalytic activity of Supernova (labeled "SN") and Aurora 1 (labeled "Aurora") in the presence of CDP-Star (left) and 4-MUP (right). Time points were analyzed using the ligation assay. See Table 1 for the sequence of Aurora 1.

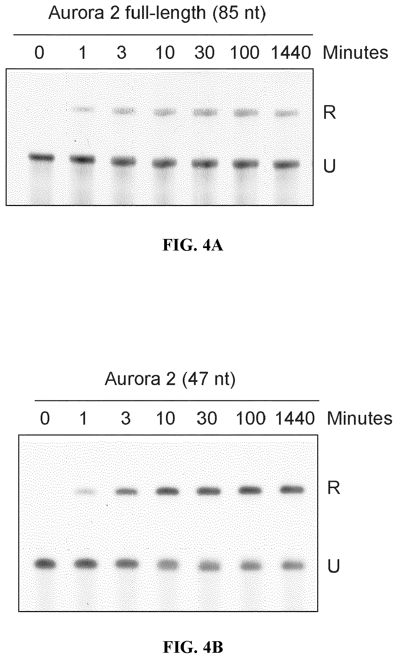

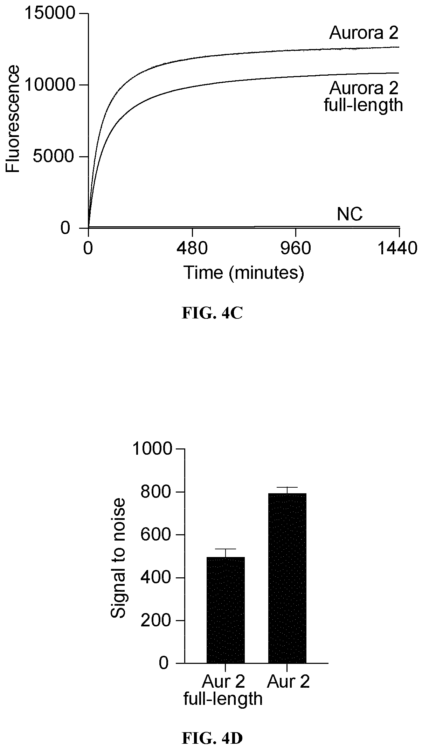

**FIGs. 4A-4D** show catalytic active of full-length and minimized Aurora 2. **FIG. 4A** shows the time course of the 85-nucleotide Aurora 2 full-length deoxyribozyme measured using the ligation assay. Ligations were performed after incubating 1 µM Aurora 2 full-length with 1 mM 4-MUP at the indicated times. **FIG. 4B** is the same experiment as in FIG. 4A, but for Aurora 2 (the 47-nucleotide minimized catalytic core). **FIG. 4C** shows the fluorescence production of Aurora 2 full-length and Aurora 2. Reactions were performed using 15 µM DNA and 30 µM 4-MUP in a buffer containing 200 mM KCl, 1 mM ZnCl₂, 5% (v/v) DMSO, and 50 mM HEPES, pH 7.4. **FIG. 4D** shows the signal-to-noise ratio of fluorescence of Aurora 2 full-length and Aurora 2. Reactions were performed using 15 µM DNA and 30 µM 4-MUP in a buffer containing 200 mM KCl, 1 mM ZnCl₂, 5% (v/v) DMSO, and 50 mM HEPES, pH 7.4. After incubating for 4 hours, 20 µl of 1 M KOH was added and fluorescence was measured using a TECAN Spark plate reader. Signal-to-noise ratio is defined as the rate of production of fluorescence in the presence of deoxyribozyme divided by the rate in the absence of deoxyribozyme. Columns show the average of three experiments, and error bars represent one standard deviation.

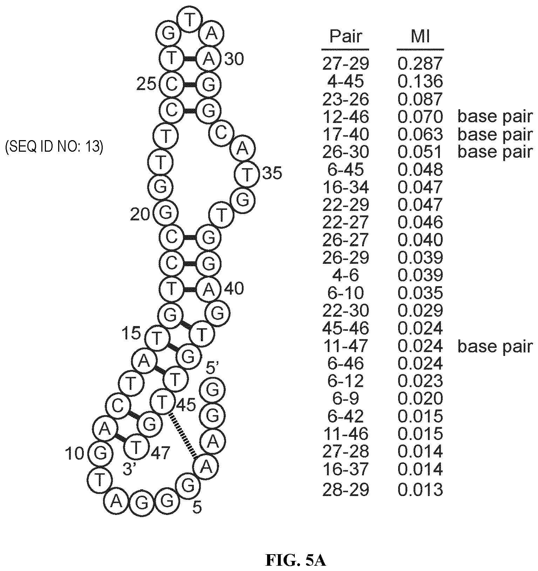

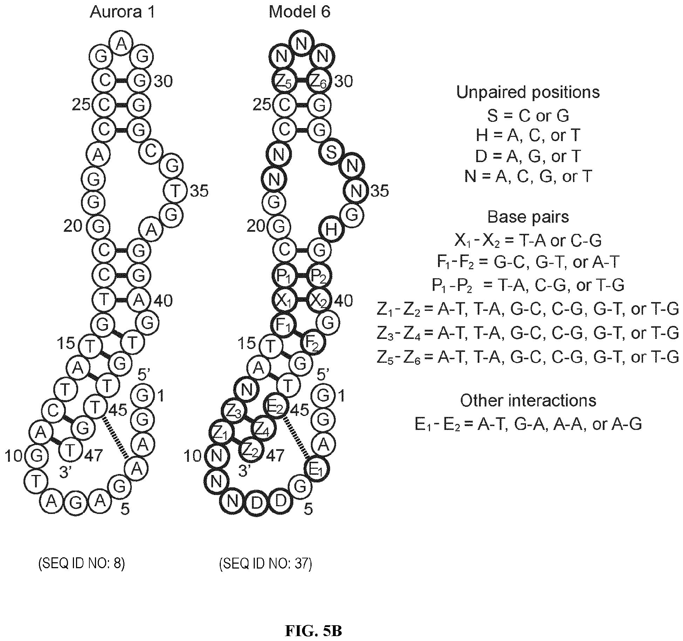

**FIGs. 5A-5B** shows the secondary structure and sequence requirements of Aurora. **FIG. 5A** shows a secondary structure model of Aurora. Base pairs are shown using solid lines, and the 4-45 interaction is indicated using a dotted line. The 25 pairs of positions with the highest mutual information values (indicating pairs of positions that change in a correlated way, which sometimes reflects proximity in the secondary and/or tertiary structure) are shown to the right. **FIG. 5B** shows a representative model ("Model 6") describing the sequence requirements of Aurora as determined by analysis of high-throughput sequencing data.

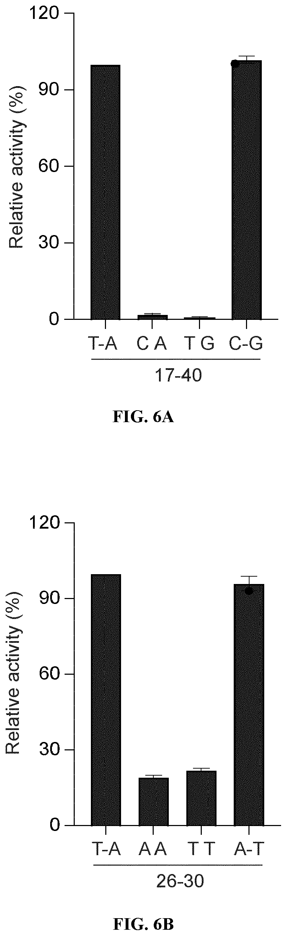

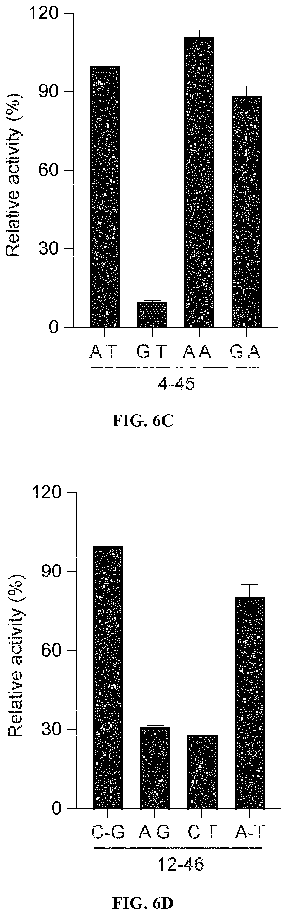

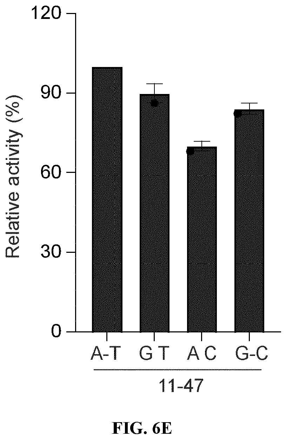

**FIGs. 6A-6E** show testing base pairs and noncanonical interactions in Aurora using double-mutant cycles. **FIG. 6A** shows testing of the 17-40 base pair. Signal-to-noise ratios are expressed relative to a variant containing a T-A base pair. **FIG. 6B** shows testing of the 26-30 base pair. Signal-to-noise ratios are expressed relative to a variant containing a T-A base pair. **FIG. 6C** shows testing of the 4-45 noncanonical pair. Signal-to-noise ratios are expressed relative to a variant containing an A-T pair. **FIG. 6D** shows testing of the 12-46 base pair. Signal-to-noise ratios are expressed relative to a variant containing a C-G base pair. **FIG. 6E** shows testing of the 11-47 base pair. Signal-to-noise ratios are expressed relative to a variant containing an A-T base pair. Reactions were performed using 15 µM Aurora and 30 µM 4-MUP in a buffer containing 200 mM KCl, 1 mM ZnCl₂, 5% (v/v) DMSO, and 50 mM HEPES, pH 7.4. After incubating for 4 hours, 20 µl of 1 M KOH was added, and fluorescence was measured using a TECAN Spark plate reader. Signal-to-noise ratio is defined as the rate of production of fluorescence in the presence of deoxyribozyme divided by the rate in the absence of deoxyribozyme. Columns show the values from three experiments, and error bars represent one standard deviation.

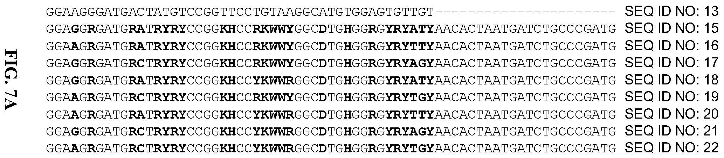

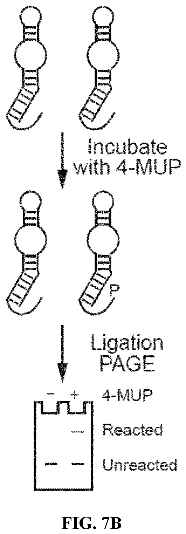

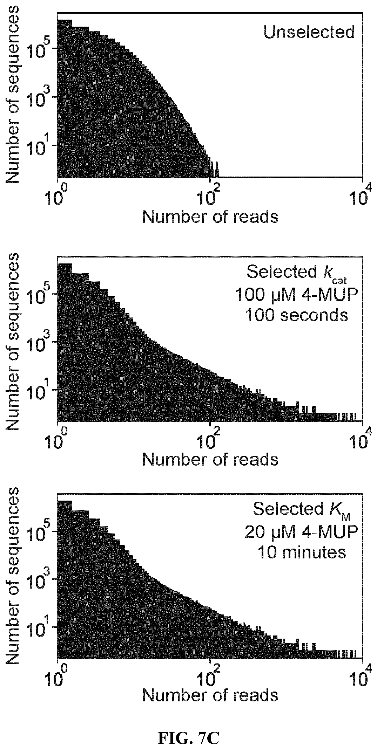

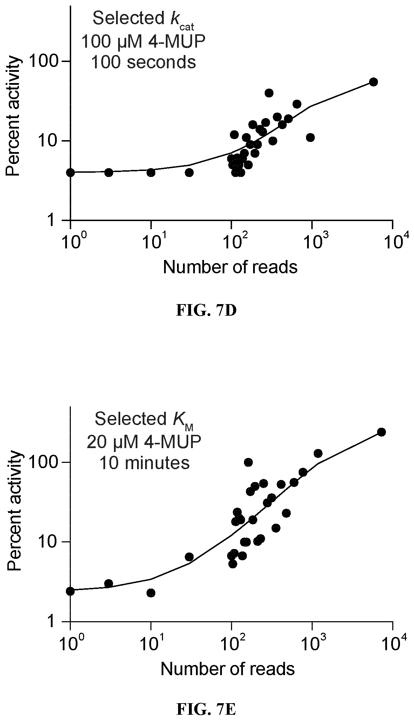

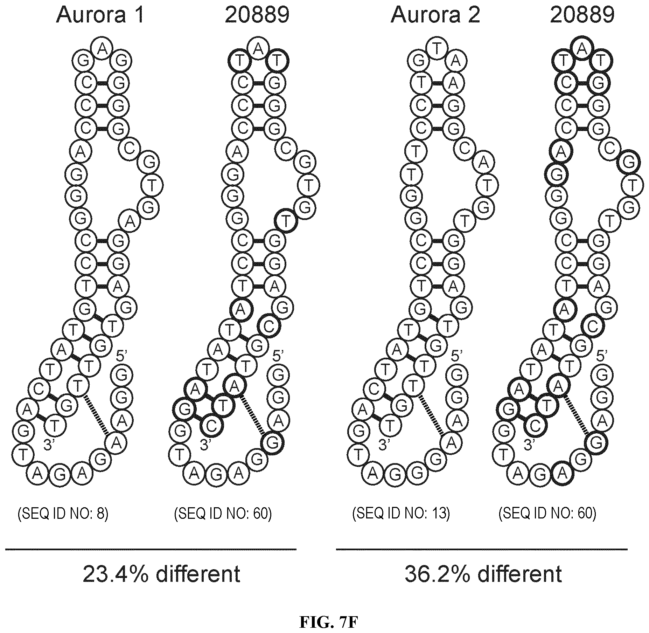

**FIGs. 7A-7E** show the characterization of Aurora using a secondary structure library. **FIG. 7A** shows the design of a secondary structure library based on Aurora. The sequence of Aurora 2 (SEQ ID NO: 13) is shown for reference. The eight oligonucleotides shown below Aurora 2 (SEQ ID NOs: 15-22) were synthesized separately and mixed in equal ratios to generate the library. Variable positions are shown in bold. R = A or G; Y = C or T; H = A or C or T; K = G or T; D = A or G or T; W = A or T. **FIG. 7B** shows the workflow of single-step selection experiments. **FIG. 7C** shows the distribution of read numbers in the starting library (unselected), in a library incubated for a short time (100 seconds) in the presence of a saturating concentration of 4-MUP (100 µM) to favor variants with improved *k*_{cat} values, and in a library incubated for a longer time (10 minutes) in the presence of a sub-saturating concentration of 4-MUP (20 µM) to favor variants with improved *K*_{M} values. **FIG. 7D** shows the correlation between read number and catalytic activity after selection for variants with improved *k_{cat}* values. The library was incubated for 100 seconds in the presence of 100 µM 4-MUP. Percent activity is relative to Aurora 2 under the same conditions as those used in the selection. **FIG. 7E** shows the correlation between read number and catalytic activity after selection for variants with improved *K*_{M} values. The library was incubated for 10 minutes in the presence of 20 µM 4-MUP. Percent activity is relative to Aurora 2 under the same conditions as those used in the selection. **FIG. 7F** shows the sequence of Aurora variant 20889 (SEQ ID NO: 60; isolated from the secondary structure library) relative to the sequences of Aurora 1 (23.4% sequence difference) and Aurora 2 (36.2% sequence difference).

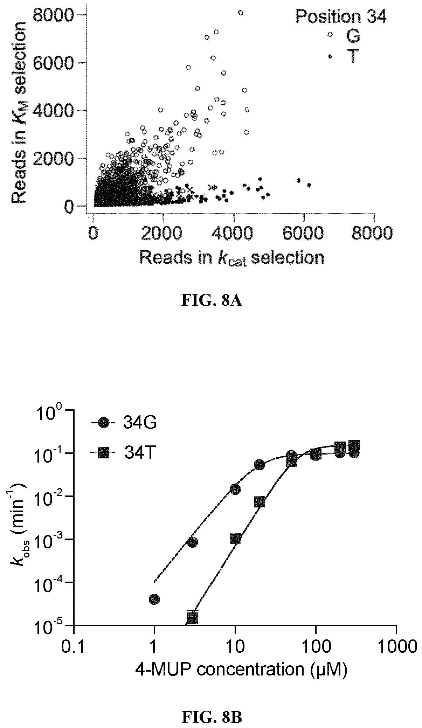

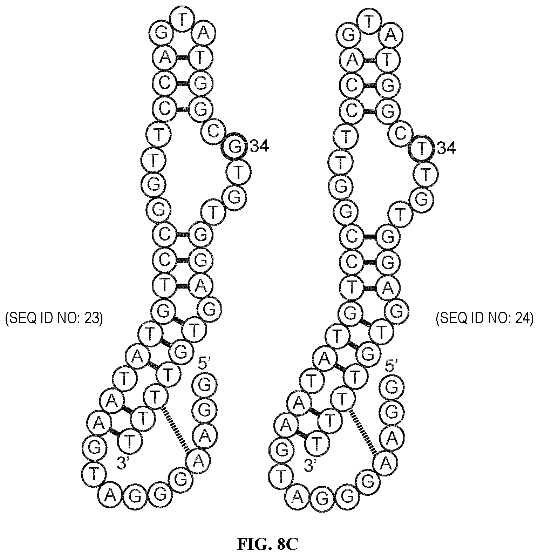

**FIGs. 8A-8C** show that secondary structure libraries and single step selections were used to identify variants of Aurora with new properties. **FIG. 8A** shows the numbers of sequences after a single-step selection for variants of Aurora with improved *k*_{cat} values (*x*-axis) and after a single-step selection for variants of Aurora with improved *K*_{M} values (*y*-axis). Note that the slope of the line formed by variants containing T at position 34 is different from the slope of the line formed by variants containing G at position 34. **FIG. 8B** shows catalytic activity of an Aurora variant containing 34G (circles) or 34T (squares) over a range of 4-MUP concentrations. **FIG. 8C** shows 34G and 34T variants of Aurora used in the experiments described in FIG. 8B.

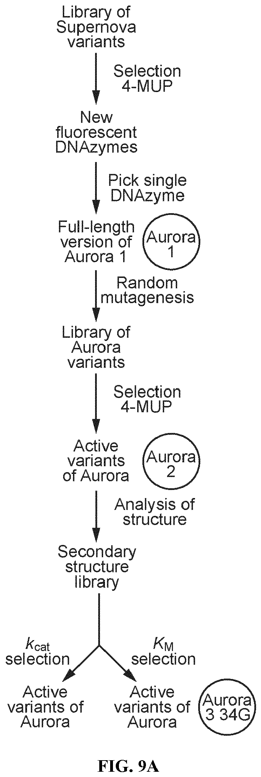

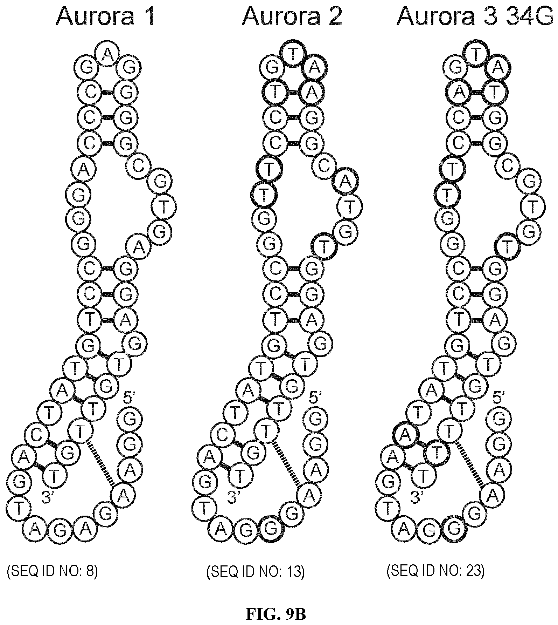

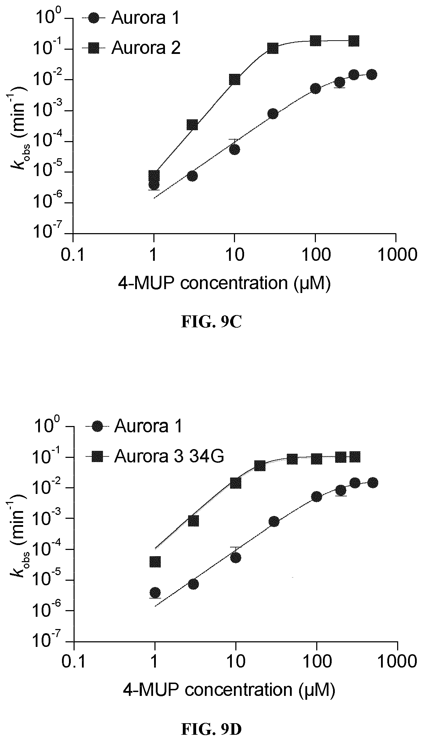

**FIGs. 9A-9D** show Aurora variants with improved catalytic properties. **FIG. 9A** shows the evolutionary lineage of Aurora 1 (the minimized catalytic core of the initial isolate of Aurora), Aurora 2 (the variant with the highest read number from the randomly mutagenized pool), and Aurora 3 (the variant with the highest read number from the secondary structure library). **FIG. 9B** shows the sequence and secondary structure model of Aurora 1, Aurora 2, and Aurora 3. Positions that differ from Aurora 1 are shown with bold outlines. **FIG. 9C** shows the catalytic activity of Aurora 1 (the initial isolate of Aurora) compared to that of Aurora 2 (the variant with the highest read number from the randomly mutagenized pool) over a range of substrate concentrations. **FIG. 9D** shows the catalytic activity of Aurora 1 (the initial isolate of Aurora) compared to that of Aurora 3 (the variant with the highest read number from the selection for variants with improved *K*_{M} values which used the secondary structure library) over a range of substrate concentrations. Rates were measured using the ligation assay.

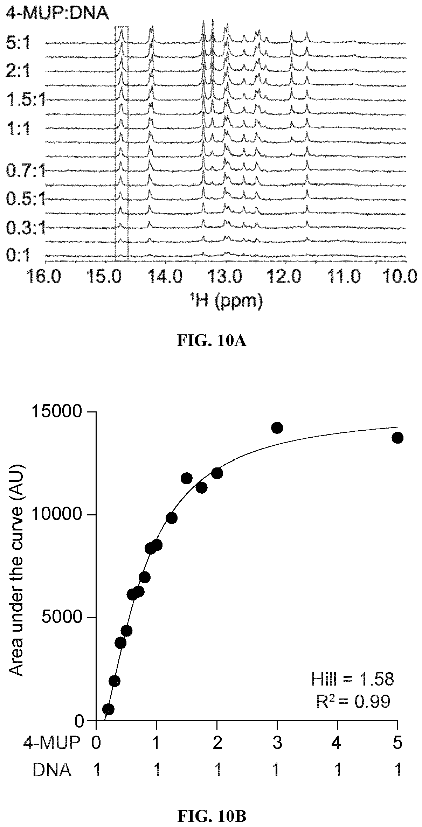

**FIGs. 10A-10B** show the cooperative effect of 4-MUP on Aurora folding. **FIG. 10A** shows the proton NMR spectra of the 17C 40G variant of Aurora over a range of 4-MUP concentrations. Spectra were measured in the presence of 500 µM DNA in a buffer containing 200 mM KCl, 1 mM ZnCl₂, 5% (v/v) DMSO, 50 mM HEPES, pH 7.4, and 0, 100, 200, 300, 400, 500, or 600 µM 4-MUP. **FIG. 10B** is a graph showing the area under the curve of the first peak from the left in FIG. 10A. The curve was fit using the Hill equation.

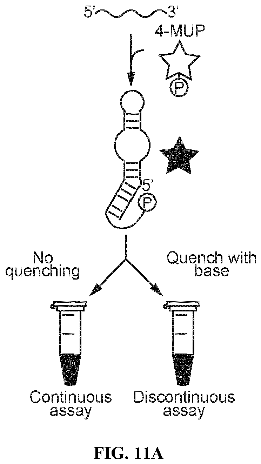

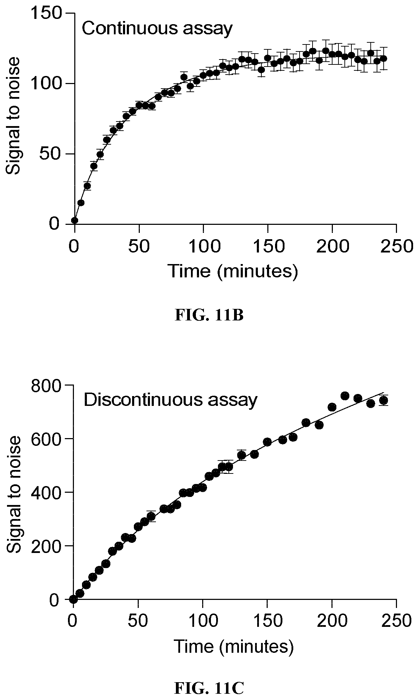

**FIGs. 11A-11C** shows the maximum signal-to-noise ratio of fluorescence of Aurora in continuous and discontinuous assays. **FIG. 11A** shows workflows for continuous and discontinuous assays. **FIG. 11B** shows the assay in which Aurora 2 was mixed with 4-MUP and fluorescence was measured continuously using a plate reader. **FIG. 11C** shows the assay in which Aurora 2 was mixed with 4-MUP and time points were quenched with 20 µl of 1 M KOH before measuring fluorescence. Reactions were performed using 15 µM Aurora and 30 µM 4-MUP in a buffer containing 200 mM KCl, 1 mM ZnCl₂, 5% (v/v) DMSO, and 50 mM HEPES, pH 7.4, and fluorescence was measured using a TECAN Spark plate reader. Signal-to-noise ratio is defined as the rate of production of fluorescence in the presence of deoxyribozyme divided by the rate in the absence of deoxyribozyme. Points show the values from three experiments, and error bars represent one standard deviation.

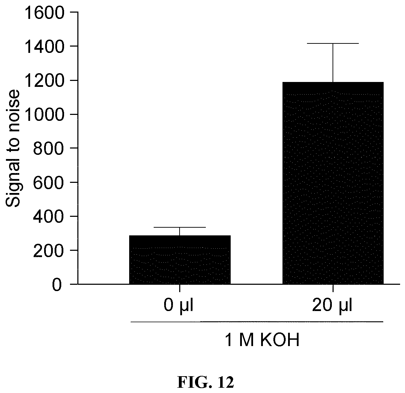

**FIG. 12** shows the signal-to-noise ratio of fluorescence of synthetic 4-MU in the absence and presence of base. Samples contained 15 µM 4-MU or 15 µM 4-MUP and 200 mM KCl, 1 mM ZnCl₂, 5% (v/v) DMSO and 50 mM HEPES, pH 7.4. After incubating for 4 hours, either 0 µl or 20 µl of 1 M KOH was added, and fluorescence was measured using a TECAN Spark plate reader. Signal-to-noise ratio is defined as the rate of fluorescence production in the presence of 4-MU divided by the rate of fluorescence production in the presence of 4-MUP. Columns show the average of at least three experiments, and error bars represent one standard deviation.

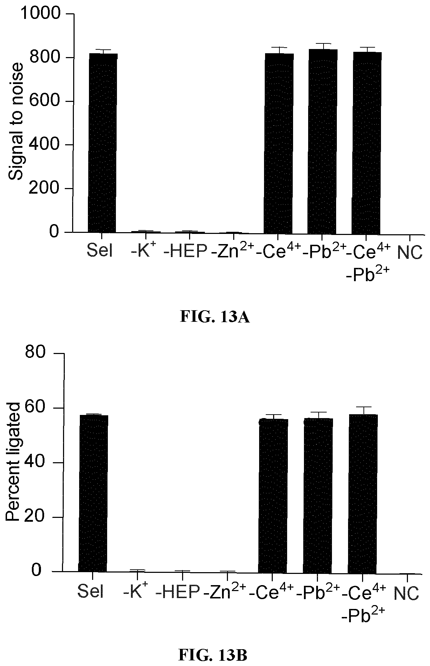

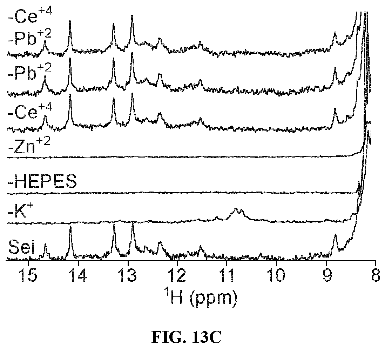

**FIGs. 13A-13C** show that Aurora requires potassium and zinc for activity and folding. **FIG. 13A** shows the signal-to-noise ratio of Aurora in a series of buffers in which different components of the original selection buffer was omitted. The original selection buffer (labeled "Sel") contained 200 mM KCl, 1 µM CeCl₃, 0.1 µM PbCl₃, 1 mM ZnCl₂, 5%(v/v) DMSO, and 50 mM HEPES, pH 7.4. Reactions were performed using 15 µM Aurora 2 and 30 µM 4-MUP. After incubating for 4 hours, 20 µl of 1 M KOH was added and fluorescence was measured using a TECAN Spark plate reader. **FIG. 13B** is the same experiment as in FIG. 13A but shows the percent of phosphorylated Aurora measured using the ligation assay. The percent ligated was determined after incubating 1 µM Aurora 2 with 1 mM 4-MUP for 1 hour. Columns show the values from at least three experiments, and error bars represent one standard deviation. Signal-to-noise ratio is defined as the rate of fluorescence production in the presence of deoxyribozyme divided by the rate of fluorescence production in the absence of deoxyribozyme. **FIG. 13C** shows the proton NMR spectra of Aurora in the same buffers used in FIG. 13A. Spectra were measured using 300 µM Aurora and 450 µM 4-MUP.

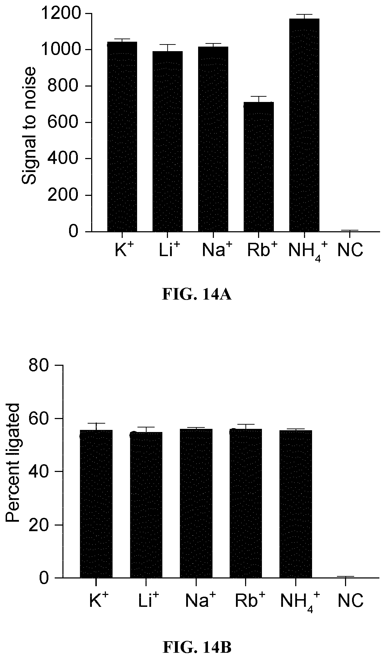

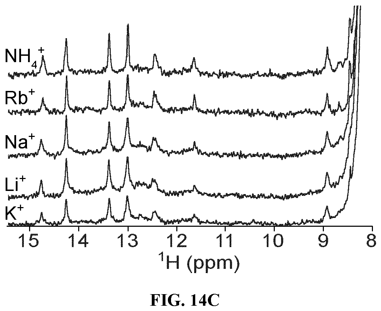

**FIGs. 14A-14C** show Aurora is active in a wide range of monovalent metal ions. **FIG. 14A** shows the replacement of potassium with other monovalent metal ions. Buffers contained the indicated monovalent cation at a concentration of 200 mM, 1 mM ZnCl₂, 5% (v/v) DMSO, and 50 mM HEPES, pH 7.4. The reaction was performed using 15 µM Aurora 2 and 30 µM 4-MUP. After incubating for 4 hours, 20 µl of 1 M KOH was added and fluorescence was measured using a TECAN Spark plate reader. **FIG. 14B** is the same experiment as in FIG. 14A but shows the percent of phosphorylated Aurora measured using the ligation assay. The percent ligated was determined after incubating 1 µM Aurora 2 with 1 mM 4-MUP for 1 hour. Columns show the values from at least three experiments, and error bars represent one standard deviation. Signal-to-noise ratio is defined as the rate of fluorescence production in the presence of deoxyribozyme divided by the rate of fluorescence production in the absence of deoxyribozyme. **FIG. 14C** shows the proton NMR spectra of Aurora in the same buffers used in FIG. 14A. Spectra were measured using 300 µM Aurora and 450 µM 4-MUP.

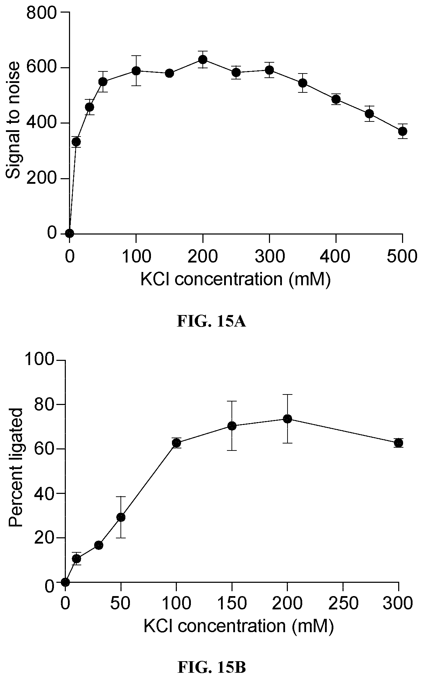

**FIGs. 15A-15B** show the dependence of Aurora activity on KCl concentration. **FIG. 15A** shows the signal-to-noise ratio of Aurora over a range of potassium concentrations. Buffers contained the indicated concentration of KCl, 1 mM ZnCl₂, 5% (v/v) DMSO, and 50 mM HEPES, pH 7.4. Reactions were performed using 15 µM Aurora 2 and 30 µM 4-MUP. After incubating for 4 hours, 20 µl of 1 M KOH was added and fluorescence was measured using a TECAN Spark plate reader. Signal-to-noise ratio is defined as the rate of fluorescence production in the presence of deoxyribozyme divided by the rate of fluorescence production in the absence of deoxyribozyme. **FIG. 15B** is the same experiment as in FIG. 15A but shows the percent of phosphorylated Aurora measured by the ligation assay. The percent ligated was determined after incubating 1 µM Aurora with 1 mM 4-MUP for 1 hour. Points show the average of three experiments, and error bars represent one standard deviation.

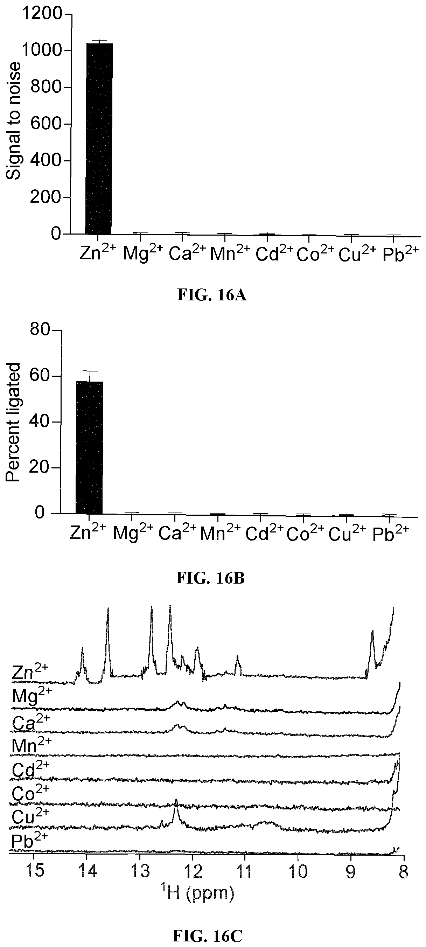

**FIGs. 16A-16C** show that Aurora is active in the presence of zinc, but not in the presence of other divalent metal ions. **FIG. 16A** shows the replacement of zinc with other divalent metal ions. Buffers contained 1 mM of the indicated divalent cation, 200 mM KCl, 5% (v/v) DMSO, and 50 mM HEPES, pH 7.4. Reactions were performed using 15 µM Aurora 2 and 30 µM 4-MUP. After incubating for 4 hours, 20 µl of 1 M KOH was added, and fluorescence was measured using a TECAN Spark plate reader. **FIG. 16B** is the same experiment as in FIG. 16A but shows the percent of phosphorylated Aurora measured using the ligation assay. The percent ligated was determined after incubating 1 µM Aurora 2 with 1 mM 4-MUP for 1 hour. Signal-to-noise ratio is defined as the rate of fluorescence production in the presence of deoxyribozyme divided by the rate of fluorescence production in the absence of deoxyribozyme. Columns show the values from at least three experiments, and error bars represent one standard deviation. **FIG. 16C** shows the proton NMR spectra of Aurora in the same buffers used in FIG. 16A. Spectra were measured using 300 µM Aurora and 450 µM 4-MUP.

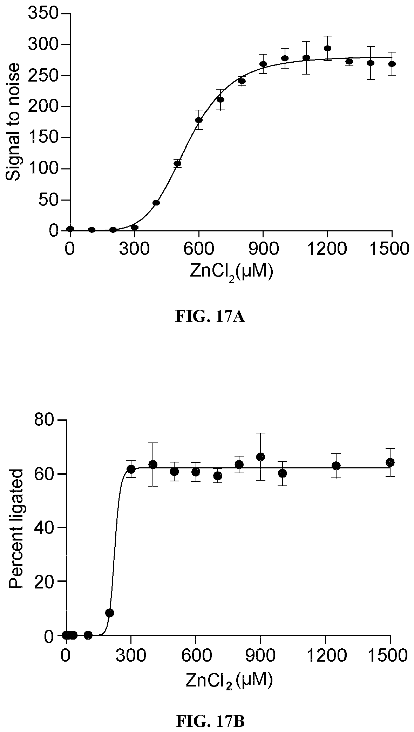

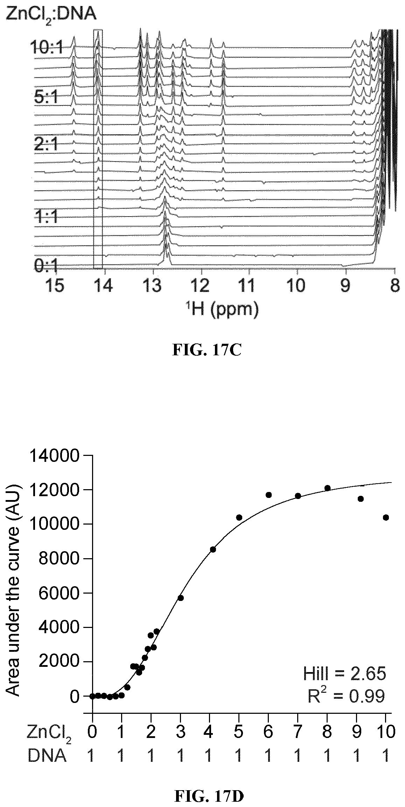

**FIGs. 17A-17D** show the cooperative effect of zinc on Aurora catalytic activity. **FIG. 17A** shows the signal-to-noise ratio of Aurora as a function of zinc concentration. Buffers contained the indicated concentration of ZnCl₂, 200 mM KCl, 5% (v/v) DMSO, and 50 mM HEPES, pH 7.4. Reactions were performed using 15 µM Aurora 2 and 30 µM 4-MUP. After incubating for 4 hours, 20 µl of 1 M KOH was added, and fluorescence was measured using a TECAN Spark plate reader. Signal-to-noise ratio is defined as the rate of fluorescence production in the presence of deoxyribozyme divided by the rate of fluorescence production in the absence of deoxyribozyme. **FIG. 17B** is the same experiment as in FIG. 17A but shows the percent of phosphorylated Aurora measured using the ligation assay. The percent ligated was determined after incubating 1 µM Aurora 2 with 1 mM 4-MUP for 1 hour. Points show the average of at least three experiments, and error bars represent one standard deviation. **FIG. 17C** shows the proton NMR spectra of the 17C 40G variant of Aurora over a series of concentrations of ZnCl₂. Spectra were measured in the presence of 500 µM DNA, 750 µM 4-MUP, 200 mM KCl, 5% (v/v) DMSO, and 50 mM HEPES, pH 7.4, and different concentrations of ZnCl₂. **FIG. 17D** is a graph showing the area under the curve of the second peak from the left (indicated with a box) in FIG. 17C.

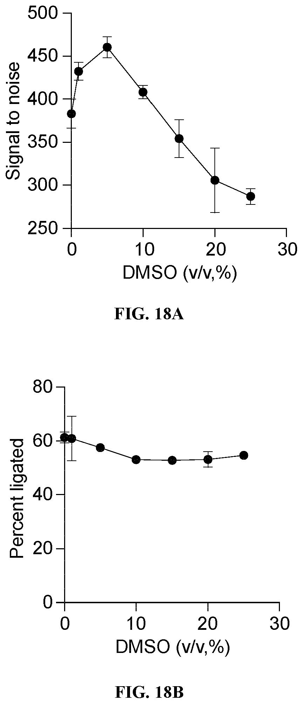

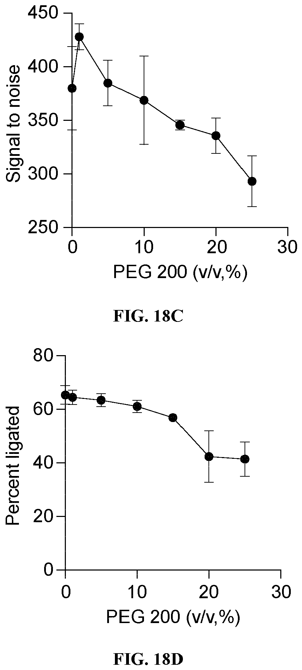

**FIGs. 18A-18D** show the effect of molecular crowding agents and organic solvents on the catalytic activity of Aurora. **FIG. 18A** shows the signal-to-noise ratio of Aurora over a range of DMSO concentrations. Buffers contained the indicated concentration of DMSO, 200 mM KCl, 1 µM CeCl₃, 0.1 µM PbCl₃, 1 mM ZnCl₂, and 50 mM HEPES, pH 7.4. Reactions were performed using 15 µM Aurora 2 and 30 µM 4-MUP. After incubating for 4 hours, 20 µl of 1 M KOH was added, and fluorescence was measured using a TECAN Spark plate reader. **FIG. 18B** is the same experiment as in FIG. 18A but shows the percent of phosphorylated Aurora measured using the ligation assay. The percent ligated was determined after incubating 1 µM Aurora with 1 mM 4-MUP for 1 hour. **FIG. 18C** is the signal-to-noise ratio of Aurora over a range of PEG 200 concentrations. Buffers contained the indicated concentration of PEG 200, 200 mM KCl,1 µM CeCl₃, 0.1 µM PbCl₃, 1 mM ZnCl₂, and 50 mM HEPES, pH 7.4. Reactions were performed using 15 µM Aurora and 30 µM 4-MUP. After incubating for 4 hours, 20 µl of 1 M KOH was added, and fluorescence was measured using a TECAN Spark plate reader. **FIG. 18D** is the same experiment as in FIG. 18C but shows the percent of phosphorylated Aurora measured using the ligation assay. The percent ligated was determined after incubating 1 µM Aurora 2 with 1 mM 4-MUP for 1 hour. Signal-to-noise ratio is defined as the rate of fluorescence production in the presence of deoxyribozyme divided by the rate of fluorescence production in the absence of deoxyribozyme. Points show the values from three experiments, and error bars represent one standard deviation.

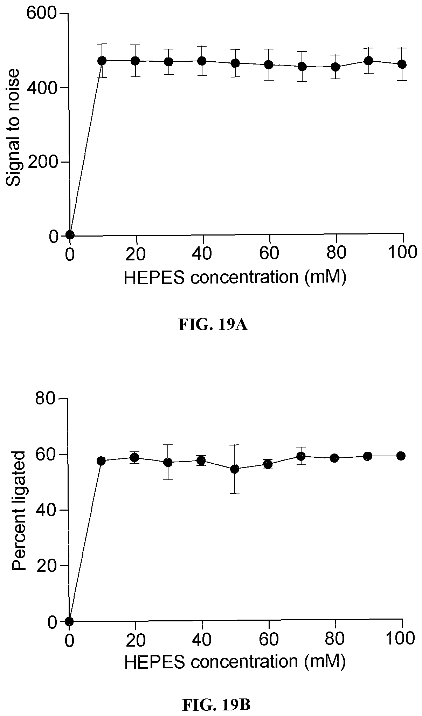

**FIGs. 19A-19B** show the dependence of Aurora activity on HEPES concentration. **FIG. 19A** shows the signal-to-noise ratio of Aurora as a function of HEPES concentration. Buffers contained the indicated concentration of HEPES, pH 7.4, 200 mM KCl, 1 mM ZnCl₂, and 5% (v/v) DMSO. Reactions were performed using 15 µM Aurora 2 and 30 µM 4-MUP. After incubating for 4 hours, 20 µl of 1 M KOH was added and fluorescence was measured using a TECAN Spark plate reader. Signal-to-noise ratio is defined as the rate of fluorescence production in the presence of deoxyribozyme divided by the rate of fluorescence production in the absence of deoxyribozyme. **FIG. 19B** is the same experiment as in FIG. 19A but shows the percent of phosphorylated Aurora measured using the ligation assay. The percent ligated was determined after incubating 1 µM Aurora 2 with 1 mM 4-MUP for 1 hour. Points show the average of three experiments, and error bars represent one standard deviation.

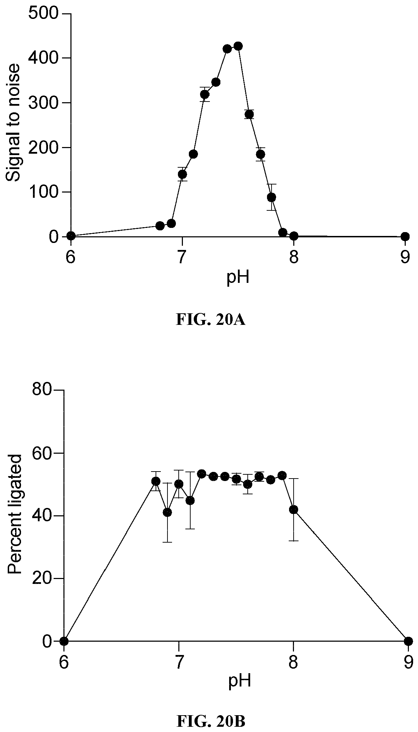

**FIGs. 20A-20B** show the effect of pH on Aurora catalytic activity. **FIG. 20A** shows the signal-to-noise ratio of Aurora as a function of pH. Buffers contained 50 mM HEPES at the indicated pH, 200 mM KCl, 1 mM ZnCl₂, and 5% (v/v) DMSO. Reactions were performed using 15 µM Aurora 2 and 30 µM 4-MUP. After incubating for 4 hours, 20 µl of 1 M KOH was added, and fluorescence was measured using a TECAN Spark plate reader. Signal-to-noise ratio is defined as the rate of fluorescence production in the presence of deoxyribozyme divided by the rate of fluorescence production in the absence of deoxyribozyme. **FIG. 20B** is the same experiment as in FIG. 20A but shows the percent of phosphorylated Aurora measured using the ligation assay. The percent ligated was determined after incubating 1 µM Aurora 2 with 1 mM 4-MUP for 1 hour. Points show the average of at least three experiments, and error bars represent one standard deviation.

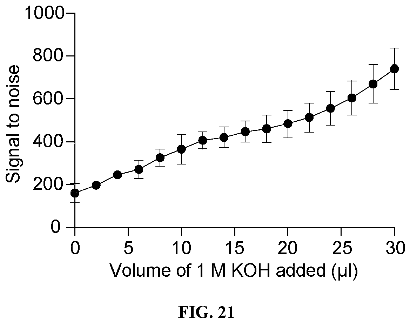

**FIG. 21** shows that the signal generated by Aurora can be enhanced by adding base before measuring fluorescence. Effect of pH adjustment on the signal-to-noise ratio of Aurora. Buffer contained 200 mM KCl, 1 µM CeCl₃, 0.1 µM PbCl₃, 1 mM ZnCl₂, and 50 mM HEPES, pH 7.4. Reactions were performed using 15 µM Aurora 2 and 30 µM 4-MUP. After incubating for 4 hours, the indicated amount of 1 M KOH was added, and fluorescence was measured using a TECAN Spark plate reader. Signal-to-noise ratio is defined as the rate of fluorescence production in the presence of deoxyribozyme divided by the rate of fluorescence production in the absence of deoxyribozyme. Points show the values from at least three experiments, and error bars represent one standard deviation.

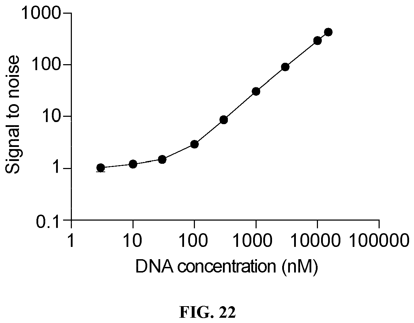

**FIG. 22** shows the detection limit of Aurora. Signal-to-noise ratio of fluorescence production over a range of concentrations of Aurora. Reactions were performed using the indicated concentration of Aurora 2 and 30 µM 4-MUP in a buffer containing 200 mM KCl, 1 mM ZnCl₂, 5% (v/v) DMSO, and 50 mM HEPES, pH 7.4. After incubating for 4 hours, 20 µl of 1 M KOH was added, and fluorescence was measured using a TECAN Spark plate reader. Signal-to-noise ratio is defined as the rate of fluorescence production in the presence of deoxyribozyme divided by the rate of fluorescence production in the absence of deoxyribozyme. Points show the average of three experiments, and error bars represent one standard deviation.

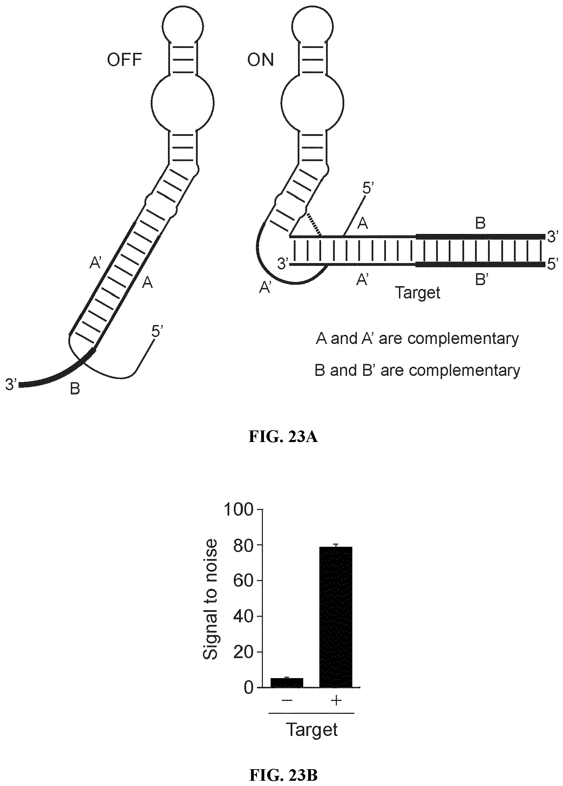

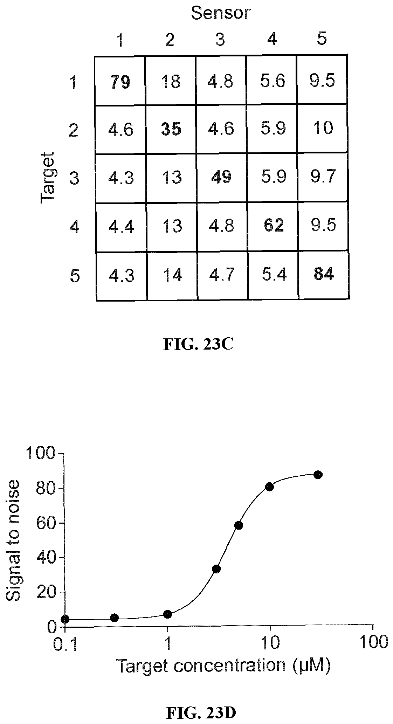

**FIGs. 23A-23D** show coupling Aurora activity to the presence of a ligand. **FIG. 23A** shows a programmable oligonucleotide sensor based on Aurora. In the OFF version of the sensor (left), part of the target sequence is inserted between nucleotides 10 and 11, and the reverse complement of the full target sequence is added to the 3' end of Aurora. Pairing between the inserted sequences forms an inhibitory helix that inactivates Aurora, presumably by increasing the distance between the reaction site (the 5' hydroxyl group) and the catalytic core. In the ON version of the sensor (right), the target oligonucleotide base pairs to the 3' end of Aurora. This prevents formation of the inhibitory helix and restores catalytic activity. **FIG. 23B** shows the production of fluorescence in the absence (left) and presence (right) of the target oligonucleotide. **FIG. 23C** shows the specificity of the sensor. Five different variants of the sensor were generated (SEQ ID NOs: 25-29), each of which was designed to detect a different target oligonucleotide (SEQ ID NOs: 30-34). These sensors generated more fluorescence in the presence of the correct target than in the presence of incorrect targets. **FIG. 23D** shows the detection limit of the sensor. Reactions were incubated for four hours in the presence of the indicated concentration of the target oligonucleotide, and after quenching with base, fluorescence was measured using a plate reader. See Table 1 for the sequences of deoxyribozymes used in the experiments described in this figure.

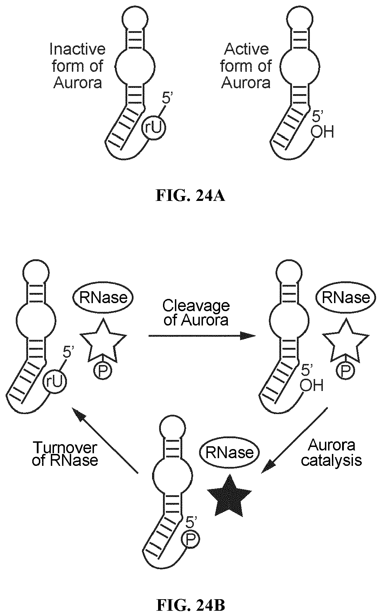

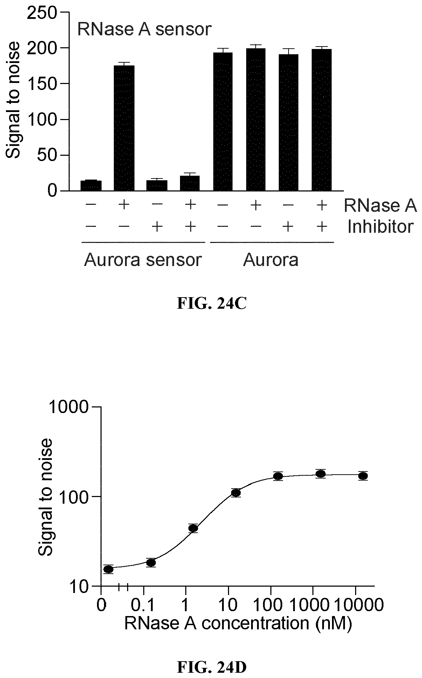

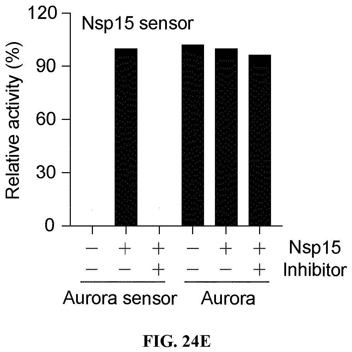

**FIGs. 24A-24E** show a second-generation sensor with 10,000-fold greater sensitivity. **FIG. 24A** shows a ribonuclease sensor based on Aurora. Left: OFF version of the sensor in which the reaction site (the 5' hydroxyl group) is blocked by a short oligonucleotide with a single ribonucleotide at its 3' end. Right: ON version of the sensor in which the blocking oligonucleotide has been cleaved to regenerate the reaction site. **FIG. 24B** shows how the signal of the sensor can be amplified by multiple turnover cleavage promoted by the target ribonuclease. **FIG. 24C** shows activation of the Aurora RNase A sensor (SEQ ID NO: 35) by RNase A. Left: the Aurora sensor is activated by RNase A, but not when the RNase inhibitor RiboLock is present. Right: neither RNase A nor RiboLock affects the catalytic activity of unmodified Aurora. Reactions were performed using 5 µM Aurora or Aurora sensor and 30 µM 4-MUP in a buffer containing 200 mM KCl, 1 mM ZnCl₂, 5% (v/v) DMSO, and 50 mM HEPES, pH 7.4. Reactions contained either 0 µM or 0.5 µM RNase A and either 0 µM or 0.5 µM Ribolock RNase inhibitor. After incubating for 4 hours, 20 µl of 1 M KOH was added, and fluorescence was measured using a TECAN Spark plate reader. Signal-to-noise ratio is defined as the rate of fluorescence production in the presence of deoxyribozyme divided by the rate of fluorescence production in the absence of deoxyribozyme. Each columns shows the average value from three experiments, and error bars represent one standard deviation. **FIG. 24D** shows the detection limit of the RNase A sensor. Reactions were incubated for 4 hours in the presence of the indicated concentration of RNase A, and after quenching with base, fluorescence was measured using a plate reader. **FIG. 24E** shows activation of the Aurora Nsp15 sensor (SEQ ID NO: 36) by Nsp15. Left columns of graph: the Aurora Nsp15 sensor is activated by Nsp15, but not when an Nsp15 inhibitor is present. Right columns of graph: neither Nsp15 nor the inhibitor affects the catalytic activity of unmodified Aurora. N sp 15 cleavage reactions were performed using 25 µM of either Aurora sensor or unmodified Aurora, 400 nM Nsp15, and either 0 µM or 200 µM inhibitor in a buffer containing 50 mM KCl, 20 mM HEPES pH 7.4, 5 mM MnCl₂, and 0.003% (v/v) Tween20 in a volume of 20 µl. After incubating at room temperature for 1 hour, 80 µl of a new buffer was added to initiate Aurora catalysis. Final conditions were 5 µM of either Aurora Nsp15 sensor or unmodified Aurora, 80 nM Nsp15, and either 0 µM or 40 µM inhibitor in a buffer containing 200 mM KCl, 1 mM ZnCl₂, 5% (v/v) DMSO, and 50 mM HEPES, pH 7.4. After incubating for 4 hours, fluorescence was measured using a TECAN Spark plate reader. Activity is relative to that of unmodified Aurora.

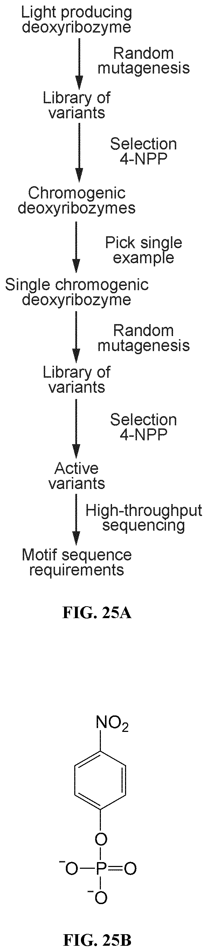

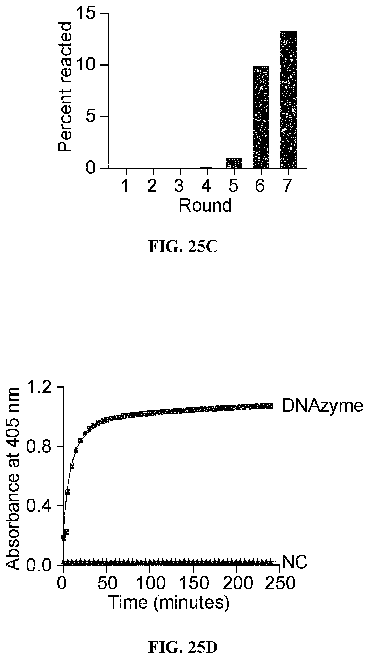

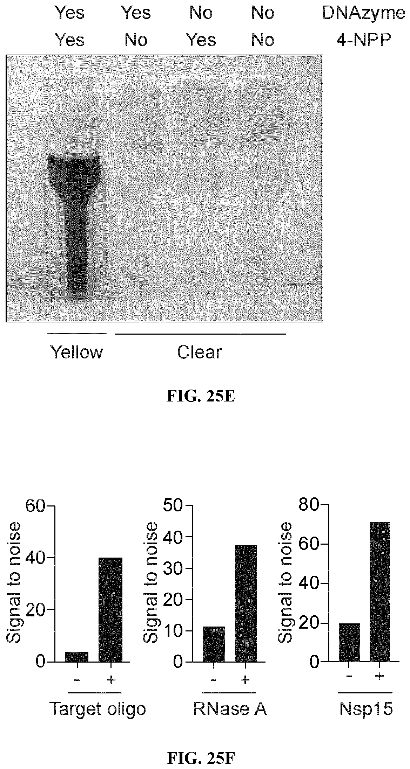

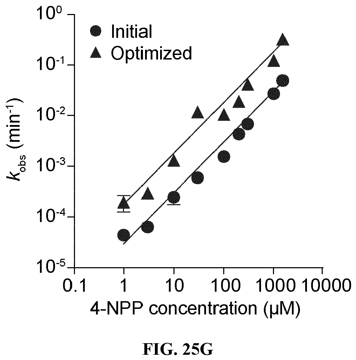

FIGs. 25A-G describe the identification and characterization of a deoxyribozyme that generates a chromogenic product. **FIG. 25A** shows the workflow used to identify and optimize chromogenic deoxyribozymes. **FIG. 25B** shows the chemical structure of the 4-NPP substrate used by chromogenic deoxyribozymes. **FIG. 25C** shows the activity of the library after each round of the initial selection. **FIG. 25D** shows a time course of formation of the product 4-NP catalyzed by a chromogenic deoxyribozyme. The reaction was performed using 300 µM deoxyribozyme (SEQ ID NO: 39) and 450 µM 4-NPP in 200 mM KCl, 1 mM ZnCl₂, and 50 mM HEPES pH 7.4. The deoxyribozyme was folded at 30 µM, and after concentrating to 300 µM using an Amicon Ultra Centrifugal Filter Unit (Sigma-Aldrich), 4-NPP was added and aborbance was measured at 405 nm for 4 hours using a Tecan Spark plate reader. **FIG. 25E** shows that the product produced by this deoxyribozyme (SEQ ID NO: 39) can be detected by eye. Conditions were the same as in FIG. 25D. The photograph shows the reaction after a 4-hour incubation. **FIG. 25F** shows the activities of engineered versions of this deoxyribozyme that only produce a product in the presence of another molecule. Left: a deoxyribozyme that only produce a yellow product in the presence of a target oligonucleotide with a specific sequence. Reactions were performed using 50 µM deoxyribozyme sensor (SEQ ID NO: 43), 100 µM 4-NPP, and 100 µM target oligonucleotide (SEQ ID NO: 44) in a buffer containing 200 mM KCl, 1 mM ZnCl₂, and 50 mM HEPES, pH 7.4. Middle: a deoxyribozyme sensor (SEQ ID NO: 45) that only produce a yellow product in the presence of the ribonuclease RNase A. Right: a deoxyribozyme sensor (SEQ ID NO: 46) that only produce a yellow product in the presence of the ribonuclease Nsp15. Final concentrations were 30 µM of the ribonuclease sensor, 300 nM RNase A or 5000 nM Nsp15, 1× RNase A buffer (200 mM KCl, 50 mM HEPES pH 7.4, 1 mM ZnCl2) or 1× Nsp15 buffer (100 mM KCl, 1 mM ZnCl₂, 5 mM MnCl2, and 20 mM HEPES, pH 7.4), and 100 µM 4-NPP. Signal to noise is defined as the absorbance at 405 nm in the presence of deoxyribozyme divided by the absorbance at 405 nm in the absence of deoxyribozyme. **FIG. 25G** shows the improvement obtained after mutagenizing this chromogenic deoxyribozyme at a rate of 21% per positions and performing a reselection for variants with higher catalytic efficiencies. Reactions were performed using 1 µM deoxyribozyme and the indicated concentration of 4-NPP in a buffer containing 200 mM KCl, 1 mM ZnCl₂, and 50 mM HEPES, pH 7.4. Incubation times varied from 10 seconds to 300 min. After stopping with EDTA, time points were ethanol precipitated and analyzed using the ligation assay. Only the linear phase of each reaction was used to calculate rates. Experiments were performed using the deoxyribozymes SEQ ID NO: 41 (labeled "initial") and SEQ ID NO: 39 (labeled "optimized").

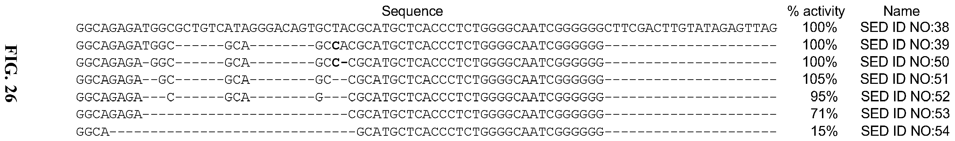

**FIG. 26** shows the effects of various deletions on catalytic activity of the chromogenic deoxyribozyme. Reactions were performed at 30 µM DNA and 100 µM 4-NPP in a buffer containing 200 mM KCl, 1 mM ZnCl₂, and 50 mM HEPES pH 7.4., and were incubated for 4 hours before measuring absorbance at 405 nm.

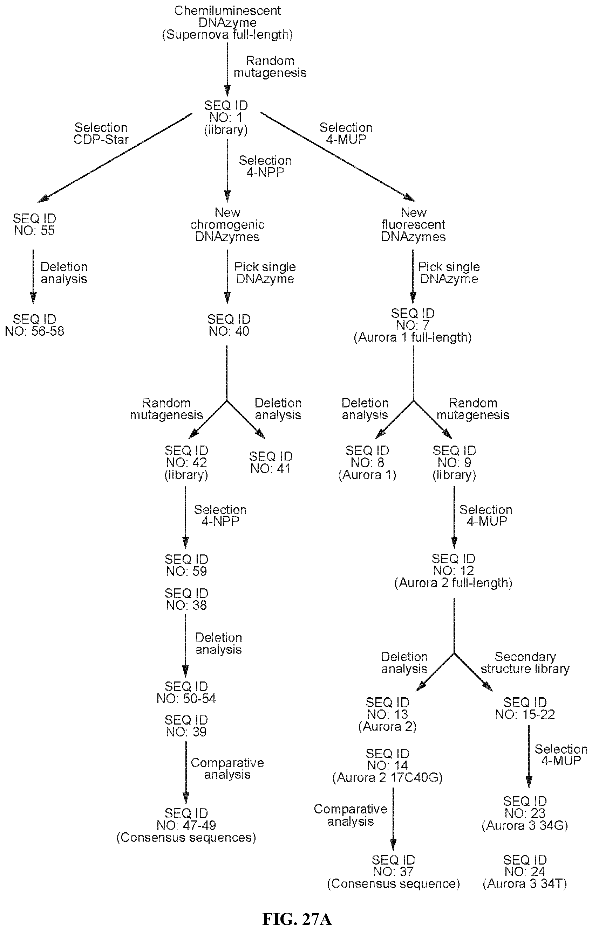

FIGs. 27A-D show the substrate specificities of representative deoxyribozymes described in this patent application.

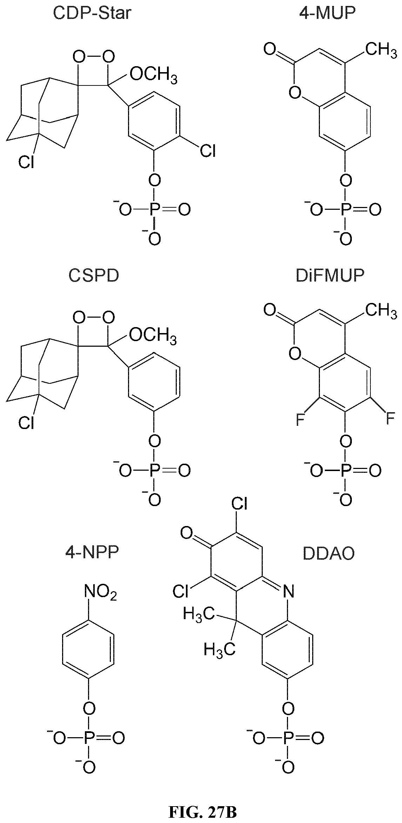

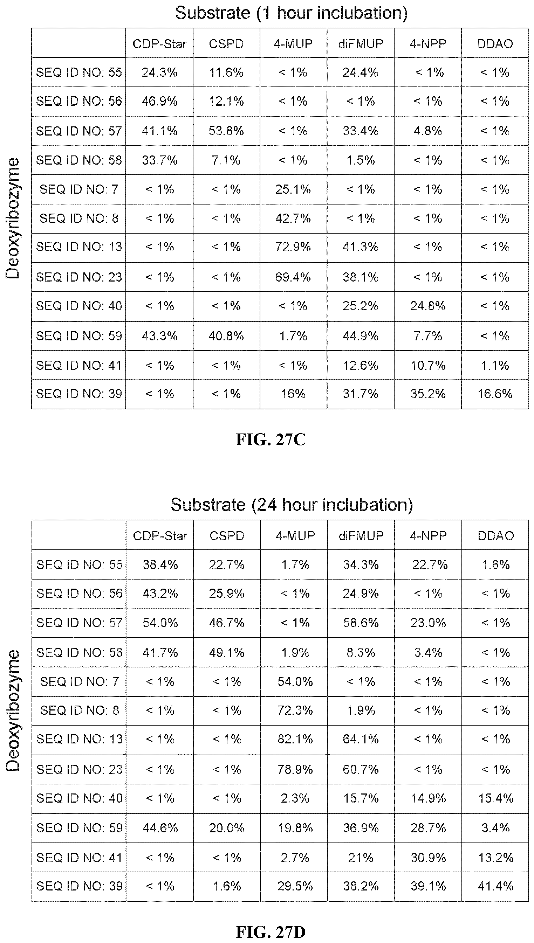

**FIG. 27A** provides a summary of the various selections used to isolate deoxyribozymes with different substrate specificities. **FIG. 27B** shows the chemical structures of the panel of substrates used in these experiments. **FIG. 27C** shows the extent to which representative deoxyribozymes react with the substrates shown in Fig. 27B in 1-hour incubations. **FIG. 27D** shows the extent to which representative deoxyribozymes react with the substrates shown in Fig. 27B in 24-hour incubations. Reactions were performed using 1 µM deoxyribozyme in a buffer containing 200 mM KCl, 1 mM ZnCl₂, 50 mM HEPES pH 7.4, and 1000 µM of the indicated substrate. After incubating for 1 or 24 hours, reactions were stopped with EDTA, ethanol precipitated and analyzed using the ligation assay.

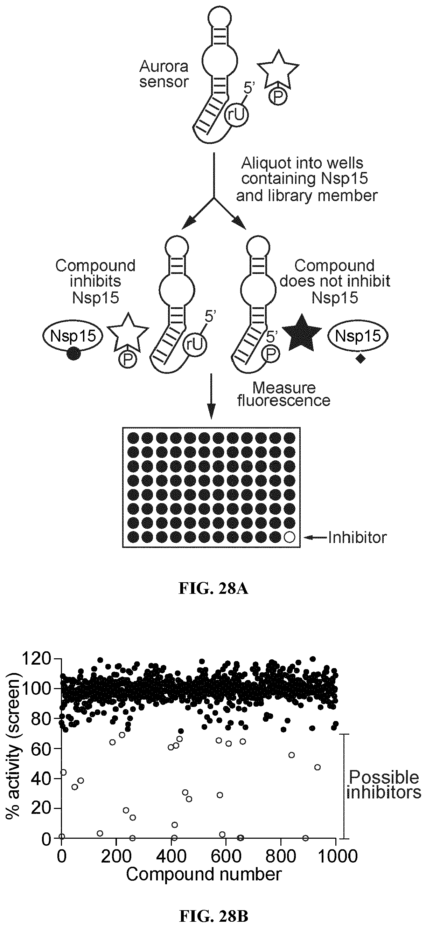

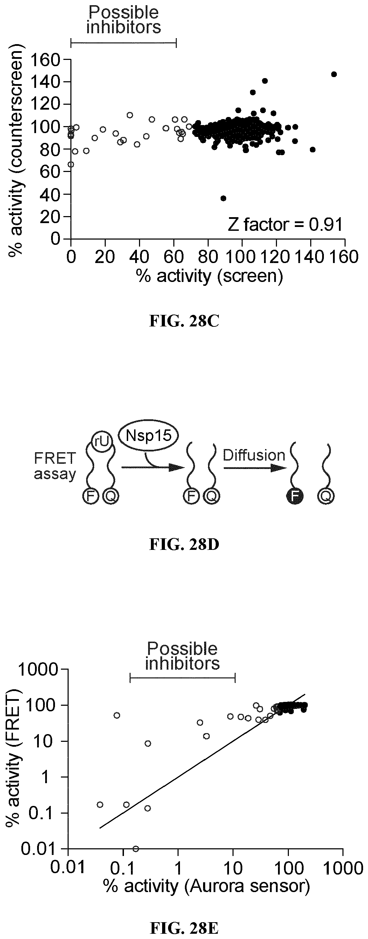

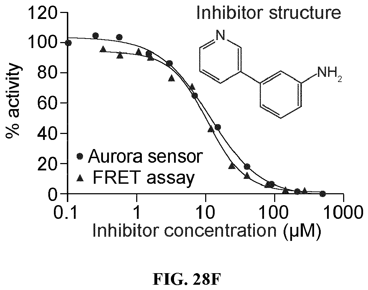

FIGs. 28A-F show the results of a high-throughput screen for RNase inhibitors using an Aurora sensor. **FIG. 28A** shows the workflow of the screen. See Table 1 for the sequence of the Aurora Nsp15 sensor (SEQ ID NO: 36). **FIG. 28B** shows the effect of each compound in a 1000-member fragment library on the fluorescence of the Aurora Nsp15 sensor. Potential inhibitors are shown using open circles within the bracket. **FIG. 28C** shows the results of a counterscreen of the library using Aurora rather than the Aurora sensor to distinguish Nsp15 inhibitors from false positives. Each point corresponds to a different compound in a 1000-member fragment library. The x-axis shows the fluorescent signal generated by the Aurora Nsp15 sensor in the presence of Nsp15 and different compounds in the library. The y-axis shows the fluorescent signal generated by Aurora itself in the presence of Nsp 15 and the same compounds. Potential inhibitors identified in the initial screen are shown using open circles within the bracket. Note that none of these compounds inhibit Aurora itself, suggesting that they are Nsp15 inhibitors. **FIG. 28D** shows the workflow of a FRET assay for Nsp15 catalytic activity. **FIG. 28E** shows a comparison of the results of a high-throughput screen for Nsp15 inhibitors using the Aurora Nsp15 sensor (x-axis) with those of a screen of the same library using a FRET assay (y-axis). Potential inhibitors identified in the initial screen using the Aurora sensor are shown using open circles within the bracket. **FIG. 28F** shows an example of an Nsp15 inhibitor identified in the screen. This compound inhibits Nsp15 with an IC50 value of 12 µM when measured using the Aurora sensor and 11 µM when measured using the FRET assay.

### DEFINITIONS

Unless defined otherwise, all technical and scientific terms used herein have the meaning commonly understood by a person skilled in the art to which this invention belongs. The following references provide one of skill with a general definition of many of the terms used in this invention: Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991). As used herein, the following terms have the meanings ascribed to them unless specified otherwise.

The term "deoxyribozyme" refers to a DNA oligonucleotide that is capable of performing a chemical reaction. In some embodiments, a deoxyribozyme is capable of catalyzing a chemical reaction. Deoxyribozymes may also be referred to as "DNAzymes" or "DNA enzymes." Exemplary deoxyribozymes include ribonucleases, which catalyze the cleavage of phosphodiester bonds in RNA through a transesterification reaction, and RNA ligases. Deoxyribozymes that catalyze other types of reaction have also been developed, including deoxyribozymes that catalyze DNA phosphorylation, DNA adenylation, DNA deglycosylation, porphyrin metalation, thymine dimer photoreversion, and DNA cleavage. Deoxyribozymes that catalyze the generation of a chemiluminescent signal through the removal of a phosphate group from a small molecule substrate (*i.e.,* Supernova, and variants thereof) have been developed as well (see Svehlova, K., Lukšan, O., Jakubec, M., Curtis, E. A. Supernova: A Deoxyribozyme that Catalyzes a Chemiluminescent Reaction. *Angew. Chem. Int. Ed Engl.* **2022**, 61, e202109347, which is incorporated herein by reference). As described further herein, deoxyribozymes that catalyze new chemical reactions are typically developed using *in vitro* selection methods and/or *in vivo* evolution methods. Deoxyribozymes are described further in Breaker, R. R. DNA enzymes. Nat. Biotechnol. 1997, 15(5), 427-431; Breaker, R., Joyce, G.F. The expanding view of RNA and DNA function. Chemistry & Biology. 2014, 21(9), 1059-1065; Silverman, S. K. Deoxyribozymes: DNA catalysts for bioorganic chemistry. Organic & Biomolecular Chemistry. 2004, 2(19), 2701-2706; Ponce-Salvatierra, A. Crystal structure of a DNA catalyst. Nature. 2016, 529(7585), 231-234; and Silverman, S. K. Catalytic DNA: Scope, Applications, and Biochemistry of Deoxyribozymes. Trends Biochem. Sci. 2016, 41(7), 595-609, each of which is incorporate herein by reference.

The term "fluorescence" refers to the emission of an optical signal (*e.g*., light) by a substance such as a small molecule that has absorbed some form of electromagnetic radiation such as light. In some embodiments, a substance such as a small molecule absorbs electromagnetic radiation of a shorter wavelength and emits light of a longer wavelength. For example, a substance such as a small molecule may absorb radiation when exposed to UV light and subsequently emit visible light. In some embodiments, a substance such as a small molecule absorbs light at a wavelength of approximately 360 nm and emits light at a wavelength of approximately 440 nm (*e.g*., in the case of 4-methylumbelliferone as described further herein). In some embodiments, fluorescence is observed when a deoxyribozyme catalyzes a reaction that chemically alters a substrate. In some embodiments, fluorescence is observed when a deoxyribozyme catalyzes the removal of a phosphate moiety from a substrate.

The term "ion" as used herein refers to an atom with a net electrical charge. In some embodiments, an ion has a net positive electrical charge and may be referred to as a "cation." In some embodiments, an ion has a net negative electrical charge and may be referred to as an "anion." An ion may have, for example, a net electrical charge of +1. Such ions may be referred to as "monovalent ions" or "monovalent cations." Exemplary monovalent ions include, but are not limited to, potassium (K⁺), lithium (Li⁺), sodium (Na⁺), rubidium (Rb⁺), and ammonium (NH₄⁺). Sources of such monovalent ions include salts of these ions, including, for example, KCl, K₂CO₃, LiCl, Li₂CO₃, NaCl, and Na₂CO₃. In some embodiments, an ion has a net electrical charge of +2. Such ions may be referred to as "divalent ions" or "divalent cations." Examplary divalent ions include, but are not limited to, zinc (Zn²⁺), magnesium (Mg²⁺), calcium (Ca²⁺), manganese (Mn²⁺), cadmium (Cd²⁺), cobalt (Co²⁺), copper (Cu²⁺), and palladium (Pd²⁺). Sources of such divalent ions include salts of these ions, including, for example, ZnCl₂, ZnBr₂, ZnI₂, ZnSO₄, and Zn₃(PO₄)₂. In some embodiments, ions (including monovalent and divalent ions) may enhance the catalytic activity of a deoxyribozyme or may be required for the catalytic activity of a deoxyribozyme. In some embodiments, ions may play a role in the structural configuration of a deoxyribozyme.

The terms "oligonucleotide," "polynucleotide," "nucleotide sequence," "nucleic acid," "nucleic acid molecule," and "nucleic acid sequence" refer to a series of nucleotide bases (also called "nucleotides") in DNA and RNA, and mean any chain of two or more nucleotides. The polynucleotides can be chimeric mixtures or derivatives or modified versions thereof, and can be single-stranded or double-stranded. The oligonucleotide can be modified at the base moiety, sugar moiety, or phosphate backbone, for example, to improve stability of the molecule, its hybridization parameters, *etc.* The oligonucleotide may comprise a modified base moiety that is selected from the group including, but not limited to, 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2- dimethylguanine, 2-methyladenine, 2-methylguanine, 3-methylcytosine, 5- methylcytosine, N6-adenine, 7-methylguanine, 5-methylaminomethyluracil, 5- methoxyaminomethyl-2-thiouracil, beta-D-mannosylqueosine, 5'-methoxycarboxymethyluracil, 5-methoxyuracil, 2-methylthio-N6-isopentenyladenine, wybutoxosine, pseudouracil, queosine, 2-thiocytosine, 5-methyl-2-thiouracil, 2-thiouracil, 4-thiouracil, 5-methyluracil, uracil- 5-oxyacetic acid methylester, uracil-5-oxyacetic acid, 5-methyl-2- thiouracil, 3-(3-amino-3-N-2-carboxypropyl) uracil, a thio-guanine, and 2,6-diaminopurine. A nucleotide sequence typically carries genetic information, including the information used by cellular machinery to make proteins and enzymes. In some embodiments, an oligonucleotide is a deoxyribozyme. In some embodiments, an oligonucleotide is a target nucleic acid sequence that is recognized by an oligonucleotide sensor comprising a deoxyribozyme.

Oligonucleotides described herein may be synthesized by standard methods known in the art, *e.g.,* by use of an automated DNA synthesizer (such as those that are commercially available from Biosearch, Applied Biosystems, *etc.*)*.* As examples, phosphorothioate oligonucleotides may be synthesized by the method of Stein et al., Nucl. Acids Res., 16, 3209, (1988), methylphosphonate oligonucleotides can be prepared by use of controlled pore glass polymer supports (Sarin et al., Proc. Natl. Acad. Sci. U.S.A. 85, 7448-7451, (1988)). The oligonucleotides may also be modified by many means known in the art. Non-limiting examples of such modifications include methylation, "caps", substitution of one or more of the naturally occurring nucleotides with an analog, and internucleotide modifications, such as, for example, those with uncharged linkages (*e.g*., methyl phosphonates, phosphotriesters, phosphoroamidates, carbamates, *etc.*) and with charged linkages (*e.g.,* phosphorothioates, phosphorodithioates, *etc.*)*.* Oligonucleotides (including, *e.g*., deoxyribozymes) may contain one or more additional covalently linked moieties, such as, for example, proteins (*e.g*., nucleases, toxins, antibodies, signal peptides, poly-L-lysine, *etc.*)*,* intercalators (*e.g.,* acridine, psoralen, *etc.*)*,* chelators (*e.g.,* metals, radioactive metals, iron, oxidative metals, *etc.*)*,* and alkylators. The oligonucleotides may be derivatized by formation of a methyl or ethyl phosphotriester or an alkyl phosphoramidate linkage. Furthermore, the oligonucleotides provided herein may also be modified with a label capable of providing a detectable signal, either directly or indirectly. Exemplary labels include radioisotopes, fluorescent molecules, isotopes (*e.g*., radioactive isotopes), biotin, and the like.

The term "substrate" as used herein refers to a chemical species that is reacted (*e.g*., using a deoxyribozyme as described herein) to produce a product (*e.g*., a fluorescent product). In some embodiments, a substrate is a small molecule. In some embodiments, a substrate is a small molecule that when modified may be transformed into a fluorescent product. In some embodiments, a substrate is a small molecule that comprises at least one phosphate moiety. Such substrates may be transformed into fluorescent products when at least one phosphate moiety moiety is cleaved or otherwise removed from the substrate (*e.g*., by a deoxyribozyme). Exemplary substrates for use with the deoxyribozymes described herein include, but are not limited to, 4-methylumbelliferyl phosphate (4-MUP), 6,8-Difluoro-4-methylumbelliferyl phosphate (diFMUP), and 9H-(1,3-Dichloro-9,9-dimethylacridin-2-one-7-yl) phosphate (DDAO). In certain embodiments, a substrate is 4-MUP.

The term "target nucleic acid sequence" refers to a sequence within a nucleic acid molecule that is recognized by any of the oligonucleotide sensors described herein. In some embodiments, a portion of an oligonucleotide sensor is complementary to a target nucleic acid sequence. In some embodiments, a portion of an oligonucleotide sensor is partially complementary to a target nucleic acid sequence (*e.g*., 70% or more complementary, 75% or more complementary, 80% or more complementary, 85% or more complementary, 90% or more complementary, 95% or more complementary, 96% or more complementary, 97% or more complementary, 98% or more complementary, or 99% or more complementary). In certain embodiments, a portion of an oligonucleotide sensor is 100% complementary to a target nucleic acid sequence. In some embodiments, these oligonucleotide sensors can distinguish between complementary target oligonucleotides (for example, targets with 100% complementarity to a portion of the sensor) and targets with mismatches (for example, targets with 20% to 35% complementarity to a portion of the target). The oligonucleotide sensors provided herein may be modified to comprise a portion that is complementary (or partially complementary) to any target nucleic acid sequence of interest. In some embodiments, an oligonucleotide sensor is capable of catalyzing the generation of a fluorescent product when bound to a target nucleic acid sequence.

The term "wherein each instance of F₁ and F₂ together represents a .... base pair" and similar terms relating to other variables refer to each of the two variables mentioned representing a nucleotide wherein the two nucleotides form a base pair which interacts through hydrogen bonds. The base pairs can be canonical base pairs (A-T, C-G) or non-canonical base pairs. The base pair can be selected from the list of base pairs provided for each pair of variables and the selection is independent of other variables in the sequence.

### DETAILED DESCRIPTION

The present disclosure provides deoxyribozymes that generate fluorescence (*e.g*., by catalyzing the transfer of a phosphate moiety from a substrate, thereby generating a fluorescent product). Kits comprising any of the deoxyribozymes described herein are also provided by the present disclosure. The present disclosure also provides methods for identifying such deoxyribozymes. Oligonucleotide sensors that utilize the deoxyribozymes described herein and are capable of generating a fluorescent product only in the presence of a specific ligand (*e.g*., a particular target nucleic acid sequence) are also provided by the present disclosure, as well as methods of using the same to detect the presence of a target nucleic acid sequence. The present disclosure further provides sensors that utilize the deoxyribozymes described herein to detect RNase activity.

### Deoxyribozymes

In one aspect, the present disclosure provides deoxyribozymes that are capable of catalyzing the formation of a fluorescent product. Such deoxyribozymes and variants thereof may be referred to herein as "Aurora." For example, the present disclosure provides deoxyribozymes capable of catalyzing the transfer of a phosphate moiety from a substrate containing at least one phosphate moiety. The transfer of a phosphate moiety from the substrate may concomitantly generate a fluorescent product. In some embodiments, the phosphate moiety is transferred to the deoxyribozyme. The phosphate moiety may be transferred to any position on the deoxyribozyme that is chemically capable of accepting such a moiety. For example, the phosphate moiety may be transferred to any nucleophilic position on the deoxyribozyme, or any position on the deoxyribozyme with nucleophilic character. In some embodiments, the phosphate moiety is transferred to a hydroxyl group on the deoxyribozyme. In certain embodiments, the phosphate moiety is transferred to the 5' hydroxyl group of the deoxyribozyme. In certain embodiments, the phosphate moiety is transferred to the 3' hydroxyl group of the deoxyribozyme.

The deoxyribozymes described herein are capable of modifying a substrate (for example, by catalyzing the transfer of a phosphate moiety from the substrate as discussed above) to produce a fluorescent product. Any substrate that can be chemically modified to produce a fluorescent product may be used in the context of the deoxyribozymes provided herein. In some embodiments, the substrate contains at least one phosphate moiety. In some embodiments, the substrate contains at least one diphosphate moiety. Exemplary substrates containing at least one phosphate moiety that may be modified by the deoxyribozymes provided herein include, but are not limited to, 4-methylumbelliferyl phosphate (4-MUP), 6,8-Difluoro-4-methylumbelliferyl phosphate (diFMUP), and 9H-(1,3-Dichloro-9,9-dimethylacridin-2-one-7-yl) phosphate (DDAO). In certain embodiments, the substrate is 4-MUP. In certain embodiments, the deoxyribozyme catalyzes the reaction:

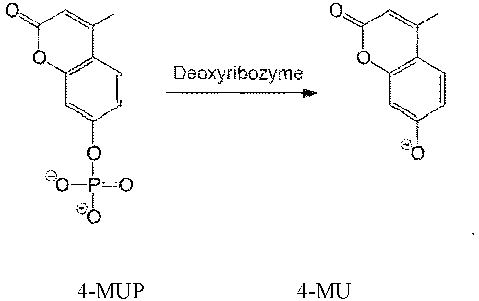

In some embodiments, the fluorescent product produced by the deoxyribozyme has greater emissions at one or more wavelengths when illuminated at one or more wavelengths than the substrate. For example, 4-MUP may be illuminated with excitation at a wavelength of about 360 nm and emit a fluorescent signal at a wavelength of about 440 nm. In some embodiments, the transfer of a phosphate moiety from the substrate may concomitantly generate a product with greater optical absorption at one or more wavelengths than the substrate.

In some embodiments, a deoxyribozyme provided herein comprises the sequence of SEQ ID NO: 7:

GGAAGAGATGACCAGGGCAGCGGGACGCTGACGAATTTTCTCACTATGTCCGGGACCCGAG
GGGCGTGAGGAGTGTTGTGCAATT (SEQ ID NO: 7).

In some embodiments, a deoxyribozyme provided herein comprises the sequence of SEQ ID NO: 8:
GGAAGAGATGACTATGTCCGGGACCCGAGGGGCGTGAGGAGTGTTGT (SEQ ID NO: 8).

In some embodiments, a deoxyribozyme provided herein comprises the sequence of SEQ ID NO: 12:

GGAAGGGATGAGCAGAGTAGCGCGACGATGACGAATTTTGTAACTATGTCCGGTTCCTGTA
AGGCATGTGGAGTGTTGTGCAATT
(SEQ ID NO: 12).

In some embodiments, a deoxyribozyme provided herein comprises the sequence of SEQ ID NO: 13:
GGAAGGGATGACTATGTCCGGTTCCTGTAAGGCATGTGGAGTGTTGT (SEQ ID NO: 13).

In some embodiments, a deoxyribozyme provided herein comprises the sequence of SEQ ID NO: 14:
GGAAGGGATGACTATGCCCGGTTCCTGTAAGGCATGTGGGGTGTTGT (SEQ ID NO: 14).

In some embodiments, a deoxyribozyme provided herein comprises the sequence of SEQ ID NO: 23:
GGAAGGGATGAATATGTCCGGTTCCAGTATGGCGTGTGGAGTGTTTT (SEQ ID NO: 23).

In some embodiments, a deoxyribozyme provided herein the sequence of SEQ ID NO: 24:
GGAAGGGATGAATATGTCCGGTTCCAGTATGGCTTGTGGAGTGTTTT (SEQ ID NO: 24).

In some embodiments, a deoxyribozyme comprises a nucleic acid sequence that is at least 60%, at least 70%, at least 80%, at least 85%, at least 90%, at least 95%, at least 96%, at least 97%, at least 98%, or at least 99% identical to the sequence of any one of SEQ ID NOs: 7, 8, 12, 13, 14, 23, and 24. In certain embodiments, a deoxyribozyme consists of the nucleic acid sequence of any one of SEQ ID NOs: 7, 8, 12, 13, 14, 23, and 24. In some embodiments, a deoxyribozyme comprises the consensus sequence GGAE₁GDDNNNZ₁Z₃NATF₁X₁P₁CGGNNCCZ₅NNNZ₆GGSNNGHGP₂X₂GF₂GTE₂Z₄Z₂ (SEQ ID NO: 37), wherein each instance of S independently represents C or G, each instance of H independently represents A, C, or T, each instance of D independently represents A, G, or T, and each instance of N independently represents A, C, G, or T; and wherein each instance of F₁ and F₂ independently represents a G-C base pair, a G-T base pair, or an A-T base pair, each instance of X₁ and X₂ independently represents a T-A base pair or a C-G base pair, each instance of P₁ and P₂ independently represents a T-A base pair, a C-G base pair, or a T-G base pair, each instance of Z₁ and Z₂ independently represents an A-T base pair, a T-A base pair, a G-C base pair, a C-G base pair, a G-T base pair, or a T-G base pair, each instance of Z₃ and Z₄ independently represents an A-T base pair, a T-A base pair, a G-C base pair, a C-G base pair, a G-T base pair, or a T-G base pair, each instance of Z₅ and Z₆ independently represents an A-T base pair, a TA base pair, a G-C base pair, a C-G base pair, a G-T base pair, or a T-G base pair, and each instance of E₁ and E₂ independently represents an A-T interaction, a G-A interaction, an A-A interaction, or an A-G interaction.

In some embodiments, a deoxyribozyme comprises a G at nucleotide position 1 relative to any one of SEQ ID NOs: 8, 13, 14, 23, and 24. In some embodiments, a deoxyribozyme comprises a G at nucleotide position 2 relative to any one of SEQ ID NOs: 8, 13, 14, 23, and 24. In some embodiments, a deoxyribozyme comprises an A at nucleotide position 3 relative to any one of SEQ ID NOs: 8, 13, 14, 23, and 24. In some embodiments, a deoxyribozyme comprises a G at nucleotide position 5 relative to any one of SEQ ID NOs: 8, 13, 14, 23, and 24. In some embodiments, a deoxyribozyme comprises an A at nucleotide position 14 relative to any one of SEQ ID NOs: 8, 13, 14, 23, and 24. In some embodiments, a deoxyribozyme comprises a T at nucleotide position 15 relative to any one of SEQ ID NOs: 8, 13, 14, 23, and 24. In some embodiments, a deoxyribozyme comprises a C at nucleotide position 19 relative to any one of SEQ ID NOs: 8, 13, 14, 23, and 24. In some embodiments, a deoxyribozyme comprises a G at nucleotide position 20 relative to any one of SEQ ID NOs: 8, 13, 14, 23, and 24. In some embodiments, a deoxyribozyme comprises a G at nucleotide position 21 relative to any one of SEQ ID NOs: 8, 13, 14, 23, and 24. In some embodiments, a deoxyribozyme comprises a C at nucleotide position 24 relative to any one of SEQ ID NOs: 8, 13, 14, 23, and 24. In some embodiments, a deoxyribozyme comprises a C at nucleotide position 25 relative to any one of SEQ ID NOs: 8, 13, 14, 23, and 24. In some embodiments, a deoxyribozyme comprises a G at nucleotide position 31 relative to any one of SEQ ID NOs: 8, 13, 14, 23, and 24. In some embodiments, a deoxyribozyme comprises a G at nucleotide position 32 relative to any one of SEQ ID NOs: 8, 13, 14, 23, and 24. In some embodiments, a deoxyribozyme comprises a G at nucleotide position 36 relative to any one of SEQ ID NOs: 8, 13, 14, 23, and 24. In some embodiments, a deoxyribozyme comprises a G at nucleotide position 38 relative to any one of SEQ ID NOs: 8, 13, 14, 23, and 24. In some embodiments, a deoxyribozyme comprises a G at nucleotide position 41 relative to any one of SEQ ID NOs: 8, 13, 14, 23, and 24. In some embodiments, a deoxyribozyme comprises a G at nucleotide position 43 relative to any one of SEQ ID NOs: 8, 13, 14, 23, and 24. In some embodiments, a deoxyribozyme comprises a T at nucleotide position 44 relative to any one of SEQ ID NOs: 8, 13, 14, 23, and 24. In certain embodiments, a deoxyribozyme comprises one or more of, two or more of, three or more of, four or more of, five or more of, six or more of, seven or more of, eight or more of, nine or more of, or ten or more of these nucleotides at the specified positions relative to any one of SEQ ID NOs: 8, 13, 14, 23, and 24.

The activity of the deoxyribozymes provided herein may be enhanced in the presence of one or more monovalent ions and/or one or more divalent ions. In some embodiments, the activity of a deoxyribozyme is dependent on the presence of one or more monovalent ions. In some embodiments, one or more monovalent and/or divalent ions play a structural role in a deoxyribozyme. Monovalent ions include, but are not limited to, potassium, lithium, sodium, rubidium, and ammonium. In certain embodiments, the monovalent ion is potassium. In some embodiments, the activity of a deoxyribozyme dependent on the presence of one or more divalent ions. Divalent ions include, but are not limited to, zinc, magnesium, calcium, manganese, cadmium, cobalt, copper, and palladium. In certain embodiments, the divalent ion is zinc.

In some embodiments, the deoxyribozymes described herein are useful in oligonucleotide sensors for detecting the presence of a ligand (*e.g*., a target nucleic acid sequence). In some embodiments, the deoxyribozymes described herein are useful for screening for inhibitors of the activity of an enzyme or other protein, including, for example, RNase. In some embodiments, the deoxyribozymes described herein are useful for diagnosing a disease or disorder in a subject.

### Methods of Identifying Deoxyribozymes

In another aspect, the present disclosure provides methods of identifying at least one deoxyribozyme capable of catalyzing the transfer of a phosphate moiety from a substrate containing at least one phosphate moiety (see, *e.g.,* the schematic provided in FIG. 6A). In some embodiments, the methods provided herein comprise the steps of:
a. annealing a library containing a plurality of single-stranded deoxyribonucleotides or regenerated according to step (j) of this method to a blocking oligonucleotide,
b. incubating the library annealed to the blocking oligonucleotide of step (a) with a substrate containing at least one phosphate moiety,
c. isolating the DNA of step (b),
d. ligating a deoxyribonucleotide oligo using a splint oligo to the purified DNAs of step (c) containing a 5' phosphate,
e. isolating the deoxyribonucleotide ligated products of step (d),
f. amplifying the isolated deoxyribonucleotide ligation products of step (e) using one forward primer which contains a ribonucleic acid (RNA) linkage 5' of the phosphorylation site and a second reverse deoxyribonucleic acid primer which contains a 5' phosphate,
g. isolating the DNA of step (f),
h. incubating the amplified deoxyribonucleotide ligation products of step (g) with lambda exonuclease to selectively degrade the antisense strand,
i. digesting the ribonucleic acid (RNA) linkage 5' of the phosphorylation site in the sense strands of DNA of step (h),
j. generating a plurality of single stranded deoxyribonucleotides having the sequence of the deoxyribonucleotide ligation products isolated in step (e), and
k. repeating steps (a)-(j) one or more times. In some embodiments, steps (a)-(j) are repeated one, two, three, four, five, six, seven, eight, nine, ten, or more than ten times. In certain embodiments, steps (a)-(j) are performed nine times.

In some embodiments, the pool of single-stranded deoxyribonucleotides of step (a) comprises sequences according to SEQ ID NO: 1:
GGAAGAGATGGCGACGACACAGGGACGATGCCGAATATCCTCAGTGCGCAGGGCCGCAGG GGGGAGTGACTTGGGATGGGGGGTCCACTAATGATCTGCCCGATG (SEQ ID NO: 1), wherein the underlined nucleotides are mutagenized at the rate of at least 1%, 5%, 10%, 20%, 21%, 25%, 30%, 35%, 40%, 45%, 50%, 55%, 60%, 65%, 70%, or 75%. In certain embodiments, the underlined nucleotides are mutagenized at a rate of 21%. In some embodiments, the pool of single-stranded deoxyribonucleotides of step (a) comprises sequences according to SEQ ID NO: 9: GGAAGAGATGACCAGGGCAGCGGGACGCTGACGAATTTTCTCACTATGTCCGGGACCCGAGG GGCGTGAGGAGTGTTGTGCAATTCTCATGAACTATCCGCTGGA (SEQ ID NO: 9), wherein the underlined nucleotides are mutagenized at the rate of at least 1%, 5%, 10%, 20%, 21%, 25%, 30%, 35%, 40%, 45%, 50%, 55%, 60%, 65%, 70%, or 75%. In certain embodiments, the underlined nucleotides are mutagenized at a rate of 21%. In some embodiments, the library containing a pool of deoxyribonucleotides contains more than 10⁶, 10⁹, 10¹², or 10¹⁵ single-stranded deoxyribonucleotide molecules containing a random sequence of at least 40, 50, 60, 70, 80, 90, or 100 nucleotides flanked by primer binding sites. In certain embodiments, the pool of deoxyribonucleotides contains more than 10¹⁶ single-stranded deoxyribonucleotide molecules. In some embodiments, the pool of deoxyribonucleotides contains a random sequence of at least 85 nucleotides flanked by primer binding sites.

In some embodiments, the pool of single-stranded deoxyribonucleotides of step (a) comprises one or more secondary structure libraries. In some embodiments, the methods described herein further comprise utilizing a pool of single-stranded deoxyribonucleotides comprising one or more secondary structure libraries (*e.g*., a secondary structure library synthesized using the synthetic shuffling method described in Curtis, E. A. & Bartel, D. P. Synthetic shuffling and in vitro selection reveal the rugged adaptive fitness landscape of a kinase ribozyme. RNA 19, 1116-1128 (2013) and Sgallová, R. & Curtis, E. A. Secondary Structure Libraries for Artificial Evolution Experiments. Mol. Basel Switz. 26, 1671 (2021), which are incorporated herein by reference). In certain embodiments, the secondary structure libraries comprise one or more of the libraries of SEQ ID NOs: 15-22: 
GGAGGRGATGRATRYRYCCGGKHCCRKWWYGGCDTGHGGRGYRYATYAACACTAATGATC
TGCCCGATG (SEQ ID NO: 15);
 
GGAAGRGATGRATRYRYCCGGKHCCRKWWYGGCDTGHGGRGYRYTTYAACACTAATGATC
TGCCCGATG (SEQ ID NO: 16);
 
GGAGGRGATGRCTRYRYCCGGKHCCRKWWYGGCDTGHGGRGYRYAGYAACACTAATGATC
TGCCCGATG (SEQ ID NO: 17);
 
GGAGGRGATGRATRYRYCCGGKHCCYKWWRGGCDTGHGGRGYRYATYAACACTAATGATC
TGCCCGATG (SEQ ID NO: 18);
 
GGAAGRGATGRCTRYRYCCGGKHCCRKWWYGGCDTGHGGRGYRYTGYAACACTAATGATC
TGCCCGATG (SEQ ID NO: 19);
 
GGAAGRGATGRCTRYRYCCGGKHCCYKWWRGGCDTGHGGRGYRYTGYAACACTAA
TGATCTGCCCGATG (SEQ ID NO: 22),
 
GGAAGRGATGRATRYRYCCGGKHCCYKWWRGGCDTGHGGRGYRYTTYAACACTAATGATC
TGCCCGATG (SEQ ID NO: 20);
 
GGAGGRGATGRCTRYRYCCGGKHCCYKWWRGGCDTGHGGRGYRYAGYAACACTAATGATC
TGCCCGATG (SEQ ID NO: 21);
 and
wherein R = A or G; Y = C or T; H = A or C or T; K = G or T;
D = A or G or T; and W = A or T.

In some embodiments, the blocking oligonucleotide of step (a) comprises the sequence CATCGGGCAGATCATTAGTG (SEQ ID NO: 3). In some embodiments, the blocking oligonucleotide of step (a) comprises the sequence TCCAGCGGATAGTTCATGAG (SEQ ID NO: 10). In some embodiments, the deoxyribonucleotide oligo of step (b) comprises the sequence ACCGCTCAGGTGTAGTATCA (SEQ ID NO: 2). In some embodiments, the deoxyribonucleotide splint comprises the sequence GTCGCCATCTCTTCCTGATACTACACCTGAGCGGT (SEQ ID NO: 4). In some embodiments, the primer that contains an RNA linkage 5' of the phosphorylation site comprises the sequence ACCGCTCAGGTGTAGTATCrA (SEQ ID NO: 5), wherein r represents the RNA linkage. In some embodiments, the second reverse deoxyribonucleic acid primer of step (f) comprises the sequence pCATCGGGCAGATCATTAGTG (SEQ ID NO: 6), wherein p represents a 5' phosphate group. In some embodiments, the second reverse deoxyribonucleic acid primer of step (f) comprises the sequence pTCCAGCGGATAGTTCATGAG (SEQ ID NO: 11), wherein p represents a 5' phosphate group.

In some embodiments, step (a) is performed by applying heat (*e.g*., heating to approximately 65°C or more for at least about two minutes) followed by cooling (*e.g*., to 25°C or less for at least 5 minutes). In some embodiments, the incubating of step (b) is performed on the order of hours, for example, for about 12, 13, 14, 15, 16, 17, 18, 19, 20, 21, 22, 23, 24, 25, 26, 27, 28, 29, 30, or more than 30 hours. In some embodiments, the incubating of step (b) is performed on the order of minutes (*e.g*., for about 1, 2, 3, 4, 5, 6, 7, 8, 9, 10, 11, 12, 13, 14, 15, 16, 17, 18, 19, 20, or more than 20 minutes). In certain embodiments, the incubating of step (b) is performed for 24 hours for seven rounds, then for 10 minutes in rounds eight and nine. In certain embodiments, the incubating of step (b) is performed for 10 minutes for the first three rounds, then for 1 minute for rounds four, five, six, and seven. In some embodiments, the incubating of step (b) is performed in the presence of a buffer containing monovalent and divalent cations (*e.g*., potassium and zinc, or any other monovalent ion and any other divalent ion described herein). In some embodiments, the isolating of step (c) is performed using ethanol precipitation. In some embodiments, the isolating of step (e) is performed using polyacrylamide gel electrophoresis. In some embodiments, the purification of step (g) is performed using ethanol precipitation. In some embodiments, the digesting of step (h) is performed in the presence of lambda exonuclease. In some embodiments, the digesting of step (i) is performed in the presence of base.

In some embodiments, the method further comprises amplifying the single-stranded deoxyribonucleotide pool obtained after the repetitions of step (k) by performing further rounds of steps

### Oligonucleotide Sensors

In another aspect, the present disclosure provides oligonucleotide sensors that utilize the deoxyribozymes described herein and are capable of sensing the presence of a particular target nucleotide sequence. In some embodiments, the oligonucleotide sensor produces a fluorescent signal in the presence of a target nucleotide sequence but does not produce a fluorescent signal in the absence of a target nucleotide sequence. For example, the present disclosure provides oligonucleotide sensors comprising a deoxyribozyme capable of catalyzing the transfer of a phosphate moiety from a substrate containing at least one phosphate or at least one diphosphate moiety, concomitantly generating a fluorescent product only when the deoxyribozyme is bound to a target nucleotide sequence. In some embodiments, the 3' end of the deoxyribozyme comprises a sequence that is the reverse complement of the target sequence. In some embodiments, an oligonucleotide sensor comprises at least 10, 15, 20, 30, 40, or 50 nucleotides of sequence from the 3' end of the target sequence inserted into the oligonucleotide sensor sequence.

In some embodiments, the oligonucleotide sensor comprises a nucleotide sequence of any one of SEQ ID NOs: 25-29: 
GGAAGGGATGGAAGGTCAATACTATGTCCGGTTCCTGTAAGGCATGTGGAGTGTTGTATTGA
CCTTCATTGACCTTC (SEQ ID NO: 25);
 
GGAAGGGATGGGGCACTGATACTATGTCCGGTTCCTGTAAGGCATGTGGAGTGTTGTATCAG
TGCCCAGTGGGCACG (SEQ ID NO: 26);
 
GGAAGGGATGGATGATCGGAACTATGTCCGGTTCCTGTAAGGCATGTGGAGTGTTGTTCCGA
TCATCCGAAGATCAG (SEQ ID NO: 27);
 
GGAAGGGATGAAGTAATAGCACTATGTCCGGTTCCTGTAAGGCATGTGGAGTGTTGTGCTAT
TACTTATCTTTCCGA (SEQ ID NO: 28);
 and

GGAAGGGATGTAAAAGATAAACTATGTCCGGTTCCTGTAAGGCATGTGGAGTGTTGTTTATC
TTTTATTCGTGTGTA (SEQ ID NO: 29).

The oligonucleotide sensors described herein may be used to detect any target nucleic acid sequence of interest. In some embodiments, a target nucleic acid sequence at least 8, at least 9, at least 10, at least 11, at least 12, at least 13, at least 14, at least 15, at least 16, at least 17, at least 18, at least 19, at least 20, at least 30, at least 40, at least 50, at least 60, at least 70, at least 80, at least 90, at least 100, at least 150, at least 200, at least 250, or at least 300 nucleotides long. In some embodiments, a target nucleic acid sequence is not more than 20, not more than 25, not more than 30, not more than 35, not more than 40, not more than 45, not more than 50, not more than 55, not more than 60, not more than 65, not more than 70, not more than 75, not more than 80, not more than 85, not more than 90, not more than 95, not more than 100, not more than 150, not more than 200, not more than 250, or not more than 300 nucleotides long. In some embodiments, the target nucleotide sequence comprises the nucleotide sequence of any one of SEQ ID NOs: 30-34:
GAAGGTCAATGAAGGTCAAT (SEQ ID NO: 30);
CGTGCCCACTGGGCACTGAT (SEQ ID NO: 31);
CTGATCTTCGGATGATCGGA (SEQ ID NO: 32);
TCGGAAAGATAAGTAATAGC (SEQ ID NO: 33); and
TACACACGAATAAAAGATAA (SEQ ID NO: 34).

In another aspect, the present disclosure provides methods for detecting the presence of a target nucleotide sequence using any of the oligonucleotide sensors described herein. In some embodiments, the methods comprise contacting a sample comprising a plurality of nucleotide sequences with an oligonucleotide sensor as provided herein, and observing a fluorescent signal when the target nucleotide sequence is present in the sample.

### RNase Detection Systems

In another aspect, the present disclosure provides RNase detection systems that utilize the deoxyribozymes described herein. In some embodiments, an RNase detection system comprises a 5' nucleotide extension to a deoxyribozyme comprising at least 1, 2, 3, 4, 5, 10, 15, 20, 30, 40, or 50 nucleotides, of which the 3' most residue of the extension is a ribonucleotide, and can be cleaved from the RNase detection system by RNase to generate a deoxyribozyme capable of catalyzing the transfer of a phosphate moiety from a substrate containing at least one phosphate or at least one diphosphate moiety, concomitantly generating a fluorescent product. In some embodiments, the RNase detection system comprises the sequence
AAAA(rC)GGAAGGGATGAATATGTCCGGTTCCTTTTAGGCGTGTGGAGTGTTTT (SEQ ID NO: 35), wherein r represents a ribonucleotide. In some embodiments, the RNase detection system comprises the sequence AAAA(rU)GGAAGGGATGAATATGTCCGGTTCCTTTTAGGCGTGTGGAGTGTTTT (SEQ ID NO: 36), wherein r represents a ribonucleotide.

### Kits

Also encompassed by the present disclosure are kits. Thus, in one aspect, provided are kits including a first container comprising a deoxyribozyme or oligonucleotide sensor described herein. The kits provided may comprise any of the deoxyribozymes or oligonucleotide sensors described herein and a container (*e.g*., a vial, ampule, bottle, syringe, and/or dispenser package, or other suitable container). In some embodiments, the kits comprise one or more substrates (*e.g*., 4-MUP, or any of the other substrates described herein). In some embodiments, the kits comprise a source of one or more divalent ions, such as zinc. In some embodiments, the kits comprise a source of one or more monovalent ions, such as potassium.

In certain embodiments, the kits are useful for detecting the presence of a ligand (*e.g*., a particular target oligonucleotide sequence). In certain embodiments, the kits are useful for diagnosing a disease in a subject. In certain embodiments, the kits are useful for screening for inhibitors of a target (*e.g*., an enzyme or other protein of interest).

In certain embodiments, a kit described herein further includes instructions for using the kit. In certain embodiments, the kits and instructions provide for detecting the presence of a ligand (*e.g*., a particular target oligonucleotide sequence). In certain embodiments, the kits and instructions provide for diagnosing a disease in a subject. In certain embodiments, the kits and instructions provide for screening for inhibitors of a target (*e.g*., an enzyme or other protein of interest).

### EXAMPLES

### Example 1. Aurora: a deoxyribozyme that generates fluorescence

Deoxyribozymes that generate signals are useful tools in diagnostics and synthetic biology. To address the need for such tools, artificial evolution was previously used to develop Supernova, a deoxyribozyme that produces light using the 1,2-dioxetane substrate CDP-Star. Deoxyribozymes that generate other types of signals could also be useful tools, especially if they are orthogonal to Supernova with respect to sequence requirements, substrate specificity, and the type of signal generated. For example, orthogonal deoxyribozymes activated by different ligands could be used in multiplex assays in which these ligands are detected in a single reaction. Orthogonal deoxyribozymes could also be useful as internal controls in the same way that pairs of orthogonal luciferases (such as those from firefly and sea pansy) are used to simultaneously measure the expression of a reporter gene encoded by a plasmid and the efficiency of transfection. In addition, the process of converting a deoxyribozyme that generates a signal into a sensor will likely be easier for some motifs than for others. For this reason, identifying a series of such deoxyribozymes with diverse folds will greatly increase the chance of developing useful sensors. As described herein, an orthogonal deoxyribozyme that generates fluorescence using the coumarin substrate 4-MUP has now been discovered. The sequence requirements and 47-nucleotide catalytic core of this deoxyribozyme, named Aurora, were characterized using both a randomly mutagenized library and a library enriched for the secondary structure of Aurora. These experiments yielded a variant that enhanced fluorescence by a factor of 700 and generated a detectable signal in minutes. A deoxyribozyme sensor was also developed that can detect picomolar concentrations of ribonuclease A in a homogeneous assay. The results described herein provide a new way to generate fluorescent sensors using DNA and represent an important step in the development of orthogonal deoxyribozymes for diagnostic applications.

### Identification of fluorescent deoxyribozymes using artificial evolution

Artificial evolution was previously used to identify Supernova, a deoxyribozyme that triggers a chemiluminescent reaction by transferring the phosphate group from the substrate CDP-Star to its 5' hydroxyl group (described in EP 4043566 A1 and Svehlova, K., Lukšan, O., Jakubec, M., Curtis, E. A. Supernova: A Deoxyribozyme that Catalyzes a Chemiluminescent Reaction. *Angew. Chem. Int. Ed Engl.* **2022**, 61, e202109347) (**FIGs. 1A-1B**). Substrates that generate orthogonal signals (such as fluorescence) when they are dephosphorylated have also been developed, and it was hypothesized that deoxyribozymes that use such substrates should be accessible using the artificial evolution protocol. To further explore this idea, attention was focused on a coumarin substrate called 4-MUP (**FIG. 1C**). Dephosphorylation of 4-MUP yields the fluorescent compound 4-MU, and it has been reasoned that a deoxyribozyme that phosphorylates itself using 4-MUP would also generate fluorescence. To test this idea, a library of 10¹⁴ variants of Supernova was generated by randomly mutagenizing its sequence at a rate of 21% per position. Supernova was used as a starting point because it catalyzes a phosphoryl transfer reaction using a substrate with some similarities to 4-MUP (compare **FIGs. 1B** and **1C**). Variants of Supernova that phosphorylated themselves when incubated with 4-MUP were then identified using a previously described selection protocol (**FIG. 1D**). After four rounds of selection activity was detected, and after one more round, the library was characterized by high-throughput sequencing. Sequences from the evolved library with high read numbers could phosphorylate themselves in the presence of 4-MUP and also generate fluorescence (**FIGs. 2A-2C** and Table 1).

**Table 1. Sequence Summary Table.**

| Name | Other name | Sequence | Description |
|---|---|---|---|
| SEQ ID NO: 1 | Pool 1 | GGAAGAGATGGCGACGACACAGGG ACGATGCCGAATATCCTCAGTGCGC AGGGCCGCAGGGGGGAGTGACTTG GGATGGGGGGTCCACTAATGATCTG CCCGATG | Library made by mutating the underlined positions of Supernova full-length at a rate of 21% per position |
| SEQ ID NO: 2 | FWD1 | ACCGCTCAGGTGTAGTATCA | Primer for selection |
| SEQ ID NO: 3 | REV1 | CATCGGGCAGATCATTAGTG | Primer for selection |
| SEQ ID NO: 4 | Splint 1 | GTCGCCATCTCTTCCTGATACTACAC CTGAGCGGT | Splint for selection |
| SEQ ID NO: 5 | FWD1r | ACCGCTCAGGTGTAGTATCrA | Primer for selection with RNA linkage (rA) at 3' end |
| SEQ ID NO: 6 | REV1p | pCATCGGGCAGATCATTAGTG | Primer for selection with 5' phosphate (p) |
| SEQ ID NO: 7 | Aurora 1 full-length | GGAAGAGATGACCAGGGCAGCGGG ACGCTGACGAATTTTCTCACTATGT CCGGGACCCGAGGGGCGTGAGGAG TGTTGTGCAATT | Full-length version of initial isolate of Aurora isolated from pool 1 |
| SEQ ID NO: 8 | Aurora 1 | GGAAGAGATGACTATGTCCGGGAC CCGAGGGGCGTGAGGAGTGTTGT | Minimized version of initial isolate of Aurora isolated from pool 1 |
| SEQ ID NO: 9 | Pool 2 | GGAAGAGATGACCAGGGCAGCGGG ACGCTGACGAATTTTCTCACTATGT CCGGGACCCGAGGGGCGTGAGGAG TGTTGTGCAATTCTCATGAACTATCC GCTGGA | Library made by mutating the underlined positions of Aurora 1 full-length at a rate of 21% per position |
| SEQ ID NO: 10 | REV2 | TCCAGCGGATAGTTCATGAG | Primer for selection |
| SEQ ID NO: 11 | REV2p | pTCCAGCGGATAGTTCATGAG | Primer for selection with a 5' phosphate (p) |
| SEQ ID NO: 12 | Aurora 2 full-length | GGAAGGGATGAGCAGAGTAGCGCG ACGATGACGAATTTTGTAACTATGT CCGGTTCCTGTAAGGCATGTGGAGT GTTGTGCAATT | Full-length version of optimized Aurora variant isolated from pool 2 |
| SEQ ID NO: 13 | Aurora 2 | GGAAGGGATGACTATGTCCGGTTCC TGTAAGGCATGTGGAGTGTTGT | Minimized version of optimized Aurora variant isolated from pool 2 |
| SEQ ID NO: 14 | Aurora 2 17C40G | GGAAGGGATGACTATGCCCGGTTCC TGTAAGGCATGTGGGGTGTTGT | Variant of Aurora 2 containing a mutated base pair |
| SEQ ID NO: 15 | Aurora secondary structured library subPool 1 | GGAGGRGATGRATRYRYCCGGKHC CRKWWYGGCDTGHGGRGYRYATYA ACACTAATGATCTGCCCGATG | One of 8 oligos used to make Aurora secondary structure library |
| SEQ ID NO: 16 | Aurora secondary structured library subPool 2 | GGAAGRGATGRATRYRYCCGGKHC CRKWWYGGCDTGHGGRGYRYTTYA ACACTAATGATCTGCCCGATG | One of 8 oligos used to make Aurora secondary structure library |
| SEQ ID NO: 17 | Aurora secondary structured library subPool 3 | GGAGGRGATGRCTRYRYCCGGKHC CRKWWYGGCDTGHGGRGYRYAGY AACACTAATGATCTGCCCGATG | One of 8 oligos used to make Aurora secondary structure library |
| SEQ ID NO: 18 | Aurora secondary structured library subPool 4 | GGAGGRGATGRATRYRYCCGGKHC CYKWWRGGCDTGHGGRGYRYATYA ACACTAATGATCTGCCCGATG | One of 8 oligos used to make Aurora secondary structure library |
| SEQ ID NO: 19 | Aurora secondary structured library subPool 5 | GGAAGRGATGRCTRYRYCCGGKHC CRKWWYGGCDTGHGGRGYRYTGY AACACTAATGATCTGCCCGATG | One of 8 oligos used to make Aurora secondary structure library |
| SEQ ID NO: 20 | Aurora secondary structured library subPool 6 | GGAAGRGATGRATRYRYCCGGKHC CYKWWRGGCDTGHGGRGYRYTTYA ACACTAATGATCTGCCCGATG | One of 8 oligos used to make Aurora secondary structure library |
| SEQ ID NO: 21 | Aurora secondary structured library subPool 7 | GGAGGRGATGRCTRYRYCCGGKHC CYKWWRGGCDTGHGGRGYRYAGY AACACTAATGATCTGCCCGATG | One of 8 oligos used to make Aurora secondary structure library |
| SEQ ID NO: 22 | Aurora secondary structured library subPool 8 | GGAAGRGATGRCTRYRYCCGGKHC CYKWWRGGCDTGHGGRGYRYTGY AACACTAATGATCTGCCCGATG | One of 8 oligos used to make Aurora secondary structure library |
| SEQ ID NO: 23 | Aurora 3 - 34G | GGAAGGGATGAATATGTCCGGTTCC AGTATGGCGTGTGGAGTGTTTT | Variant of Aurora isolated from the secondary structure library containing a G at position 34 |
| SEQ ID NO: 24 | Aurora 3 - 34T | GGAAGGGATGAATATGTCCGGTTCC AGTATGGCTTGTGGAGTGTTTT | Variant of Aurora isolated from the secondary structure library containing a T at position 34 |
| SEQ ID NO: 25 | Oligo sensor 1 | GGAAGGGATGGAAGGTCAATACTAT GTCCGGTTCCTGTAAGGCATGTGGA GTGTTGTATTGACCTTCATTGACCTT C | Variant of Aurora that is only active in the presence of target oligo 1 |
| SEQ ID NO: 26 | Oligo sensor 2 | GGAAGGGATGGGGCACTGATACTAT GTCCGGTTCCTGTAAGGCATGTGGA GTGTTGTATCAGTGCCCAGTGGGCA CG | Variant of Aurora that is only active in the presence of target oligo 2 |
| SEQ ID NO: 27 | Oligo sensor 3 | GGAAGGGATGGATGATCGGAACTAT GTCCGGTTCCTGTAAGGCATGTGGA GTGTTGTTCCGATCATCCGAAGATC AG | Variant of Aurora that is only active in the presence of target oligo 3 |
| SEQ ID NO: 28 | Oligo sensor 4 | GGAAGGGATGAAGTAATAGCACTAT GTCCGGTTCCTGTAAGGCATGTGGA GTGTTGTGCTATTACTTATCTTTCCG A | Variant of Aurora that is only active in the presence of target oligo 4 |
| SEQ ID NO: 29 | Oligo sensor 5 | GGAAGGGATGTAAAAGATAAACTAT GTCCGGTTCCTGTAAGGCATGTGGA GTGTTGTTTATCTTTTATTCGTGTGT A | Variant of Aurora that is only active in the presence of target oligo 5 |
| SEQ ID NO: 30 | Target oligo 1 | GAAGGTCAATGAAGGTCAAT | Oligo target recognized by Aurora oligo sensor 1 |
| SEQ ID NO: 31 | Target oligo 2 | CGTGCCCACTGGGCACTGAT | Oligo target recognized by Aurora oligo sensor 1 |
| SEQ ID NO: 32 | Target oligo 3 | CTGATCTTCGGATGATCGGA | Oligo target recognized by Aurora oligo sensor 1 |
| SEQ ID NO: 33 | Target oligo 4 | TCGGAAAGATAAGTAATAGC | Oligo target recognized by Aurora oligo sensor 1 |
| SEQ ID NO: 34 | Target oligo 5 | TACACACGAATAAAAGATAA | Oligo target recognized by Aurora oligo sensor 1 |
| SEQ ID NO: 35 | Aurora RNase A sensor | AAAA(rC)GGAAGGATGAATATGTC CGGTTCCTTTTAGGCGTGTGGAGTG TTTT | Version of Aurora with a single RNA linkage (rC) that is only active in the presence of RNase |
| SEQ ID NO: 36 | Aurora Nsp15 sensor | AAAA(rU)GGAAGGATGAATATGTC CGGTTCCTTTTAGGCGTGTGGAGTG TTTT | Version of Aurora with a single RNA linkage (rU) that is only active in the presence of RNase |
| SEQ ID NO: 37 | Consensus sequence of Aurora | GGAE1GDDNNNZ1Z3NATF1X1P1CGGN NCCZ5NNNZ6GGSNNGHGP2X2GF2GT E2Z4Z2 | Consensus sequence of Aurora |
| SEQ ID NO: 38 | Chro 4 full-length | GGCAGAGATGGCGCTGTCATAGGG ACAGTGCTACGCATGCTCACCCTCT GGGGCAATCGGGGGGCTTCGACTT GTATAGAGTTAG | Full-length version of optimized variant of chromogenic deoxyribozyme isolated from Chro 3 library |
| SEQ ID NO: 39 | Chro 4 | GGCAGAGATGGCGCAGCCACGCAT GCTCACCCTCTGGGGCAATCGGGG GG | Minimized version of optimized variant of chromogenic deoxyribozyme isolated from Chro 3 library |
| SEQ ID NO: 40 | Chro 3 full-length | GGCAGAGACGGCGGCGTCATAGGG ACAGTGCTGTACATGCTCACCCTCA GGGGCAATCGGGGGGCGTGAACTT ATATGGAGTTAC | Full-length version of initial isolate of a chromogenic deoxyribozyme isolated from pool 1 |
| SEQ ID NO: 41 | Chro 3 | GGCAGAGACGGCGCAGCTGTACAT GCTCACCCTCAGGGGCAATCGGGG GG | Minimized version of initial isolate of a chromogenic deoxyribozyme isolated from pool 1 |
| SEQ ID NO: 42 | Chro 3 library | GGCAGAGACGGCGGCGTCATAGGG ACAGTGCTGTACATGCTCACCCTCA GGGGCAATCGGGGGGCGTGAACTT ATATGGAGTTACTAACACTCTTGAC GAGCCGT | Library made by randomly mutagenzing underlined positions in Chro 3 full length at a rate of 20% per position |
| SEQ ID NO: 43 | Chromogenic oligo sensor | GGCAGAGATGGCGAAGGTCAATGC CACGCATGCTCACCCTCTGGGGCAA TCGGGGGGATTGACCTTCATTGACC TTCGCCATCTCTGCC | Sensor that only produces a chromogenic signal in the presence of an oligo target |
| SEQ ID NO: 44 | Target for oligo sensor | GGCAGAGATGGCGAAGGTCAATGA AGGTCAAT | Oligo target that activates chromogenic sensor |
| SEQ ID NO: 45 | Chromogenic sensor for RNase A | pAAAAAAAAA(rC)GGCAGAGATGGC GCAGCCACGCATGCTCACCCTCTGG GGCAATCGGGGG | Sensor with a single RNA linkage (rC) and a 5' phosphate (p) that only produces a chromogenic signal in the presence of RNase |
| SEQ ID NO: 46 | Chromogenic sensor for Nsp 15 | pAAAAAAAAA(rU)GGCAGAGATGG CGCAGCCACGCATGCTCACCCTCTG GGGCAATCGGGGG | Sensor with a single RNA linkage (rU) and a 5' phosphate that only produces a chromogenic signal in the presence of RNase |
| SEQ ID NO: 47 SEQ ID NO: 48 | Consensus sequence of a chromogenic deoxyribozym e | GGCAGAGAHRGC (SEQ ID NO: 47) -loop- KCHDYRCATGCTCMCCCKBDGGGG CAATCGGGGGG (SEQ ID NO: 48) | Consensus sequence of chromogenic deoxyribozyme |
| SEQ ID NO: 49 | Consensus sequence of shorter variant of chromogenic deoxyribozym e | GGCAGAGAYRCATGCTCMCCCKBD GGGGCAATCGGGGGG | Consensus sequence of shorter variant of chromogenic deoxyribozyme |
| SEQ ID NO: 50 | - | GGCAGAGAGGCGCAGCCCGCATGC TCACCCTCTGGGGCAATCGGGGGG | Deletion mutant of a chromogenic DNAzyme |
| SEQ ID NO: 51 | - | GGCAGAGAGCGCAGCCGCATGCTC ACCCTCTGGGGCAATCGGGGGG | Deletion mutant of a chromogenic DNAzyme |
| SEQ ID NO: 52 | - | GGCAGAGACGCAGCGCATGCTCAC CCTCTGGGGCAATCGGGGGG | Deletion mutant of a chromogenic DNAzyme |
| SEQ ID NO: 53 | - | GGCAGAGACGCATGCTCACCCTCTG GGGCAATCGGGGGG | Deletion mutant of a chromogenic DNAzyme |
| SEQ ID NO: 54 | - | GGCAGCATGCTCACCCTCTGGGGCA ATCGGGGGG | Deletion mutant of a chromogenic DNAzyme |
| SEQ ID NO: 55 | - | GGAAGATATGGCGCGAACAAATGG ACAATGCCGAATATCCCCCGCACGC AGGGCAACAAGGGGGAGTGACTTG GGATGGGGGCTGCACTAATGATCTG CCCGATG | Supernova variant tested against a panel of substrates |
| SEQ ID NO: 56 | - | GGAAGAAAAAGAATATCCCCAAAA GGGGAGTGACTTGGGATGGGGG | Supernova variant tested against a panel of substrates |
| SEQ ID NO: 57 | - | GGCAGAAAAAGAATATCCCCAAAA GGGGAGTGACTTGGGATGGGGG | Supernova variant tested against a panel of substrates |
| SEQ ID NO: 58 | - | GGAAGAAAAAGAATATCCCCAAAA GGGGAGTGACTTGCGATGGGGG | Supernova variant tested against a panel of substrates |
| SEQ ID NO: 59 | - | GGCAGAGATGGCAACGTCAACATG AGGATGCCGCATATCCCCAGTGCAC ACTTGGGCAGTGGGGAGTGACTTG GGATGGGGGAAA | Chromogenic DNAzyme tested against a panel of substrates |
| SEQ ID NO: 60 | 20889 in FIG. 27 | GGAGGAGATGGATATATCCGGGACC CTATGGGCGTGTGGAGCGTATC | Aurora variant with many mutations relative to Aurora 1-3 |

However, although they were isolated from a library of Supernova variants, most appeared to be structurally and functionally distinct. The mutational distances of sequences in the evolved pool with that of Supernova were compared. When a selection was previously performed to identify variants in this library that used CDP-Star (described in EP 4043566 A1 and Svehlova, K., Lukšan, O., Jakubec, M., Curtis, E. A. Supernova: A Deoxyribozyme that Catalyzes a Chemiluminescent Reaction. *Angew. Chem. Int. Ed Engl.* **2022**, *61*, e202109347), the average mutational distance of sequences in the evolved pool from Supernova was 18.42 (**FIG. 3A**). In comparison, the average mutational distance of variants that used 4-MUP was 30.68 (**FIG. 3A**). Analysis of individual sequences from the evolved library provided additional support for this idea: most of these sequences could not form the secondary structure of Supernova, and they also often contained mutations in unpaired regions that are not consistent with the sequence requirements of Supernova (**FIG. 3B**). The substrate specificities of these new deoxyribozymes were also different from that of Supernova. For example, the deoxyribozyme Aurora 1 could use 4-MUP but not CDP-Star as a substrate, whereas Supernova reacted with CDP-Star but not 4-MUP (**FIG. 3C**). These results demonstrate that the previously described approach could be extended to the identification of deoxyribozymes that generate fluorescence. They also suggest that, despite being isolated from a library based on Supernova, most variants that can react with 4-MUP form structures that are distinct from that of Supernova.

### Characterization of Aurora using a randomly mutagenized library

The fluorescent deoxyribozyme with the highest read number was chosen in the initial selection (which was named Aurora) for further characterization. One goal was to characterize the secondary structure, sequence requirements, and minimized catalytic core of this deoxyribozyme. Another was to identify variants with improved catalytic activity. To address both of these goals, a second library was synthesized by randomly mutating the sequence of Aurora at a rate of 21% per position. At this rate of mutagenesis, all possible variants within ~4 mutations of the starting sequence were expected to be present in the library (Table 2).

**Table 2. Coverage of sequence space in a randomly mutagenized library. This example showed expected coverage in a library generated by randomly mutagenizing 85 positions in Aurora 1 full-length at a rate of 21% per position.**

| **Mutations** | **Probability** | **Possible** | **Average copies** |
|---|---|---|---|
| 0 | 1.99 × 10⁻⁹ | 1 | 1.99 × 10⁵ |
| 1 | 4.49 × 10⁻⁸ | 2.55 × 10² | 1.76 × 10⁴ |
| 2 | 5.01 × 10⁻⁷ | 3.21 × 10⁴ | 1.56 × 10³ |
| 3 | 3.69 × 10⁻⁶ | 2.67 × 10⁶ | 1.38 × 10² |
| 4 | 2.01 × 10⁻⁵ | 1.64 × 10⁸ | 1.22 × 10¹ |
| 5 | 8.65 × 10⁻⁵ | 7.97 × 10⁹ | 1.09 × 10⁰ |
| 6 | 3.07 × 10⁻⁴ | 3.19 × 10¹¹ | 9.61 × 10⁻² |
| 7 | 9.20 × 10⁻⁴ | 1.08 × 10¹³ | 8.52 × 10⁻³ |
| 8 | 2.38 × 10⁻³ | 3.16 × 10¹⁴ | 7.55 × 10⁻⁴ |
| 9 | 5.42 × 10⁻³ | 8.01 × 10¹⁵ | 6.69 × 10⁻⁵ |
| 10 | 1.10 × 10⁻² | 1.85 × 10¹⁷ | 5.93 × 10⁻⁶ |
| 11 | 1.99 × 10⁻² | 3.78 × 10¹⁸ | 5.25 × 10⁻⁷ |
| 12 | 3.26 × 10⁻² | 6.99 × 10¹⁹ | 4.66 × 10⁻⁸ |
| 13 | 4.86 × 10⁻² | 1.18 × 10²¹ | 4.13 × 10⁻⁹ |
| 14 | 6.64 × 10⁻² | 1.82 × 10²² | 3.66 × 10⁻¹⁰ |
| 15 | 8.36 × 10⁻² | 2.58 × 10²³ | 3.24 × 10⁻¹¹ |

Catalytically active variants were then identified by artificial evolution and characterized by high-throughput sequencing. A total of 849,535 unique sequences were obtained, including 3 with a read number ≥ 10,000, 424 with a read number ≥ 1,000, and 9,856 with a read number ≥ 100 (Table 3). This highlights that many different sequences are consistent with the sequence requirements of Aurora.

**Table 3. Number of unique sequences with different read numbers in a library of Aurora variants after selection. This library was generated by randomly mutagenizing 85 positions in the full-length version of Aurora at a rate of 21% per position. After enriching the library for catalytically active variants using artificial evolution, it was characterized by high-throughput sequencing.**

| **Read number** | **Unique sequences** |
|---|---|
| ≥ 1 | 849,535 |
| ≥ 3 | 232,121 |
| ≥ 10 | 84,984 |
| ≥ 30 | 32,791 |
| ≥ 100 | 9,856 |
| ≥ 300 | 2,561 |
| ≥ 1,000 | 424 |
| ≥ 3,000 | 50 |
| ≥ 10,000 | 3 |

Comparative analysis revealed two highly conserved regions of the sequence (corresponding to nucleotides 1-10 and 43-79) separated by 32 less conserved positions. The catalytic activity of a 47 nucleotide deoxyribozyme in which the nucleotides at positions 11-42, 80-85, and in the 3' primer binding site were deleted was similar to that of the full-length sequence (**FIGs. 4A-4D**). Further analysis revealed four pairs of covarying positions in the deoxyribozyme (11-47, 12-46, 17-40, and 26-30) with mutational patterns consistent with those expected of base pairs (**FIG. 5A**). Mutagenesis experiments in which these and other putative base pairs were disrupted by point mutations and restored by compensatory mutations provided additional support for the proposed interactions (**FIGs. 6A-6E**). These constraints suggest that Aurora forms an 11-base pair hairpin interrupted by an asymmetric bulge (**FIG. 5A**). This hairpin is capped by a three-nucleotide loop formed by positions 27, 28, and 29. One of the most highly enriched mutations identified in the selection occurred in this loop (Table 4). In addition, several correlations (including 22-29, 22-27, and 23-26) suggest that this loop interacts with the asymmetric bulge of Aurora rather than extending into solution. If this is the case, the overall architecture of Aurora is likely that of a bent hairpin. Covariation analysis suggested that the 5' end of Aurora (which includes the phosphorylation site) does not form canonical base pairs with itself or the rest of the deoxyribozyme. However, a network of correlations among positions 4, 6, and 45 (including an AT to GA covariation between positions 4 and 45, which is one of the strongest in the dataset) was consistent with a tertiary interaction that anchors the 5' end of Aurora to the rest of the catalytic core. It is possible that this interaction helps to position the nucleophilic 5' hydroxyl group in the vicinity of the phosphate group of 4-MUP.

Characterization of additional mutations in both unpaired positions (Table 4) and base pairs (Table 5) revealed that enrichment values (defined as the frequency of a mutation in the evolved library divided by its calculated frequency in the starting library) could be used to identify mutations compatible with catalytic activity. It also revealed information about the sequence requirements of Aurora (**FIG. 5B**, Table 4, and Table 5).

**Table 4. Relationship between mutational frequencies in the evolved library and catalytic activity for mutations in unpaired positions.**

| **Enrichment values from high-throughput sequencing** | | | **Percent activity relative to Aurora** | |
|---|---|---|---|---|
| **Nucleotide in Aurora 2** | **Mutation** | **Enrichment** | **Average** | **SD** |
| 29A | 29T | 4.484088598 | 94 | 4 |
| 34A | 34T | 4.181303722 | 103 | 9 |
| 27G | 27T | 3.957247676 | 85 | 6 |
| 23T | 23C | 1.621630771 | 69 | 4 |
| 37T | 37C | 1.230633933 | 82 | 4 |
| 22T | 22G | 1.126613481 | 64 | 3 |
| 28T | 28A | 0.966224809 | 99 | 3 |
| 37T | 37A | 0.920584945 | 94 | 4 |
| 27G | 27A | 0.823144973 | 100 | 10 |
| 23T | 23A | 0.782215152 | 91 | 9 |
| 10G | 10A | 0.741638955 | 103 | 3 |
| 35T | 35A | 0.686320323 | 82 | 4 |
| 27G | 27C | 0.652736484 | 80 | 5 |
| 34A | 34G | 0.615210901 | 95 | 4 |
| 28T | 28C | 0.58841792 | 72 | 3 |
| 6G | 6A | 0.548335445 | 74 | 1 |
| 10G | 10T | 0.529464423 | 95 | 4 |
| 9T | 9A | 0.436177888 | 90 | 8 |
| 29A | 29C | 0.395648574 | 72 | 7 |
| 28T | 28G | 0.392258803 | 85 | 1 |
| 9T | 9G | 0.331792741 | 24 | 2 |
| 34A | 34C | 0.322381041 | 86 | 2 |
| 35T | 35C | 0.305562064 | 80 | 2 |
| 22T | 22A | 0.30049256 | 70 | 6 |
| 8A | 8T | 0.237433302 | 101 | 5 |
| 8A | 8G | 0.20227796 | 89 | 1 |
| 13T | 13A | 0.182367928 | 91 | 5 |
| 6G | 6T | 0.135054003 | 65 | 7 |
| 7G | 7A | 0.131714019 | 93 | 9 |
| 10G | 10C | 0.129954195 | 86 | 3 |
| 9T | 9C | 0.120347681 | 50 | 6 |
| 22T | 22C | 0.103145569 | 93 | 5 |
| 45T | 45C | 0.098532796 | 13 | 1 |
| 45T | 45 G | 0.098407249 | 79 | 4 |
| 7G | 7T | 0.091712128 | 96 | 3 |
| 35T | 35G | 0.084768076 | 70 | 3 |
| 29A | 29G | 0.075750334 | 52 | 9 |
| 37T | 37G | 0.059624031 | 15 | 1 |
| 33C | 33G | 0.052810322 | 11 | 3 |
| 13T | 13G | 0.041276843 | 69 | 3 |
| 33C | 33A | 0.031146494 | 13 | 1 |
| 8A | 8C | 0.025685197 | 86 | 2 |
| 23T | 23G | 0.020427373 | 7 | 1 |
| 13T | 13C | 0.016823306 | 27 | 5 |
| 4A | 4C | 0.013714934 | 10 | 3 |

**Table 5. Relationship between mutational frequencies in the evolved library and catalytic activity for mutations in base pairs and noncanonical pairs.**

| **Enrichment values from high-throughput sequencing** | | | **Percent activity relative to Aurora** | |
|---|---|---|---|---|
| **Pair in Aurora 2** | **Mutation** | **Enrichment** | **Average** | **SD** |
| 4A 45T | 4G 45A | 7.725256969 | 89 | 4 |
| 12C 46G | 12A 46T | 2.80965663 | 81 | 5 |
| 17T 40A | 17C 40G | 2.447301838 | 102 | 1 |
| 26T 30A | 26C 30G | 1.457301123 | 77.5 | 0.7 |
| 16G 42T | 16G 42C | 1.429893887 | 86 | 2 |
| 26T 30A | 26T 30G | 1.304179686 | 20.2 | 0.3 |
| 16G 42T | 16A 42T | 1.05074834 | 0 | n.d. |
| 26T 30A | 26A 30T | 0.773505953 | 96 | 3 |
| 11A 47T | 11G42C | 0.692116825 | 84 | 2 |
| 16G 42T | 16A 42C | 0.474388536 | 1.7 | 0.2 |
| 12C 46G | 12G 46C | 0.359417114 | 56 | 4 |
| 12C 46G | 12T 46A | 0.237240652 | 35 | 2 |
| 11A 47T | 11T 47A | 0.224314871 | 44 | 2 |
| 12C 46G | 12G 46T | 0.212904313 | 60 | 12 |
| 12C 46G | 12T 46T | 0.203256362 | 30 | 1 |
| 18C 39G | 18T 39G | 0.193644422 | 80 | 11 |
| 4A 45T | 4A 45A | 0.172505278 | 111 | 2 |
| 18C 39G | 18T 39A | 0.144626503 | 103 | 2 |
| 26T 30A | 26G 30C | 0.088223094 | 75 | 4 |
| 11A 47T | 11C 47G | 0.065432901 | 43 | 6 |
| 12C 46G | 12T 46G | 0.038039499 | 27 | 5 |
| 11A 47T | 11G 47T | 0.02427371 | 94 | 4 |
| 4A 45T | 4G 45C | 0.002442911 | 0 | n.d. |
| 19C 38G | 19T 38A | 0.001793529 | 12 | 1 |
| 15T 43G | 15C 43G | 0.000712401 | 0 | n.d. |
| 15T 43G | 15T 43A | 0.000394561 | 0 | n.d. |
| 25C 31G | 25T 31A | 0.000185538 | 0 | n.d. |
| 14A 44T | 14C 44G | 6.18458E-05 | 0 | n.d. |
| 24C 32G | 24T 32T | 0 | 0 | n.d. |

### Characterization of Aurora using a secondary structure library

Although this second selection yielded deoxyribozymes with improved catalytic efficiencies, it is possible that the library did not contain the most active possible variant of Aurora. This is because only a small portion of the sequence space of Aurora (the set of all sequences that can form the structure of Aurora) was sampled in the randomly mutagenized library (Table 2). For example, a tiny fraction of the 19,683 possible Aurora variants in which each of the nine canonical base pairs have changed to another canonical base pair would have been present. To address this limitation of randomly mutagenized libraries, a synthetic method was developed that can be used to generate libraries enriched for a desired motif. The method uses degenerate bases during the synthesis (such as R-Y and Y-R pairs, where R = A or G and Y = C or T) to increase the probability that positions that form base pairs in the secondary structure of the motif also do so in the library. Nucleotides that can occur at unpaired positions were chosen based on their frequencies in sequence alignments of variants of the motif (determined in this case using the experiments described in the previous section). Such an approach can increase the number of unique sequences with the potential to form a desired secondary structure by orders of magnitude relative to conventional approaches that employ random mutagenesis, especially for more complex folds. This method was used to synthesize a secondary structure library (encoded by 8 different degenerate oligonucleotides that were mixed to generate the library) based on Aurora (**FIG. 7A**). The library contained 2.8 × 10⁷ possible sequences, and this size was chosen so that it would be possible to identify active variants in a single round of selection followed by high-throughput sequencing (**FIG. 7B**). In the case of the selections described herein, the secondary structure library was incubated for either a short time in the presence of a saturating concentration of 4-MUP (to favor variants with improved *k*_{cat} values) or for a longer time in the presence of a sub-saturating concentration of 4-MUP (to favor variants with improved *K*_{M} values). Both selections yielded tens of thousands of enriched sequences (**FIG. 7C**, and Table 6). Read numbers were correlated with catalytic activity (FIGs. 7D-E), indicating that this approach can be used to provide quantitative information about large numbers of deoxyribozyme variants. Some of these variants were also significantly different from previously isolated variants (**FIG. 7F**), showing that this approach can be used to explore distant parts of sequence space that are not accessible using randomly mutagenized libraries.

**Table 6. Number of unique sequences with different read numbers in a secondary structure library based on Aurora. This library was synthesized in such a way that it was enriched for sequences with the secondary structure of Aurora (see FIG. 7A for sequences of the eight oligonucleotides used to encode this library). After performing single-step selections for variants with improved k_{cat} values (by incubating for 100 seconds with 100 µM 4-MUP) or K_{M} values (by incubating for 10 minutes with 20 µM 4-MUP), it was characterized by high-throughput sequencing.**

| **Read number** | **Unique sequences (initial library)** | **Unique sequences (*k*_{cat} selection)** | **Unique** sequences **(*K*_{M} selection)** |
|---|---|---|---|
| ≥ 1 | 3,857,605 | 3,892,241 | 3,167,689 |
| ≥ 3 | 1,687,052 | 1,321,084 | 680,676 |
| ≥ 10 | 222,368 | 132,159 | 44,228 |
| ≥ 30 | 7,030 | 37,428 | 14,001 |
| ≥ 100 | 10 | 14,087 | 4,883 |
| ≥ 300 | 0 | 3,841 | 1,432 |
| ≥ 1,000 | 0 | 521 | 262 |
| ≥ 3,000 | 0 | 38 | 33 |
| ≥ 10,000 | 0 | 0 | 0 |

Sequences that were enriched in one of these selections were often also enriched in the other (**FIG. 8A**). However, mutations at position 34 (in the asymmetric bulge of the deoxyribozyme) affected the slope of the line relating read number in the *k*_{cat} selection to read number in the *K*_{M} selection (**FIG. 8A**). Variants with high read numbers in the *k*_{cat} selection but not the *K*_{M} selection contained T at this position (**FIG. 8A**), while variants with high read numbers in both selections contained G (**FIG. 8A**). Catalytic assays confirmed the importance of position 34: a variant of Aurora containing T at position 34 was slightly more active at saturating concentrations of 4-MUP, whereas a variant containing G was ~100-fold more active at sub-saturating concentrations (**FIG. 8B**). This observation was consistent with the possibility that position 34 forms part of the substrate binding pocket of Aurora (**FIG. 8C**). Another striking feature of these datasets was the extent to which many of the base pairs in the secondary structure of Aurora were conserved. Examples include an A-T pair at 11-47, a T-A pair at 12-46, an A-T pair at 14-44, a T-G wobble pair at 15-43, and a T-A pair at 17-40. Each of these base pairs was almost invariant despite other possibilities (such as G-C at 11-47, C-G at 12-46, G-C at 14-44, C-G and T-A at 15-43, and C-G and 17-40) being equally likely in the library. On the other hand, two of the most frequently observed base pairs in these datasets (12-46 T-A and 26-30 A-T) differed from those present in both Aurora 1 (the original isolate of the deoxyribozyme; **FIGs. 3A-3C**) and Aurora 2 (the deoxyribozyme with the highest read number in the randomly mutagenized library; **FIG 5**). This observation highlighted the power of secondary structure libraries to identify new sequence variants of functional motifs. Taken together, these experiments indicated that the combination of secondary structure libraries and single-step selections is a powerful way to search sequence space for new sequence variants of a motif. They show that this approach can also be used to identify variants with novel functional properties.

### Aurora enhanced fluorescence by a factor of 700

To evaluate the extent to which these artificial evolution experiments yielded improved variants of Aurora, the catalytic activity of the initial isolate (Aurora 1; SEQ ID NO: 8), the variant with the highest read number from the randomly mutagenized library (Aurora 2; SEQ ID NO: 13), and one of the variants with the highest read number from the secondary structure library (Aurora 3 34G; SEQ ID NO: 23) were measured (the evolutionary lineage of each of these deoxyribozymes is shown in **FIG. 9A**). Each variant was characterized in the context of the 47-nucleotide minimized catalytic core (**FIG. 9B**), and measurements were performed over a range of 4-MUP concentrations using the ligation assay. These experiments revealed that both the randomly mutagenized library and the secondary structure library contained variants with catalytic activities more than 100-fold higher than that of the initial isolate (**FIGs**. **9C-9D**). Rates of Aurora 2 (from the randomly mutagenized library) and Aurora 3 34G (from the secondary structure library) were comparable (**FIGs. 9C-9D**), although Aurora 3 34G was 5-fold faster at the lowest concentration of 4-MUP tested. Surprisingly, 4-MUP concentration affected activity in a cooperative way, and cooperativity was also observed in proton NMR experiments in which Aurora folding was characterized as a function of 4-MUP concentration (**FIGs. 10A-10B**). This could indicate that Aurora contains multiple binding sites for 4-MUP, or a single site that binds multiple 4-MUP molecules. The extent to which Aurora enhanced fluorescence was also investigated using two different experimental setups (**FIG. 11A**). When using an experimental setup in which 4-MUP was mixed with Aurora 2 and fluorescence was continuously monitored using a plate reader, signal-to-noise ratios of fluorescence of 10-fold were obtained in minutes, and 100-fold in hours (**FIG. 11B**). When using a discontinuous setup in which reactions were quenched with base before measurement, signal-to-noise ratios of fluorescence were about 6-fold higher, and values exceeding 700-fold could be achieved (FIGs. 11B-C). Maximum signal-to-noise ratios in both the absence and the presence of base were similar to those obtained from samples containing synthetic 4-MU at the same concentration as that of 4-MUP used in the assays (**FIG. 12**). This indicated that Aurora generated the maximum possible signal-to-noise ratio of fluorescence for 4-MUP, although it is possible that higher signal-to-noise ratios could be achieved by deoxyribozymes that minimize quenching of fluorescence by the solvent.

### Aurora required multiple zinc ions for activity

Although the selection experiments provided extensive information about the sequence requirements of Aurora, they revealed little about how external factors (such as components of the buffer) influence the reaction. Such factors can affect both enzymatic and background rates and can also provide clues about catalytic mechanisms. The effects of metal ions on the reaction can play both structural and catalytic roles in ribozymes and deoxyribozymes. The experiments revealed both differences and similarities between Aurora (**FIGs. 13A-22**) and Supernova (described in EP 4043566 A1 and Svehlova, K., Lukšan, O., Jakubec, M., Curtis, E. A. Supernova: A Deoxyribozyme that Catalyzes a Chemiluminescent Reaction. *Angew. Chem. Int. Ed Engl.* **2022**, *61*, e202109347). An important difference was that Aurora requires monovalent ions for activity (**FIGs. 13A-15B**) while Supernova does not. On the other hand, both Aurora (**FIGs. 16A-17D**) and Supernova require zinc. The dependence of catalytic rate on zinc concentration was also highly cooperative for both deoxyribozymes (**FIGs. 17A-****17D**), suggesting that multiple zinc ions are needed for catalytic activity. However, these experiments did not indicate whether zinc played structural roles, catalytic roles, or both structural and catalytic roles in the reaction. To further distinguish among these possibilities, proton NMR was used to investigate the effect of zinc and other divalent metal ions on the folding of Aurora. In a buffer that contained zinc, multiple chemical shifts were observed in the region of the spectrum between 15 ppm and 10 ppm, which is consistent with formation of a secondary structure containing canonical base pairs (**FIG. 16C**). In contrast, these chemical shifts were absent in buffers in which zinc was replaced with other divalent metal ions (**FIG. 16C**). Titration experiments also showed that zinc affected Aurora folding in a highly cooperative way (**FIGs. 17C-17D**). Taken together, these experiments indicated that multiple zinc ions are required for Aurora function. They also showed that zinc played a structural role by promoting the folding of Aurora into its catalytically active conformation and leave open the possibility that zinc also plays catalytic roles in the reaction.

### Example 2. An engineered form of Aurora can detect ligands in solution

Variants of Aurora that only generate fluorescence in the presence of a ligand of interest could be useful for diagnostic applications. This is especially true for variants that can be activated in solution without the need for wash steps or biochemical purifications. To determine whether the catalytic activity of Aurora can be modulated by a ligand, rational design was used to construct a programmable sensor that only produces fluorescence in the presence of a specific oligonucleotide sequence (**FIGs. 23A-23D**). The approach was conceptually similar to the one previously used to generate a programmable version of Supernova that detects oligonucleotides, although the mechanisms of these two sensors are different. To inactivate Aurora in the absence of the target, the length of the helix that connects the 5' end of Aurora to the rest of the deoxyribozyme was extended. This was achieved by inserting the part of the sequence that was to be detected between nucleotides 10 and 11 and the reverse complement of the full sequence that was to be detected at the 3' end of Aurora (**FIG. 23A**, left panel). Pilot experiments showed that catalytic activity was significantly reduced when this helix was longer than ten base pairs, presumably because the rigidity of the helix prevents the reaction site (the 5' hydroxyl group) from reaching the substrate. In the presence of the target oligonucleotide, however, it was hypothesized that this inhibitory helix would not form, and instead the 3' end of the sensor would base pair with the target (**FIG. 23A**, right panel). This was expected to restore catalytic activity because the sequence inserted between nucleotides 10 and 11 would then be single-stranded, and flexible enough for the 5' hydroxyl group to reach the active site (**FIG. 23A**, right panel). Consistent with these expectations, the sensor produced significantly more fluorescence in the presence of the target than in the absence of the target (**FIG. 23B**). The sensor was programable and could be designed to detect a range of targets (**FIG. 23C**). It was also specific: sensors were activated by the target oligonucleotide they were designed to detect, but not by target oligonucleotides with different sequences (**FIG. 23C**). The detection limit of the sensor was approximately 1 µM of target (**FIG. 23D**), which was similar to that of a light-producing oligonucleotide sensor that was recently developed. This was about 30-fold higher than the detection limit of Aurora itself (approximately 30 nM; **FIG. 22**), indicating that the allosteric network linking ligand binding to catalytic activity is not yet optimal. These experiments showed that Aurora could be used to detect ligands in solution without the need for washes or biochemical purifications. They also highlighted the limitations of Aurora-based sensors that do not rely on signal amplification.

### Example 3. Increasing the sensitivity of assays using blocked Aurora

The sensitivity of Aurora is limited by catalytic turnover because, unlike classical enzymes, a single molecule of Aurora can only generate one molecule of fluorescent product. One way to increase sensitivity would be to link the signal generated by Aurora to the catalytic activity of an enzyme that itself catalyzes a multiple turnover reaction. Because Aurora is made of DNA, it was hypothesized that this type of coupling would be easiest to achieve using enzymes that modify nucleic acids. A variant of Aurora that is activated by enzymes that cleave RNA was developed. The sensor was constructed by fusing a short DNA oligonucleotide containing a ribonucleotide at its 3' end to the 5' end of Aurora (**FIG. 24A**, left). Because Aurora uses its 5' hydroxyl group as the nucleophile in the reaction, this modification was expected to abolish catalytic activity and eliminate the production of fluorescence. In the presence of a ribonuclease that cleaves RNA at internal sites to generate 3' phosphate (or 2'-3' cyclic phosphate) and 5' hydroxyl termini, however, the RNA linkage will be cleaved, which will regenerate the 5' end of Aurora and therefore restore catalytic activity (**FIG. 24A**, right). In addition, because ribonuclease enzymes typically promote multiple turnover reactions, a single ribonuclease molecule can activate multiple molecules of this type of sensor and therefore amplify the signal (**FIG. 24B**). The sensor was tested using ribonuclease A. This ribonuclease activated blocked Aurora and enhanced fluorescence more than 10-fold (**FIG. 24C**). The detection limit of the sensor under these conditions (~100 pM) was 10,000-fold lower than the oligonucleotide sensor (compare **FIGs. 24D** and **23D**). This dramatic increase in sensitivity is likely due to the high turnover number of RNase A. To further probe the mechanism of this sensor, it was investigated whether it was affected by RiboLock, a commercially available inhibitor of RNase A. RiboLock had no effect on Aurora 2 but prevented the Aurora sensor from being activated by RNase A (**FIG. 24C**). This provided additional evidence that the Aurora sensor is activated by cleavage. It also suggested that such a Aurora could be a suitable tool for the discovery of ribonuclease inhibitors in high-throughput screens. A second sensor was designed that was activated by Nsp15, a ribonuclease from SARS-CoV-2 (**FIG. 24E**). Taken together, these experiments showed that assays that use blocked Aurora can be orders of magnitude more sensitive than those that use unmodified Aurora. They also indicated that blocked Aurora is useful for detecting the presence of a known ribonuclease inhibitor and in high-throughput screens for novel inhibitors.

### Discussion of Examples 1-3

Artificial evolution was used to identify Aurora, a deoxyribozyme that generates a fluorescent signal using the coumarin substrate 4-MUP. Aurora offers a number of advantages when compared to other types of signaling molecules. Both Aurora and its substrate are inexpensive and commercially available. Aurora is also chemically stable and resistant to ribonuclease. The reaction is label free, which can reduce cost and background. It also generates a stable fluorescent product, and therefore provides a more permanent readout than aptamers, which enhance fluorescence using noncovalent interactions. Formation of the product generated by Aurora can be monitored in solution without the need for wash steps or biochemical purifications. And because Aurora is orthogonal to Supernova (a light-producing deoxyribozyme) with respect to both the substrate specificity and the type of signal generated, it is possible that these deoxyribozymes could be used in tandem for multiplex assays, to increase the sensitivity of assays for single ligands, and to provide internal controls.

A covalently blocked version of Aurora with increased sensitivity was also developed that detects (and is activated by) an enzyme that itself catalyzes a multiple turnover reaction. This increased the detection limit ~10,000-fold and made it possible to detect as little as ~100 pM of ribonuclease A in a homogeneous assay. This detection limit compares favorably with those of many recently developed homogeneous assays that use aptamers in combination with signaling elements such as fluorophores, dyes, quantum dots, or gold nanoparticles. However, such assays are often not label free, which can increase both cost and background. This comparison highlights a significant advantage of both Supernova and Aurora-based sensors as compared to those that use covalently linked small molecules to generate signals.

In addition to expanding the toolkit of functional DNA parts, the experiments also provide insights into how to explore sequence space for active variants of deoxyribozyme motifs. The sequence requirements, secondary structure, and 47-nucleotide minimized catalytic core of Aurora were identified using a standard approach in which a library was generated by randomly mutagenizing a single variant of Aurora. A second library enriched for the secondary structure of Aurora was generated using a synthetic method that was recently developed. Deoxyribozymes were identified by performing a single round of selection followed by high-throughput sequencing. This revealed tens of thousands of new variants of Aurora, including one with improved catalytic activity at sub-saturating substrate concentrations (**FIGs. 9C-9D**). It also contained variants with combinations of mutations that were not observed in either the initial isolate of Aurora or the randomly mutagenized library. An advantage of combining secondary structure libraries with one-step selections is that it makes it possible to rapidly screen libraries for a series of phenotypes of interest. In the case of Aurora, this could facilitate identification of variants that use different substrates, that promote multiple turnover reactions, or that only generate fluorescence in the presence of a specific ligand. In the case of other functional nucleic acids, it was suggested that it could also help to identify variants with a wide range of useful and interesting properties.

### Materials and Methods for Examples 1-3

### Oligonucleotides

Oligonucleotides were chemically synthesized by GENERI BIOTECH s.r.o., Sigma- Aldrich, or IDT and purified by PAGE or HPLC. See Table 1 for the sequences of oligonucleotides used.

### Pool design

The library used in the initial selection (Pool 1 in Table 1) was generated by randomly mutagenizing Supernova (a recently discovered chemiluminescent deoxyribozyme) at a rate of 21% per position. The library was previously used to identify variants of Supernova that phosphorylate themselves in the presence of CDP-Star. It was used here to instead identify variants of Supernova that phosphorylate themselves in the presence of 4-MUP.

### Initial Selection

The initial selection conditions were similar to those described in Svehlova, K., Lukšan, O., Jakubec, M., Curtis, E. A. Supernova: A Deoxyribozyme that Catalyzes a Chemiluminescent Reaction. *Angew. Chem. Int. Ed Engl.* **2022**, *61,* e202109347 but with the following differences. First, pool 1 was incubated with 1 mM 4-MUP (Sigma-Aldrich) for 2.4 hours in each of the five rounds of the initial selection. Second, PCR amplification was performed using Q5 HotStart Polymerase (NEB). Third, different sequences were used for the blocking oligonucleotide/reverse primer (REV1/REV1p), splint oligonucleotide (Split 1), and ligation acceptor oligo/forward primer (FWD1/FWD1r) (Table 1). Fourth, after PCR amplification, DNA was purified using the NucleoSpin Gel and PCR Clean-up kit (Macharey-Nagel). Fifth, single stranded pool was generated by digestion of the reverse strand by λ-exonuclease (NEB) followed by another purification using the NucleoSpin Gel and PCR Clean-up kit. And sixth, the pool from the last round was sequenced by Eurofins Genomics, using an amplicon paired-end sequencing run.

### Reselection

The pool for the reselection was generated by randomly mutagenizing an 85-nucleotide region of the variant from the initial selection with the highest catalytic activity at a rate of 21% per position. A new reverse primer binding site was also added (see Pool 2 in Table 1 for the sequence). Reselection conditions were similar to those in Svehlova, K., Lukšan, O., Jakubec, M., Curtis, E. A. Supernova: A Deoxyribozyme that Catalyzes a Chemiluminescent Reaction. *Angew. Chem. Int. Ed Engl.* **2022**, *61*, e202109347 but with the following differences. First, pool 2 was incubated with 1 mM 4-MUP (Sigma-Aldrich). Second, the incubation time during each of the six rounds of the selection was 14.4 minutes. Third, sequences of the blocking oligonucleotide/reverse primer (REV2/REV2p), splint oligonucleotide (Splint 1), and ligation acceptor oligo/forward primer (FWD1/FWD1r) were different (Table 1). Fourth, the pool from the last round was sequenced by Eurofins Genomics, using an amplicon paired-end sequencing run.

### Optimization of reaction conditions

To maximize fluorescence, optimal reaction conditions were determined. The optimal DNA, 4-MUP, KCl, ZnCl₂ and HEPES concentrations were determined by titration experiments. The effects of different monovalent and divalent metal ions, an organic solvent (DMSO), and a molecular crowding agent (PEG 200) on activity was also tested. Titration experiments to determine the optimal pH during and after the reaction were also performed. Aurora 2 (Table 1) was used for these experiments if not stated otherwise. Activity was measured by analysis of fluorescence production (using a plate reader assay) and self-phosphorylation (using a ligation assay).

### Secondary structured library and single step selection

Eight sub-libraries (each encoded by a different partially degenerate oligonucleotide) were synthesized separately and mixed in equimolar ratios to obtain the secondary structure library (see Table 1, SEQ ID NOs: 15-22). This library was mixed with the blocking oligonucleotide REV1 in Milli-Q water. After heating at 65 °C for 2 minutes and cooling at room temperature for 5 minutes, 5× Aurora buffer (1 M KCl, 5 mM ZnCl₂, 250 mM HEPES pH 7.4, 25 (v/v) % DMSO) was added. The mixture was then incubated at 20 µM 4-MUP for 10 minutes (*K*_{M} selection) or at 100 µM 4-MUP for 100 seconds (*k*_{cat} selection). Conditions for single step selections were similar to those described in Svehlova, K., Lukšan, O., Jakubec, M., Curtis, E. A. Supernova: A Deoxyribozyme that Catalyzes a Chemiluminescent Reaction. *Angew. Chem. Int. Ed Engl.* **2022**, *61*, e202109347 but with the following differences. First, oligonucleotides encoding the 87-nucleotide starting library were purified by 6% Urea-PAGE. Second, after incubating the library with 4-MUP and performing the ligation reaction, reacted library members for purified twice by 6% Urea-PAGE to increase purity. Third, different sequences were used for the blocking oligonucleotide/reverse primer (REV1/REV1p), splint oligonucleotide (Split 1), and ligation acceptor oligo/forward primer (FWD1/FWD1r) (Table 1). The pool was sequenced by Eurofins Genomics, using an amplicon paired-end sequencing run.

### Analysis of Fluorescence Production

Oligonucleotides corresponding to individual sequences from evolved libraries were ordered from GENERI BIOTECH s.r.o. Fluorescence production was measured as follows: oligonucleotides were resuspended in Milli-Q water, heated at 65 °C for 2 minutes, and cooled at room temperature for 5 minutes. After adding 5× selection buffer or 5× Aurora buffer, samples were transferred to a white half-area 96-well plate (Corning). 4-MUP was added, and fluorescence was then measured for 4 hours using a Tecan Spark plate reader (Tecan Group). After incubating for 4 hours, samples were quenched with 20 µl of 1 M KOH, and fluorescence was measured using a plate reader. In a typical experiment, final concentrations were 15 µM of the tested oligonucleotide and either 1× selection buffer (200 mM KCl, 1 mM ZnCl₂, 1 µM Ce(SO4)2, 0.1 µM PbCl₂, 50 mM HEPES pH 7.4) or 1× Aurora buffer (200 mM KCl, 1 mM ZnCl₂, 50 mM HEPES, pH 7.4, 5% (v/v) DMSO), and 30 µM 4-MUP.

### Analysis of Phosphorylation

Analysis of self-phosphorylation was performed as described in Svehlova, K., Lukšan, O., Jakubec, M., Curtis, E. A. Supernova: A Deoxyribozyme that Catalyzes a Chemiluminescent Reaction. *Angew. Chem. Int. Ed Engl.* **2022**, *61*, e202109347 but with the following differences. First, reactions were performed in the presence of 4-MUP rather than CDP-Star, and a different concentration was used. Second, reactions were performed in 1× Aurora buffer (200 mM KCl, 1 mM ZnCl₂, 50 mM HEPES, pH 7.4, 5% (v/v) DMSO).

### Next generation sequencing and data analysis

All libraries were sequenced by Eurofins Genomics using amplicon paired-end sequencing runs. Raw reads were processed using a pipeline consisting of adaptor trimming (cutadapt v 1.18), read merging (fastq-join v1.3.1), unifying of read orientation (fastx barcode splitter), primer clipping (cutadapt v1.18), length filtering (cutadapt v1.18) and counting of unique sequences (bash). All further analysis was performed using in-house python scripts available at github.com/Jardic/aurora_selection_analysis.

### Oligonucleotide detection using an engineered version of Aurora

The oligonucleotide sensor was mixed with the target oligonucleotide in water, heated at 98 °C for 2 minutes, and immediately cooled on ice for 5 minutes. 5× Aurora buffer and DMSO were then added. Samples were transferred to a white half-area 96-well plate (Corning), 4-MUP was added, and the reaction mixture was incubated for 4 hours at room temperature. Final concentrations were 5 µM of the oligonucleotide sensor, 10 µM of the target oligonucleotide, 1× Aurora buffer (200 mM KCl, 50 mM HEPES, pH 7.4, 1 mM ZnCl₂, 5% (v/v) DMSO), and 30 µM 4-MUP. After 4 hours, the reaction was stopped by adding 20 µl of 1 M KOH, and fluorescence was then measured using a Tecan Spark plate reader. Analysis of fluorescence production was performed as described below in "Calculation of signal-to-noise ratios."

### Ribonuclease sensor based on Aurora

The RNase A sensor was heated at 65°C for 2 minutes and cooled at room temperature for 5 minutes. Then, 5× Aurora buffer and DMSO were added. Samples were transferred to a white half-area 96-well plate (Corning), and 4-MUP and either RNase A (Thermo Fisher Scientific) alone or RNase A and RiboLock (Thermo Fisher Scientific) were added. The reaction mixture was incubated for 4 hours at room temperature. Final concentrations were 5 µM of the RNase A sensor, 500 nM RNase A, or 500 nM RNase A plus 500 nM RiboLock, 1× Aurora buffer (200 mM KCl, 50 mM HEPES pH 7.4, 1 mM ZnCl₂ and 5% (v/v) DMSO), and 30 µM 4-MUP if not stated otherwise. After 4 hours the reaction was stopped by adding 20 µl of 1 M KOH to the reaction mixture. Fluorescence was then measured using a Tecan Spark plate reader. Analysis of fluorescence production was performed as described below in the section "Calculation of signal-to-noise ratios."

### Kinetics measurements and analysis

Kinetic measurements, data analysis, and curve fitting were performed as described in Svehlova, K., Lukšan, O., Jakubec, M., Curtis, E. A. Supernova: A Deoxyribozyme that Catalyzes a Chemiluminescent Reaction. *Angew. Chem. Int. Ed Engl.* **2022**, *61*, e202109347 but with the following differences. First, reactions were performed in the presence of 4-MUP rather than CDP-Star, and a different concentration was used. Second, incubations were performed in 1× Aurora buffer (200 mM KCl, 1 mM ZnCl₂, 50 mM HEPES, pH 7.4, 5% (v/v) DMSO). See Table 1 for the sequences of deoxyribozymes used in kinetic assays.

### Calculation of signal-to-noise ratios

Signal-to-noise ratios were defined as the fluorescence of a sample in the presence of deoxyribozyme divided by the fluorescence of the sample in the absence of the deoxyribozyme. The background signal was defined as the fluorescence of 1× Aurora buffer (200 mM KCl, 50 mM HEPES, pH 7.4, 1 mM ZnCl₂, and 5% (v/v) DMSO) and was subtracted before calculating signal-to-noise ratios.

### NMR experiments

*Sample preparation.* HPLC-purified DNA was purchased from GENERI BIOTECH s.r.o. DNA was resuspended in Milli-Q water, heated at 65 °C for 2 minutes, cooled at room temperature for 5 minutes, and 5× Aurora buffer was then added. Concentrations at this point were 15 µM DNA, 200 mM KCl, 50 mM HEPES, pH 7.4, and 1 mM ZnCl₂. Samples were concentrated using Ultra-Amicon Centrifugal Filter Units (cutoff 3 kDa) to 500 µM DNA and a 1.5 molar excess of 4-MUP, D2O, and DSS were added. Final concentrations were 500 µM DNA, 200 mM KCl, 50 mM HEPES, pH 7.4, 1 mM ZnCl₂, 750 µM 4-MUP, 10% (v/v) D2O, and a trace amount of DSS.

*NMR measurements.* NMR experiments were performed on a Bruker Avance III HD 850 MHz system equipped with an inverse triple resonance cryo-probe. Spectral analyses were performed using TOPSPIN (Bruker) software.

### Example 4. A deoxy ribozymes that generate a chromogenic product using 4-NPP

In principle, it could be possible to apply the general method described in this patent application to isolate deoxyribozymes that generate other kinds of signals by transferring a phosphate group from an appropriate substrate to themselves. To see if this was possible in practice, we used artificial evolution to search our library of mutants of the light-producing deoxyribozyme Supernova (used previously to isolate variants that react with the chemiluminescent substrate CDP-Star or the fluorescent substrate 4-MUP) for catalytic motifs that react with the chromogenic substrate 4-NPP (**FIGs. 25A-25B**). This selection yielded deoxyribozymes that phosphorylate themselves using 4-NPP (**FIG. 25C**) and generate a product that can be detected using a spectrophotometer (**FIG. 25D**) or by eye (**FIG. 25E**). As was the case for deoxyribozymes isolated from this library that use 4-MUP, these catalytic motifs were distinct from Supernova. The sequence with the highest read number was further characterized. The buffer requirements of this deoxyriboyzme were similar to those of deoxyribozmes that react with CDP-Star and 4-MUP (including a requirement for multiple zinc ions). It was also possible to engineer this deoxyribozyme to act as a sensor to detect oligonucleotides and ribonucleases (**FIG. 25F**). The sequence requirements of one of these chromogenic deoxyribozymes were characterized by randomly mutagenizing its sequence at a rate of 21% per position, enriching the library for active variants by artificial evolution, and analyzing the evolved library by high-throughput sequencing (**FIG. 25A**). This yielded an improved variant with a catalytic efficiency 10-fold higher than that of the original isolate (**FIG. 25G**). It also revealed the 50-nucleotide minimized catalytic core of the deoxyribozyme (**FIG. 26**), facilitated identification of shorter constructs that also retain significant activity (**FIG. 26**), and provided information about conservation at each position in the motif (Table 7).

**Table 7. Enrichment values in a chromogenic deoxyribozyme. A library was generated by randomly mutagenizing 85 positions in the full-length version of this deoxyribozyme at a rate of 21% per position. Active variants were isolated by artificial evolution, and the evolved library was characterized by high-throughput sequencing. Enrichment is defined as the frequency of the mutation in the evolved library divided by its calculated frequency in the starting library.**

| **Enrichment values from high-throughput sequencing** | | | |
|---|---|---|---|
| **Position** | **Original nt** | **Mutation** | **Enrichment** |
| 1 | G | G | 1.266 |
| 2 | G | G | 1.266 |
| 3 | C | C | 1.265 |
| 4 | A | A | 1.265 |
| 5 | G | G | 1.266 |
| 6 | A | A | 1.266 |
| 7 | G | G | 1.266 |
| 8 | A | A | 1.265 |
| 9 | C | T | 11.664 |
| | | A | 0.642 |
| 10 | G | G | 1.238 |
| | | A | 0.285 |
| 11 | G | G | 1.249 |
| 12 | C | C | 1.262 |
| 30 | G | G | 1.218 |
| | | T | 0.243 |
| 31 | C | C | 1.245 |
| 32 | T | A | 1.311 |
| | | T | 1.052 |
| | | C | 0.950 |
| 33 | G | A | 9.861 |
| | | T | 1.796 |
| | | G | 0.233 |
| 34 | T | C | 8.948 |
| | | T | 0.467 |
| 35 | A | G | 12.000 |
| | | A | 0.202 |
| 36 | C | C | 1.266 |
| 37 | A | A | 1.264 |
| 38 | T | T | 1.266 |
| 39 | G | G | 1.265 |
| 40 | C | C | 1.266 |
| 41 | T | T | 1.265 |
| 42 | C | C | 1.266 |
| 43 | A | C | 3.003 |
| | | A | 1.000 |
| 44 | C | C | 1.266 |
| 45 | C | C | 1.266 |
| 46 | C | C | 1.266 |
| 47 | T | T | 1.247 |
| | | G | 0.211 |
| 48 | C | C | 1.196 |
| | | G | 0.516 |
| | | T | 0.248 |
| 49 | A | T | 10.330 |
| | | A | 0.328 |
| | | G | 0.253 |
| 50 | G | G | 1.266 |
| 51 | G | G | 1.266 |
| 52 | G | G | 1.266 |
| 53 | G | G | 1.266 |
| 54 | C | C | 1.266 |
| 55 | A | A | 1.265 |
| 56 | A | A | 1.265 |
| 57 | T | T | 1.266 |
| 58 | C | C | 1.266 |
| 59 | G | G | 1.266 |
| 60 | G | G | 1.266 |
| 61 | G | G | 1.266 |
| 62 | G | G | 1.266 |
| 63 | G | G | 1.266 |
| 64 | G | G | 1.266 |

### Example 5. Deoxyribozymes that use additional substrates

Two additional methods were used to identify deoxyribozymes with new substrate specificities. In one approach, representative deoxyribozymes isolated in selections using CDP-Star (SEQ ID NOs: 55-58 in **FIG. 27A**), 4-MUP (SEQ ID NOs: 7-8, SEQ ID NO: 13, and SEQ ID NO: 23 in **FIG. 27A**), or 4-NPP (SEQ ID NOs: 39-41 and SEQ ID NO: 59 in **FIG. 27A**) were tested for their ability to react with a panel of substrates, including some not used in the original selections (**FIG. 27B**). These experiments revealed that Supernova can use CSPD as well as CDP-Star (as described in EP 4043566 A1; see also SEQ ID NOs: 55-56 in **FIGs. 27C-27D**), that the fluorescent deoxyribozyme described in this patent application (particularly the 34G variant) can use diFMUP as well as 4-MUP (SEQ ID NOs: 7-8, SEQ ID NO: 13, and SEQ ID NO: 23 in FIGs. 27C-D), and that some deoxyribozymes originally selected for their ability to react with 4-NPP can also use DDAO (SEQ ID NOs: 39-41 and SEQ ID NO: 59 in FIGs. 27C-D). In a second approach, high-throughput sequencing datasets generated by selecting for variants of Supernova that could use 4-MUP or 4-NPP were filtered to identify sequences that retained the fold of Supernova. Nucleotide frequencies at each variable position in these datasets were then compared to those in a dataset of Supernova variants selected for the ability to use the original CDP-Star substrate to identify mutations likely to affect deoxyribozyme substrate specificity. This revealed that the 3C and 38C mutations change the substrate specificity of Supernova, especially with respect to its ability to utilize diFMUP (SEQ ID NOs: 57-58 in FIGs. 27C-D). Taken together, these results indicate that our approach is relatively general. They also suggest that similar selections could be used to identify deoxyribozymes that activate other types of substrates by removal of a phosphate group (including substrates not described here).

### Discussion of Examples 4 and 5

Our initial results using CDP-Star and 4-MUP results raise the possibility that the general approach described here could be used to identify deoxyribozymes that generate other types of signals. In support of this idea, we showed that our method can be used to identify deoxyribozymes that generate a chromogenic signal by transferring the phosphate group from the substrate 4-NPP to themselves. As was the case for deoxyribozymes that generate chemiluminescence using CDP-Star (described in EP 4043566 A1) and that generate fluorescence using 4-MUP (described in this patent application), these deoxyribozymes could also be engineered to act as sensors that only generate a signal in the presence of inputs such as oligonucleotides with specific sequences and ribonucleases that generate 5' hydroxyl and 2',3'-cyclic phosphate termini. Although not as sensitive as our chemiluminescent and fluorescent deoxyribozymes, an advantage of these chromogenic deoxyribozymes is that they generate a product that can be detected without expensive instrumentation. Several other approaches were used to identify deoxyribozymes with new substrate specificities. For example, by simply screening representative deoxyribozymes using a panel of new substrates, we identified a motif that can react with DDAO (a fluorescent substrate not used in any of our selections). Analysis of mutational frequencies in datasets of Supernova variants selected for the ability to use 4-MUP or 4-NPP also revealed that the 3C and 38C mutations change the substrate specificity of Supernova. Together, these results highlight the versatility of our method, and show that it can be used to identify deoxyribozymes that generate a wide range of signals.

### Materials and Methods for Examples 4-5

### Analysis of Production of the yellow product 4-NP

Oligonucleotides were ordered from GENERI BIOTECH s.r.o or Eurofins Genomics GmbH. Production of the product 4-NP was measured as follows: oligonucleotides were resuspended in Milli-Q water, heated at 65 °C for 2 minutes, and cooled at room temperature for 5 minutes. After adding 5× buffer, samples were transferred to a clear half-area 96-well plate (Corning). 4-NPP was added, and absorbance at 405 nm was measured for 4 hours using a Tecan Spark plate reader (Tecan Group) if not stated otherwise. In a typical experiment final concentrations were 30 µM deoxyribozyme and 1× buffer (200 mM KCl, 1 mM ZnCl₂, 50 mM HEPES pH 7.4), and 100 µM 4-NPP. Signal-to-noise ratio is defined as the absorbance at 405 nm in the presence of deoxyribozyme divided by the absorbance at 405 nm in the absence of deoxyribozyme.

### Oligonucleotide detection using an engineered version of our chromogenic deoxyribozyme

The oligonucleotide sensor was mixed with the target oligonucleotide in water, heated at 98 °C for 2 minutes, and immediately cooled on ice for 5 minutes. 5× buffer were then added. Samples were transferred to a clear half-area 96-well plate (Corning), 4-NPP was added, and the reaction mixture was incubated for 24 hours at room temperature. Final concentrations were 50 µM of the oligonucleotide sensor, 100 µM of the target oligonucleotide, 1× buffer (200 mM KCl, 50 mM HEPES pH 7.4 and 1 mM ZnCl₂), and 100 µM 4-NPP. After 24 hours the absorbance at 405 nm was then measured using a Tecan Spark plate reader. Signal-to-noise ratio is defined as the absorbance at 405 nm in the presence of deoxyribozyme divided by the absorbance at 405 nm in the absence of deoxyribozyme.

### Ribonuclease sensor based on our chromogenic deoxyribozyme

The ribonuclease sensor was heated at 65°C for 2 minutes, and cooled at room temperature for 5 minutes. Then 5× buffer was added. Samples were transferred to a clear half-area 96-well plate (Corning), and 4-NPP and either RNase A (Thermo Fisher Scientific) or Nsp15 (MyBioSourse, Inc.) were added. The reaction mixture was incubated for 4 hours (RNase A) or 24 hours (Nsp15) at room temperature. Final concentrations were 30 µM of the ribonuclease sensor, 300 nM RNase A or 5000 nM Nsp15, 1× RNase A buffer (200 mM KCl, 50 mM HEPES pH 7.4, 1 mM ZnCl₂) or 1× Nsp15 buffer (100 mM KCl, 1 mM ZnCl₂, 5 mM MnCl₂, and 20 mM HEPES, pH 7.4), and 100 µM 4-NPP if not stated otherwise. After 4 hours (RNase A) or 24 hours (Nsp15) the absorbance at 405 nm was measured using a Tecan Spark plate reader. Signal-to-noise ratio is defined as the absorbance at 405 nm in the presence of deoxyribozyme divided by the absorbance at 405 nm in the absence of deoxyribozyme.

### Example 6. Using blocked Aurora to identify Nspl5 inhibitors in a high-throughput screen

Because assays using Aurora can be performed rapidly and inexpensively, they appear to be well-suited for applications such as high-throughput screens. To establish a proof-of-principle for this idea, we used an Aurora ribonuclease sensor to search a 1000-member fragment library for inhibitors of the SARS-CoV-2 endoribonuclease Nsp15. This ribonuclease cleaves 3' of pyrimidines (preferentially uridines) to generate 2'-3' cyclic phosphate and 5' hydroxyl termini. It helps to prevent host recognition by degrading double-stranded viral intermediates, and inhibitors could potentially be useful as antiviral agents. Pilot experiments showed that it was possible to construct a version of blocked Aurora that was activated by Nsp15. To perform a screen using this sensor, it was incubated with Nsp15 and compounds from the library (**FIGs. 28A-28B**). In the absence of inhibitor, the sensor was expected to be cleaved by Nsp15 and generate fluorescence. In the presence of inhibitor, however, the sensor was not expected to be cleaved and fluorescence was not expected to be generated. A counterscreen was also performed in which library members were incubated on the presence of Aurora itself rather than blocked Aurora (**FIG. 28C**). This showed that the hits identified in our initial screen inhibit Nsp15 rather than Aurora itself (**FIG. 28C**). To compare these results to those obtained using standard methods, we repeated the screen using a FRET assay in which Nsp15 was incubated with library members and a hairpin substrate containing a fluorophore at one end and a quencher at the other (**FIG. 28D**). Cleavage of the substrate by Nsp15 was expected to result in an increase in fluorescence, while the fluorescence in wells containing Nsp15 inhibitors was expected to remain at background levels. The results of this FRET screen were virtually identical to those obtained using the Aurora sensor (**FIG. 28E**). Several hits were further characterized as a function of concentration using both the Aurora sensor and the FRET assay. The most potent of these compounds inhibited Nsp15 with an IC50 of 12 µM in an assay that used the Aurora sensor and 11 µM in an assay that used a FRET assay (**FIG. 28F**). These results show that Aurora-based sensors can be used to identify enzyme inhibitors in high-throughput screens.

### Materials and Methods for Example 6

### High-throughput screen for Nsp15 inhibitors using an Aurora Nsp15 sensor

Small molecules from a 1000-member fragment screen library (Maybridge) were transferred to the wells of 384-well plates using an Echo 550 liquid handler. Nsp15 protein in 1× Nsp15 buffer (50 mM KCl, 20 mM HEPES pH 7.4, 5 mM MnCl2, 0.003% (v/v) Tween20) was then added using a CERTUS Flex liquid handler. After mixing, the Aurora Nsp15 sensor (in 1× Nsp15 buffer) was added using a CERTUS Flex liquid handler. Reactions were mixed again. Final concentrations were 25 µM Aurora Nsp15 sensor, 400 nM Nsp15 protein, 1× Nsp15 buffer (50 mM KCl, 20 mM HEPES pH 7.4, and 5 mM MnCl2), 0.003% (v/v) Tween20, and 200 µM small molecule from the fragment screen library in a volume of 20 µl. After incubating at room temperature for 1 hour to allow Nsp15 to cleave and activate the Aurora Nsp15 sensor, 80 µl of 1× Aurora reaction mixture (50 mM KCl, 20 mM HEPES pH 7.4, 1.25 mM ZnCl2, 6.25% (v/v) DMSO, and 18.75 µM 4-MUP) was added using a CERTUS Flex liquid handler. The ZnCl₂ in this buffer inhibited the Nsp15 protein while activating Aurora for catalysis. Final concentrations were 5 µM Aurora Nsp15 sensor, 80 nM Nsp15 protein, 40 µM small molecule from the fragment screen library, 1× Aurora/Nsp15 buffer (50 mM KCl, 20 mM HEPES pH 7.4, 1 mM ZnCl2 1 mM MnCl2), 0.0006% Tween20, 5% (v/v) DMSO, and 15 µM 4-MUP in a volume of 100 µl. The reaction mixture was incubated at room temperature for 4 hours, and fluorescence was measured using a Tecan Spark plate reader. Fluorescence was measured in a black flat bottom 384-well plate (Corning). A counter screen was also performed to confirm that the inhibitors identified in the initial screen inhibit the Nsp15 protein rather than Aurora. The counter screen was performed as described above, but Aurora 2 was used instead of the Aurora Nsp15 sensor.

### High-throughput screen for Nsp15 inhibitors using a FRET assay

Small molecules from a 1000-member fragment screen library (Maybridge) were transferred to the wells of 384-well plates using an Echo 550 liquid handler. Nsp15 protein in 1× Nsp15 buffer (50 mM KCl, 20 mM HEPES pH 7.4, 5 mM MnCl₂, 0.003% (v/v) Tween20) was then added using a CERTUS Flex liquid handler. After mixing, the FRET substrate (5'-FAM-AAArUAA-BHQ1-3') in 1× Nsp15 buffer was added using a CERTUS Flex liquid handler. Reactions were mixed again. Final concentrations were 25 µM FRET substrate, 400 nM Nsp15 protein, 1× Nsp15 buffer (50 mM KCl, 20 mM HEPES pH 7.4, and 5 mM MnCl₂), 0.003% (v/v) Tween20, and 200 µM small molecule from the fragment screen library in a volume of 20 µl. Fluorescence was measured every 20 minutes for 1 hour in a black flat bottom 384-well plate (Corning) using a Tecan Spark plate reader with the following settings: excitation 485 (±5) nm, emission 527 (±5) nm, gain 134, 30 flashes, Z position calculated from the well.

### Analysis of data from high-throughput screens

Wells containing 1 mM ZnCl₂ (which inhibited Nsp15 at this concentration) served as negative controls, and were used to determine background levels of fluorescence. The average value of this background was subtracted from the fluorescence values obtained from all other wells. Wells containing aliquots of DMSO alone rather than DMSO plus small molecule were used as positive controls. After subtraction of the background, the average value of these positive controls was defined as 100% Nsp15 activity. Activity of Nsp15 in the presence of small molecules from the fragment screen library was calculated relative to this positive control value. Z-factors were calculated for each 384-well plate to determine the quality of the screen. Z-factors were calculated using the equation Z-factor = 1 - [3(*σ*ₚ + *σ*ₙ)] / (*µ*ₚ - *µ*ₙ) where *µ*ₚ is the mean fluorescence of the positive control, *µ*ₙ is the mean fluorescence of the negative control, *σ*ₚ is the standard deviation of the mean fluorescence of the positive control, and *σ*ₙ is the standard deviation of the mean fluorescence of the negative control.

### Calculation of IC₅₀ values

IC₅₀ values were measured for small molecules that strongly inhibited Nsp15 in high-throughput screens. Solutions containing different concentrations of these inhibitors were transferred to the wells of 384-well plates using an Echo 550 liquid handler. To obtain the same volume in each well, the drops containing small molecules were backfilled with DMSO to 200 nl. For each inhibitor characterized, IC₅₀ values were measured using both the Aurora Nsp15 sensor and using the FRET assay. After determining the relative activity of Nsp15 at each concentration of inhibitor, IC₅₀ values were calculated using Prism 9 software (GraphPad).

### EQUIVALENTS AND SCOPE

In the claims articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or all of the group members are present in, employed in, or otherwise relevant to a given product or process.

Furthermore, the invention encompasses all variations, combinations, and permutations in which one or more limitations, elements, clauses, and descriptive terms from one or more of the listed claims is introduced into another claim. For example, any claim that is dependent on another claim can be modified to include one or more limitations found in any other claim that is dependent on the same base claim. Where elements are presented as lists, *e.g*., in Markush group format, each subgroup of the elements is also disclosed, and any element(s) can be removed from the group. It should it be understood that, in general, where the invention, or aspects of the invention, is/are referred to as comprising particular elements and/or features, certain embodiments of the invention or aspects of the invention consist, or consist essentially of, such elements and/or features. For purposes of simplicity, those embodiments have not been specifically set forth *in haec verba* herein. It is also noted that the terms "comprising" and "containing" are intended to be open and permits the inclusion of additional elements or steps. Where ranges are given, endpoints are included. Furthermore, unless otherwise indicated or otherwise evident from the context and understanding of one of ordinary skill in the art, values that are expressed as ranges can assume any specific value or sub-range within the stated ranges in different embodiments of the invention, to the tenth of the unit of the lower limit of the range, unless the context clearly dictates otherwise.

This application refers to various issued patents, published patent applications, journal articles, and other publications, all of which are incorporated herein by reference. If there is a conflict between any of the incorporated references and the instant specification, the specification shall control. In addition, any particular embodiment of the present invention that falls within the prior art may be explicitly excluded from any one or more of the claims. Because such embodiments are deemed to be known to one of ordinary skill in the art, they may be excluded even if the exclusion is not set forth explicitly herein. Any particular embodiment of the invention can be excluded from any claim, for any reason, whether or not related to the existence of prior art.

Those skilled in the art will recognize or be able to ascertain using no more than routine experimentation many equivalents to the specific embodiments described herein. The scope of the present embodiments described herein is not intended to be limited to the above Description, but rather is as set forth in the appended claims. Those of ordinary skill in the art will appreciate that various changes and modifications to this description may be made without departing from the spirit or scope of the present invention, as defined in the following claims.

## Claims

1. A deoxyribozyme capable of catalyzing the transfer of a phosphate moiety from a substrate containing at least one phosphate moiety, concomitantly generating a fluorescent or a chromogeneic product.

2. The deoxyribozyme of claim 2, wherein the phosphate moiety is transferred to the deoxyribozyme, preferably wherein the phosphate moiety is transferred to a hydroxyl group on the deoxyribozyme, more preferably wherein the hydroxyl group is the 5' hydroxyl group of the deoxyribozyme.

3. The deoxyribozyme of any one of claims 1-2, wherein the product has greater or lesser fluorescent emissions or chromogeneic absorption at one or more wavelengths when illuminated at one or more wavelengths than the substrate.

4. The deoxyribozyme of any one of claims 1-3, wherein the substrate containing at least one phosphate moiety is selected from the group consisting of 4-methylumbelliferyl phosphate (4-MUP), 6,8-Difluoro-4-methylumbelliferyl phosphate (diFMUP), 9H-(1,3-Dichloro-9,9-dimethylacridin-2-one-7-yl) phosphate (DDAO), and 4-nitrophenyl phosphate (4-NPP).

5. The deoxyribozyme of any one of claims 2-4, wherein the substrate containing at least one phosphate moiety is 4-MUP.

6. The deoxyribozyme of any one of claims 1-5, wherein the deoxyribozyme comprises the nucleic acid sequence of any one of SEQ ID NOs: 7, 8, 12, 13, 14, 23, and 24, or a nucleic acid sequence that is at least 60%, at least 70%, at least 80%, at least 90%, at least 95%, at least 96%, at least 97%, at least 98%, or at least 99% identical to the sequence of any one of SEQ ID NOs: 7, 8, 12, 13, 14, 23, and 24.

7. The deoxyribozyme of claim 6, wherein the deoxyribozyme comprises one or more of, two or more of, three or more of, four or more of, five or more of, six or more of, seven or more of, eight or more of, nine or more of, or ten or more of: a G at nucleotide position 1, a G at nucleotide position 2, an A at nucleotide position 3, a G at nucleotide position 5, an A at nucleotide position 14, a T at nucleotide position 15, a C at nucleotide position 19, a G at nucleotide position 20, a G at nucleotide position 21, a C at nucleotide position 24, a C at nucleotide position 25, a G at nucleotide position 31, a G at nucleotide position 32, a G at nucleotide position 36, a G at nucleotide position 38, a G at nucleotide position 41, a G at nucleotide position 43, and a T at nucleotide position 44, relative to any one of SEQ ID NOs: 8, 13, 14, 23, and 24.

8. The deoxyribozyme of any one of claims 1-7, wherein the deoxyribozyme comprises the consensus sequence
GGAE₁GDDNNNZ₁Z₃NATF₁X₁P₁CGGNNCCZ₅NNNZ₆GGSNNGHGP₂X₂GF₂GTE₂Z₄Z₂ (SEQ ID NO: 37), wherein each instance of S independently represents C or G, each instance of H independently represents A, C, or T, each instance of D independently represents A, G, or T, and each instance of N independently represents A, C, G, or T; and wherein each instance of F₁ and F₂ together represents a G-C base pair, a G-T base pair, or an A-T base pair, each instance of X₁ and X₂ together represents a T-A base pair or a C-G base pair, each instance of P₁ and P₂ together represents a T-A base pair, a C-G base pair, or a T-G base pair, each instance of Z₁ and Z₂ together represents an A-T base pair, a T-A base pair, a G-C base pair, a C-G base pair, a G-T base pair, or a T-G base pair, each instance of Z₃ and Z₄ together represents an A-T base pair, a T-A base pair, a G-C base pair, a C-G base pair, a G-T base pair, or a T-G base pair, each instance of Z₅ and Z₆ together represents an A-T base pair, a T-A base pair, a G-C base pair, a C-G base pair, a G-T base pair, or a T-G base pair, and each instance of E₁ and E₂ together represents an A-T interaction, a G-A interaction, an A-A interaction, or an A-G interaction.

9. The deoxyribozyme of any one of claims 1-4, wherein the substrate containing at least one phosphate moiety is 4-NPP.

10. The deoxyribozyme of any one of claims 1-4 or claim 9, wherein the deoxyribozyme comprises the nucleic acid sequence of any one of SEQ ID NOs: 38, 39, 40, 41, 50, 51, 52, 53, and 59 or a nucleic acid sequence that is at least 60%, at least 70%, at least 80%, at least 90%, at least 95%, at least 96%, at least 97%, at least 98%, or at least 99% identical to the sequence of any one of SEQ ID NOs: 38, 39, 40, 41, 50, 51, 52, 53, and 59.

11. The deoxyribozyme of claim 10, wherein the deoxyribozyme comprises nucleotides 1-8 of SEQ ID NO: 38 and residues 34-64 of SEQ ID NO: 38; and preferably the deoxyribozyme further comprises nucleotides 9-12 and 30-33 of SEQ ID NO: 38 as well as a loop (such as GCA) inserted between nucleotides 12 and 30.

12. The deoxyribozyme of any one of claims 1-4 or claims 9-11, wherein the deoxyribozyme comprises any one of the consensus sequences GGCAGAGAHRGC (SEQ ID NO: 47) -loop-KCHDYRCATGCTCMCCCKBDGGGGCAATCGGGGGG (SEQ ID NO: 48) and GGCAGAGAYRCATGCTCMCCCKBDGGGGCAATCGGGGGG (SEQ ID NO: 49), wherein each instance of B independently represents C, G, or T, each instance of D independently represents A, G, or T, each instance of H independently represents A, C, or T, each instance of K represents G or T, each instance of M represents A or C, each instance of R represents A or G, and each instance of Y represents C or T.

13. A method of identifying at least one deoxyribozyme according to claim 1 capable of catalyzing the transfer of a phosphate moiety from a substrate containing at least one phosphate moiety, said method comprising the steps of:
a. annealing a library containing a plurality of single-stranded deoxyribonucleotides or regenerated according to step (j) of this method to a blocking oligonucleotide,
b. incubating the library annealed to the blocking oligonucleotide of step (a) with a substrate containing at least one phosphate moiety,
c. isolating the DNA of step (b),
d. ligating a deoxyribonucleotide oligo using a splint oligo to the purified DNAs of step (c) containing a 5' phosphate,
e. isolating the deoxyribonucleotide ligated products of step (d),
f. amplifying the isolated deoxyribonucleotide ligation products of step (e) using one forward primer which contains a ribonucleic acid (RNA) linkage 5' of the phosphorylation site and a second reverse deoxyribonucleic acid primer which contains a 5' phosphate,
g. isolating the DNA of step (f),
h. incubating the amplified deoxyribonucleotide ligation products of step (g) with lambda exonuclease to selectively degrade the antisense strand,
i. digesting the ribonucleic acid (RNA) linkage 5' of the phosphorylation site in the sense strands of DNA of step (h),
j. generating a plurality of single stranded deoxyribonucleotides having the sequence of the deoxyribonucleotide ligation products isolated in step (e), and
k. repeating steps (a)-(j) one or more times;
optionally wherein the pool of single-stranded deoxyribonucleotides of step (a) comprises the sequences of SEQ ID NO: 1, SEQ ID NO: 9, or SEQ ID NO: 42, or optionally wherein the pool of single-stranded deoxyribonucleotides of step (a) comprises one or more secondary structure libraries.

14. An oligonucleotide sensor comprising a deoxyribozyme according to any one of claims 1 to 12 capable of catalyzing the transfer of a phosphate moiety from a substrate containing at least one phosphate, concomitantly generating a fluorescent or chromogenic product only when the deoxyribozyme is bound to a target nucleotide sequence.

15. The oligonucleotide sensor of claim 14 having the sequence of any one of SEQ ID NOs: 25-29 and SEQ ID NO: 43, optionally wherein the nucleotide target sequence is the sequence of any one of SEQ ID NOs: 30-34 and SEQ ID NO: 44.

16. A method for detecting the presence of a target nucleotide sequence comprising contacting a sample comprising a plurality of nucleotide sequences with the oligonucleotide sensor of claim 14 or 15, wherein fluorescence or absorption is observed when the target nucleotide sequence is present in the sample.

17. An RNase detection system comprising a deoxyribozyme according to any one of claims 1 to 12 and comprising a 5' nucleotide extension to a deoxyribozyme, the extension preferably comprising at least 1, 2, 3, 4, 5, 10, 15, 20, 30, 40, or 50 nucleotides, of which the 3' most residue of the extension is a ribonucleotide, and can be cleaved from the RNase detection system by RNase to generate a deoxyribozyme capable of catalyzing the transfer of a phosphate moiety from a substrate containing at least one phosphate, concomitantly generating a fluorescent or chromogeneic product.

18. The RNase detection system of claim 17 comprising any one of the sequences of SEQ ID NOs: 35, 36, 45, and 46.

19. A method for detecting the presence of a ribonuclease or an inhibitor of a ribonuclease comprising contacting a sample comprising the ribonuclease or ribonuclease and inhibitor with the RNase detection system of claim 17 or 18, wherein fluorescence or absorption is observed when the target ribonuclease is present in the sample, whereas no fluorescence or absorption is observed when both the target ribonuclease and an inhibitor of the ribonuclease is present in the sample.
